# EUROPEAN PATENT APPLICATION

(11) **EP 2 159 217 A1**
(43) Date of publication of application: **03.03.2010**
(21) Application number: 08765691.4
(22) Date of filing: 17.06.2008
(51) Int. Cl.: C07C 211/61, C09K 11/06, H01L 51/50

(54) **AROMATIC AMINE DERIVATIVE AND ORGANIC ELECTROLUMINESCENT DEVICE USING THE SAME**

(30) Priority: 18.06.2007 JP 2007160563
(71) Applicant: Idemitsu Kosan Co., Ltd., Chiyoda-ku Tokyo 100-8321 (JP)
(72) Inventor: FUNAHASHI, Masakazu, Sodegaura-shi Chiba 299-0293 (JP); IWAMOTO, Shintaro, Sodegaura-shi Chiba 299-0293 (JP); ITO, Mitsunori, Sodegaura-shi Chiba 299-0293 (JP); MIZUKI, Yumiko, Sodegaura-shi Chiba 299-0293 (JP)
(74) Representative: Vossius & Partner
(86) International application number: PCT/JP2008/061058
(87) International publication number: WO 2008/156089

(57) **Abstract**

An aromatic amine derivative having ring structures on the both sides of central double bond structure, an organic electroluminescence device including the aromatic amine derivative, and an organic electroluminescence material-containing solution including the aromatic amine derivative as one of organic electroluminescence materials and a solvent, the organic electroluminescence device having a long lifetime and high luminous efficiency and being capable of emitting blue light having a high color purity, and being realized with the aromatic amine derivative and the organic electroluminescence material-containing solution.

## Description

### Technical Field

The present invention relates to an aromatic amine derivative and an organic electroluminescence device using the same, in particular, an organic electroluminescence device having a long lifetime and high luminous efficiency, and capable of emitting blue light having a high color purity, and an aromatic amine derivative for realizing the device.

### Background Art

A large number of organic electroluminescence (EL) devices each using an organic substance have been developed because of their potential to find applications in solid light emission type, inexpensive, large-area, full-color display devices. In general, an EL device is constituted of a light emitting layer and a pair of opposing electrodes between which the layer is interposed. Light emission is the phenomenon in which when an electric field is applied between both the electrodes, an electron is injected from a cathode side and a hole is injected from an anode side, and, further, the electron recombines with the hole in the light emitting layer to produce an excited state, and energy generated upon return of the excited state to a ground state is emitted as light.
A conventional organic EL device was driven at a voltage higher than the voltage at which an inorganic light emitting diode is driven, and had emission luminance and luminous efficiency lower than those of the diode. In addition, the properties of the device deteriorated remarkably, so the device has not been put into practical use. Although a recent organic EL device has been gradually improved, additionally high luminous efficiency and an additionally long lifetime of the device are requested. For example, a technique involving the use of a single monoanthracene compound as an organic light emitting material is disclosed (Patent Document 1). However, the technique provides a luminance as low as 1, 650 cd/m² at a current density of, for example, 165 mA/cm², and provides extremely low efficiency, specifically, 1 cd/A, so the technique is not practical. In addition, a technique involving the use of a single bisanthracene compound as an organic light emitting material is disclosed (Patent Document 2). However, even the technique provides an efficiency as low as about 1 to 3 cd/A, so an improvement for putting the technique into practical use has been demanded. Meanwhile, a long-lifetime organic EL device obtained by adding, for example, styrylamine to a distyryl compound to be used as an organic light emitting material is proposed (Patent Document 3). However, the device does not have a sufficient lifetime, and the additional improvement of the device has been demanded.
In addition, techniques each involving the use of each of a monoanthracene or bisanthracene compound and a distyryl compound in an organic light emitting medium layer is disclosed (Patent Document 4). However, in each of those techniques, the conjugate structure of the styryl compound lengthens the wavelength of an emission spectrum, with the result that a color purity is deteriorated. Further, Patent Document 5 discloses a blue light emitting device using an asymmetric styryl compound.
However, although the device is excellent in luminous efficiency, the device does not have a sufficient lifetime, and the additional improvement of the device has been demanded.

Patent Document 1: JP 11-3782 A
Patent Document 2: JP 08-12600 A
Patent Document 3: WO 94/006157
Patent Document 4: JP 2001-284050 A
Patent Document 5: WO 06/49860

### Disclosure of the Invention

### Problem to be solved by the Invention

The present invention has been made with a view to solving the above problems, and an object of the present invention is to provide an organic EL device having a long lifetime and high luminous efficiency, and capable of emitting blue light having a high color purity, and an aromatic amine derivative and an organic EL material-containing solution for realizing the device.

### Means for solving the Problem

The inventors of the present invention have made extensive studies with a view to developing an aromatic amine derivative having the above preferred nature and an organic EL device using the derivative. As a result, the inventors have found that the object can be achieved with the utilization of an aromatic amine derivative having a specific structure represented by the following general formula (I). The present invention has been completed on the basis of such finding.

That is, the present invention provides an aromatic amine derivative represented by the following general formula (I):

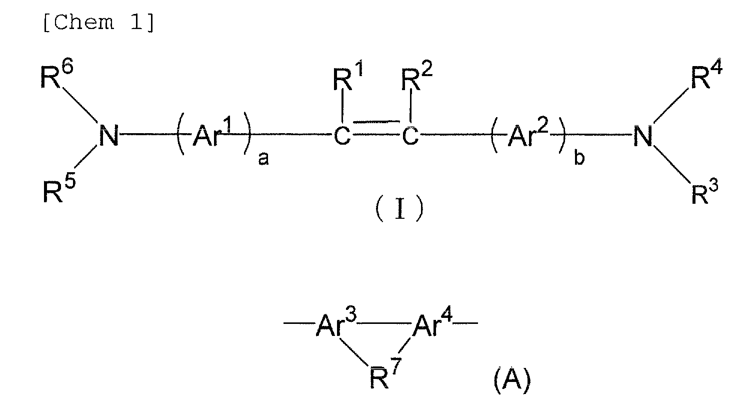

where:
R¹ and R² each independently represent a hydrogen atom, a substituted or unsubstituted alkyl group having 1 to 50 carbon atoms, or a substituted or unsubstituted aryl group having 5 to 50 carbon atoms;
R³ to R⁶ each independently represent a substituted or unsubstituted alkyl group having 1 to 50 carbon atoms, a substituted or unsubstituted aryl group having 5 to 50 carbon atoms, or a substituted or unsubstituted heterocyclic group having 5 to 50 carbon atoms;
Ar¹ and Ar² each independently represent a substituted or unsubstituted arylene group having 5 to 50 carbon atoms, a substituted or unsubstituted, divalent heterocyclic group having 5 to 50 carbon atoms, or a group represented by the above general formula (A) where Ar³ and Ar⁴ each independently represent a substituted or unsubstituted arylene group having 5 to 50 carbon atoms, or a substituted or unsubstituted, divalent heterocyclic group having 5 to 50 carbon atoms, and R⁷ represents a substituted or unsubstituted alkylene group having 1 to 50 carbon atoms, -O-, -S-, -SO₂-, -SiR⁸R⁹-, or -NR¹⁰- where R⁸, R⁹, and R¹⁰ each independently represent a substituted or unsubstituted alkyl group having 1 to 50 carbon atoms, or a substituted or unsubstituted aryl group having 5 to 50 carbon atoms;
a and b each independently represent an integer of 1 to 3, when a represents 2 or more, multiple Ar¹'s may be identical to or different from each other, and when b represents 2 or more, multiple Ar²'s may be identical to or different from each other; and
R¹ and R², R¹ and Ar¹ R² and Ar² R³ and R⁴ R⁵ and R⁶, Ar¹ and R⁵ and/or R⁶, or Ar² and R³ and/or R⁴ may be bonded and linked to each other to wholly form a saturated or unsaturated ring,
provided that, when all R³ to R⁶ each represent an unsubstituted phenyl group and a=b=1, at least one of Ar¹ and Ar² represents a group except an unsubstituted 1,4-phenylene group and an unsubstituted 1,4-naphthylene group.

Further, the present invention provides an organic EL device including an organic thin film layer formed of one or more layers including at least a light emitting layer and interposed between a cathode and an anode, in which at least one layer of the organic thin film layer contains the aromatic amine derivative.
Still further, the present invention provides an organic EL material-containing solution including the aromatic amine derivative as one of organic EL materials, and a solvent.

### Effect of the Invention

An organic EL device using the aromatic amine derivative of the present invention provides practically sufficient emission luminance at a low applied voltage, has high luminous efficiency, hardly deteriorates even when used for a long time period, and has a long lifetime.

### Best Mode for carrying out the Invention

An aromatic amine derivative of the present invention is a compound represented by the following general formula (I).

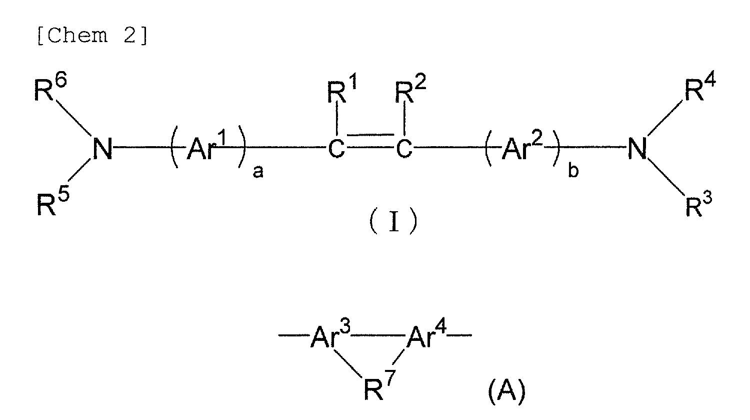

In the general formula (I):
R¹ and R² each independently represent a hydrogen atom, a substituted or unsubstituted alkyl group having 1 to 50 carbon atoms, or a substituted or unsubstituted aryl group having 5 to 50 carbon atoms; R³ to R⁶ each independently represent a substituted or unsubstituted alkyl group having 1 to 50 carbon atoms, a substituted or unsubstituted aryl group having 5 to 50 carbon atoms, or a substituted or unsubstituted heterocyclic group having 5 to 50 carbon atoms.

Examples of the alkyl group having 1 to 50 carbon atoms (an alkyl group having preferably 1 to 20 carbon atoms and more preferably 1 to 10 carbon atoms) represented by each of R¹ to R⁶ include a methyl group, an ethyl group, a propyl group, an isopropyl group, a butyl group, a sec-butyl group, a tert-butyl group, a pentyl group, a hexyl group, a heptyl group, an octyl group, a stearyl group, a 2-phenylisopropyl group, a trichloromethyl group, a trifluoromethyl group, a benzyl group, an α-phenoxybenzyl group, an α,α-dimethylbenzyl group, an α,α-methylphenylbenzyl group, an α,α-ditrifluoromethylbenzyl group, a triphenylmethyl group, and an α-benzyloxybenzyl group.

Examples of the aryl group having 5 to 50 carbon atoms (an aryl group having preferably 5 to 20 carbon atoms) represented by each of R¹ to R⁶ include a phenyl group, a 2-methylphenyl group, a 3-methylphenyl group, a 4-methylphenyl group, a 4-ethylphenyl group, a biphenyl group, a 4-methylbiphenyl group, a 4-ethylbiphenyl group, a 4-cyclohexylbiphenyl group, a terphenyl group, a 3,5-dichlorophenylgroup, a naphthyl group, a 5-methylnaphthylgroup, a 1-naphthyl group, a 2-naphthyl group, an anthryl group, a pyrenyl group, a fluorenyl group, a crysenyl group, a phenanthyl group, a 9,9-dimethylfluorene-1-yl group, a 9,9-dimethylfluorene-2-yl group, a 9,9-dimethylfluorene-3-yl group, and a 9,9-dimethylfluorene-4-yl group.
Examples of the heterocyclic group having 5 to 50 carbon atoms (a heterocyclic group having preferably 5 to 20 carbon atoms) represented by each of R³ to R⁶ include residues of imidazole, benzimidazole, pyrrole, furan, thiophene, benzothiophene, oxadiazoline, indoline, carbazole, pyridine, quinoline, isoquinoline, benzoquinone, pyralozine, imidazolidine, and piperidine.

In the general formula (I), R¹ and R² may be in cis positions or trans positions with respect to the central -C=C-.
In the general formula (I), Ar¹ and Ar⁴ each independently represent a substituted or unsubstituted arylene group having 5 to 50 carbon atoms, a substituted or unsubstituted, divalent heterocyclic group having 5 to 50 carbon atoms, or a group represented by the above general formula (A).
Examples of the arylene group and the heterocyclic group each represented by any one of Ar¹ to Ar⁴ include examples obtained by making the examples of the aryl group and the heterocyclic group described for R¹ to R⁶ divalent.
In addition, when Ar¹ and Ar² each represent an arylene group, the arylene group is preferably a fused ring. When Ar¹ represents a phenyl group, Ar² preferably represents a fused ring, or specifically, a naphthyl group, a fluorenyl group, a 9,9-dimethylfluoren-1-yl group, a 9,9-dimethylfluoren-2-yl group, a 9,9-dimethylfluoren-3-yl group, or a 9,9-dimethylfluoren-4-yl group.

In the general formula (A), Ar³ and Ar⁴ each independently represent a substituted or unsubstituted arylene group having 5 to 50 carbon atoms, or a substituted or unsubstituted, divalent heterocyclic group having 5 to 50 carbon atoms, and R⁷ represents a substituted or unsubstituted alkylene group having 1 to 50 carbon atoms, -O-, -S-, -SO₂-, -SiR⁸R⁹-, or -NR¹⁰- (R⁸, R⁹, and R¹⁰ each independently represent a substituted or unsubstituted alkyl group having 1 to 50 carbon atoms, or a substituted or unsubstituted aryl group having 5 to 50 carbon atoms), and examples of those groups include the same examples as those described for R¹ to R⁶.
It is preferred that Ar¹ and Ar² in the general formula (I) each independently represent a group represented by the general formula (A), and it is more preferred that Ar¹ and Ar² be identical to each other and each represent a group represented by the general formula (A). In addition, when Ar¹ represents a substituted or unsubstituted arylene group having 5 to 50 carbon atoms, or a substituted or unsubstituted, divalent heterocyclic group having 5 to 50 carbon atoms, Ar¹ and Ar² preferably represent different groups.

In the general formula (I):
a and b each independently represent an integer of 1 to 3, when a represents 2 or more, multiple Ar¹'s may be identical to or different from each other, and when b represents 2 or more, multiple Ar²'s may be identical to or different from each other; and
R¹ and R², R¹ and Ar¹, R² and Ar², R³ and R⁴, R⁵ and R⁶, Ar¹ and R⁵ and/or R⁶, or Ar² and R³ and/or R⁴ may be bonded and linked to each other to form a saturated or unsaturated ring.
Examples of the ring formed by bonding between R¹ and R² include cyclopentene and cyclohexene.
Examples of the ring formed by bonding between R¹ and Ar² or between R² and Ar² include indene, dihydronaphthalene, and naphthalene.
The ring formed by bonding between R³ and R⁴ or between R⁵ and R⁶ is, for example, carbazole.
The ring formed by bonding between Ar¹ and R⁵ and/or R⁶ or between Ar² and R³ and/or R⁴ is, for example, carbazole.
It should be noted that, when all R³ to R⁶ each represent an unsubstituted phenyl group and a=b=1 in the general formula (I), the case where both Ar¹ and Ar² each represent an unsubstituted 1,4-phenylene group or an unsubstituted 1,4-naphthylene group is excluded.

The aromatic amine derivative of the present invention preferably has a structure in which the general formula (I) satisfies the following conditions:
R¹ to R⁶, and Ar¹ and Ar² each independently have the same meaning as that described above;
in the formula (A), Ar³ and Ar⁴ each independently have the same meaning as that described above, and R⁷ represents -O-, -S-, -SO₂-, -SiR⁸R⁹-, or -NR¹⁰- where R⁸, R⁹, and R¹⁰ each independently represent a substituted or unsubstituted alkyl group having 1 to 50 carbon atoms, or a substituted or unsubstituted aryl group having 5 to 50 carbon atoms;
a and b each independently represent an integer of 1; and
R¹ and R² may be linked to each other to form a ring except an aromatic ring,
provided that at least one of Ar¹ and Ar² represents a group except a substituted or unsubstituted 1,4-phenylene group, a substituted or unsubstituted 1,4-naphthylene group, a substituted or unsubstituted 2,6-naphthylene group, and a substituted or unsubstituted 9,10-anthracenylene group.

In addition, the aromatic amine derivative of the present invention more preferably has a structure in which the general formula (I) satisfies the following conditions:
R¹ to R⁶ each independently have the same meaning as that described above;
Ar¹ represents a group represented by the above general formula (A) where Ar³, Ar⁴, and R⁷ each independently have the same meaning as that described above;
Ar²'s each independently represent a substituted or unsubstituted arylene group having 5 to 50 carbon atoms, or a substituted or unsubstituted, divalent heterocyclic group having 5 to 50 carbon atoms;
a represents an integer of 1 to 3, or preferably 1, and b represents an integer of 2 or 3; and
R¹ and R² may be linked to each other to form a ring except an aromatic ring.

Further, the aromatic amine derivative of the present invention represented by the general formula (I) preferably has a structure represented by the following general formulae (II) to (XVIII).
General formula (II)

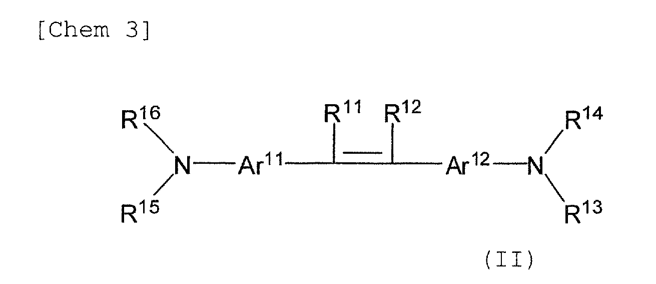

In the formula:
R¹¹ and R¹² each independently have the same meaning as that of each of R¹ and R²;
R¹³ to R¹⁶ each independently have the same meaning as that of each of R³ to R⁶;
Ar¹¹ and Ar¹² each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms, provided that Ar¹¹ and Ar¹² are different from each other; and
R¹² and R¹² may be linked to each other to form a ring except an aromatic ring.
Examples of the fused ring group represented by each of Ar¹¹ and Ar¹² include a 1, 4-naphthalene group, a 2, 6-naphthalene group, a 2, 7-naphthalene group, a 2, 5-naphthalene group, a 1, 5-naphthalene group, a 9,10-anthracene group, a 1,4-anthracene group, a 2,6-anthracene group, a 6, 12-chrysene group, a 9,10-phenanthrene group, a 1,6-pyrene group, a 2,7-pyrene group, a 1,8-pyrene group, a 7,12-benzanthracene group, a 7,10-fluoranthene group, and a 7,12-benzo[k]fluoranthene group, all of which are divalent and substituted or unsubstituted groups, and preferred are a 1,4-naphthalene group, a 2,6-naphthalene group, a 2,7-naphthalene group, a 9,10-anthracene group, a 2,6-anthracene group, a 6, 12-chrysene group, a 1,6-pyrene group, a 2,7-pyrene group, a 1,8-pyrene group, a 7,12-benzanthracene group, a 1,5-anthracene group, a 1,4-triphenylene group, and a 2,7-triphenylene group, all of which are divalent and substituted or unsubstituted groups.

General formula (III)

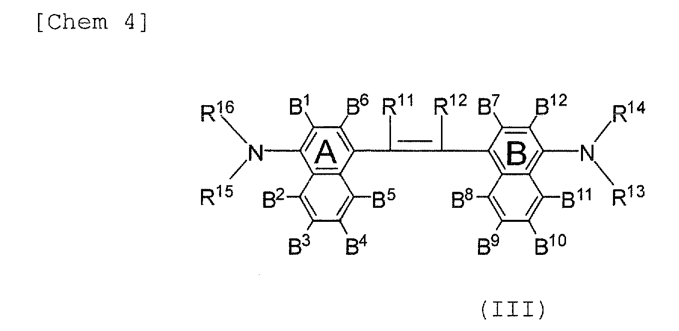

In the formula:
R¹¹ to R¹⁶ each independently have the same meaning as that described above;
a ring A and a ring B each independently represent a substituted or unsubstituted fused ring group having 10 to 50 carbon atoms, the group having a skeleton of a 1, 4-naphthalene residue, preferably a substituted or unsubstituted fused ring group having 10 to 20 carbon atoms, the group having a skeleton of a 1, 4-naphthalene residue, or more preferably a substituted or unsubstituted fused ring group having 10 to 14 carbon atoms, the group having a skeleton of a 1,4-naphthalene residue;
B¹ to B¹² each independently represent a hydrogen atom, an alkyl group having 1 to 4 carbon atoms, a cycloalkyl group having 5 or 6 carbon atoms, or an aryl group having 6 to 16 carbon atoms, provided that groups represented by one or more pairs selected from B¹ and B⁶, B² and B³, B³ and B⁴, B⁴ and B⁵, B⁷ and B¹², B⁸ and B⁹, B⁹ and B¹⁰, and B¹⁰ and B¹¹ are linked to each other to form unsaturated rings, and the unsaturated rings may each independently have a hydrogen atom, an alkyl group having 1 to 4 carbon atoms, a cycloalkyl group having 5 or 6 carbon atoms, or an aryl group having 6 to 16 carbon atoms, and the ring A and the ring B are different from each other; and R¹¹ and R¹² may be linked to each other to form a ring except an aromatic ring.
Examples of the ring A formed by linking between groups represented by a pair selected from B¹ and B⁶, B² and B³, B³ and B⁴, and B¹ and B⁵, and the ring B formed by linking between groups represented by a pair selected from B⁷ and B¹², B⁸ and B⁹, B⁹ and B¹⁰, and B¹⁰ and B¹¹ can be selected from the examples of Ar¹¹ and Ar¹².
Examples of B¹ to B¹², and the alkyl group and the aryl group each of which the unsaturated rings that may be formed by linking among them each have include examples each having a corresponding carbon number out of the examples described for R¹ to R⁶. In addition, examples of the cycloalkyl group include a cyclopropyl group, a cyclopentyl group, and a cyclohexyl group.

General formula (IV)

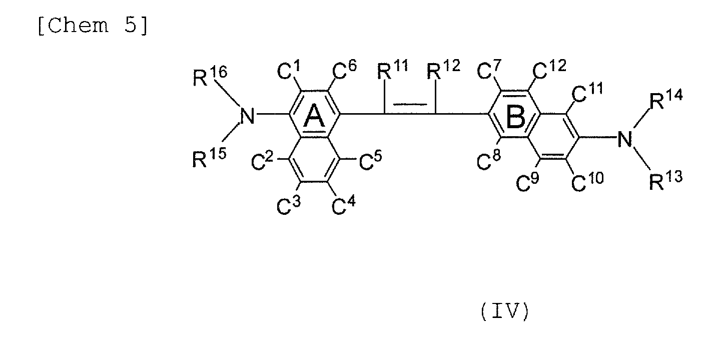

In the formula:
R¹¹ to R¹⁶ each independently have the same meaning as that described above;
C¹ to C¹² each independently represent a hydrogen atom, an alkyl group having 1 to 4 carbon atoms, a cycloalkyl group having 5 or 6 carbon atoms, or an aryl group having 6 to 16 carbon atoms, C¹ and C⁶, C² and C³, C³ and C⁴, C⁴ and C⁵, C⁷ and C¹², or C⁹ and C¹⁰ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and R¹¹ and R¹² may be linked to each other to form a ring except an aromatic ring.
Examples of the fused ring group of the ring A formed by linking between groups represented by a pair selected from C¹ and C⁶, C² and C³, C³ and C⁴, and C⁴ and C⁵, and examples of the ring B formed by linking between groups represented by a pair selected from C⁷ and C¹², and C⁹ and C¹⁰ can be selected from the examples of Ar¹¹ and Ar¹².
Examples of C¹ to C¹², and the alkyl group, the cycloalkyl group, and the aryl group each of which the unsaturated rings that may be formed by linking among them each have include the same examples as those described for B¹ to B¹²

General formula (V)

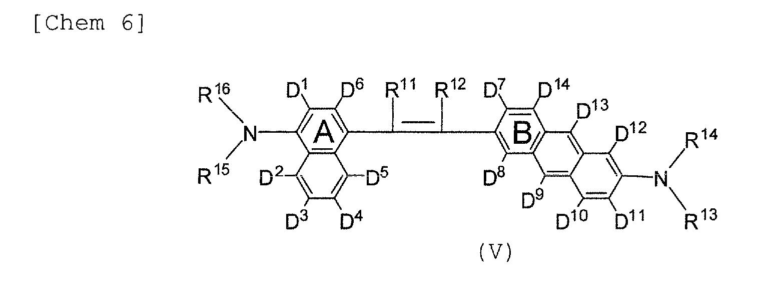

In the formula:
R¹¹ to R¹⁶ each independently have the same meaning as that described above;
a ring A and a ring B each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms;
D¹ to D¹⁴ each independently represent a hydrogen atom, an alkyl group having 1 to 4 carbon atoms, a cycloalkyl group having 5 or 6 carbon atoms, or an aryl group having 6 to 16 carbon atoms, D¹ and D⁶, D² and D³, D³ and D⁴, D⁴ and D⁵, D⁷ and D¹⁴, or D¹⁰ and D¹¹ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.
Examples of the ring A formed by linking between groups represented by a pair selected from D¹ and D⁶, D² and D³, D³ and D⁴, and D⁴ and D⁵, and the ring B formed by linking between groups represented by a pair selected from D⁷ and D¹⁴, and D¹⁰ and D¹¹ can be selected from the examples of Ar¹¹ and Ar¹².
Examples of D¹ to D¹⁴, and the alkyl group, the cycloalkyl group, and the aryl group each of which the unsaturated rings that may be formed by linking among them each have include the same examples as those described for B¹ to B¹².

General formula (VI)

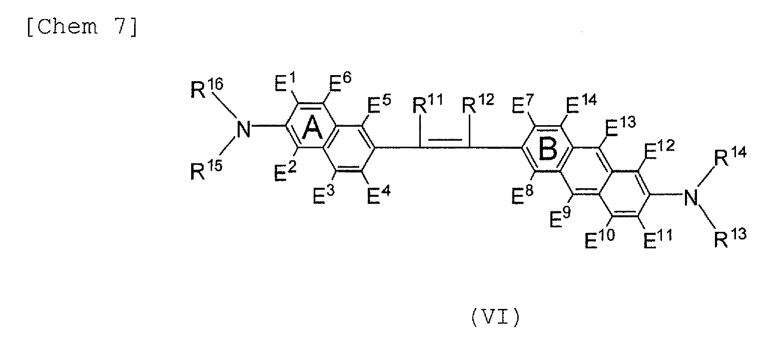

In the formula:
R¹¹ to R¹⁶ each independently have the same meaning as that described above;
a ring A and a ring B each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms;
E¹ to E¹⁴ each independently represent a hydrogen atom, an alkyl group having 1 to 4 carbon atoms, a cycloalkyl group having 5 or 6 carbon atoms, or an aryl group having 6 to 16 carbon atoms, E¹ and E⁶, E³ and E⁴, E¹⁰ and E¹¹, or E⁷ and E¹⁴ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.
Examples of the ring A formed by linking between groups represented by a pair selected from E¹ and E⁶, and E³ and E⁴, and the ring B formed by linking between groups represented by a pair selected from E¹⁰ and E¹¹, and E⁷ and E¹⁴ can be selected from the examples of Ar¹¹ and Ar¹².
Examples of E¹ to E¹⁴, and the alkyl group, the cycloalkyl group, and the aryl group each of which the unsaturated rings that may be formed by linking among them each have include the same examples as those described for B¹ to B¹².

General formula (VII)

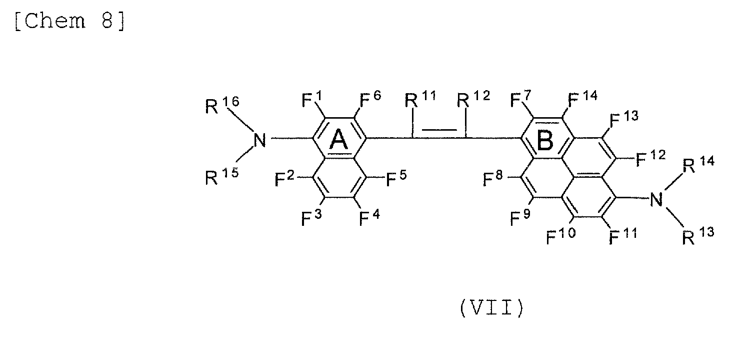

In the formula:
R¹² to R¹⁶ each independently have the same meaning as that described above;
a ring A and a ring B each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms;
F¹ to F¹⁴ each independently represent a hydrogen atom, an alkyl group having 1 to 4 carbon atoms, a cycloalkyl group having 5 or 6 carbon atoms, or an aryl group having 6 to 16 carbon atoms, F¹ and F⁶, F² and F³, F³ and F⁴, F⁴ and F⁵ F⁸ and F⁹ F¹⁰ and F¹¹, F¹² and F¹³, or F¹⁴ and F⁷ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and R¹¹ and R¹² may be linked to each other to form a ring except an aromatic ring.
Examples of the ring A formed by linking between groups represented by a pair selected from F¹ and F⁶, F² and F³, F³ and F⁴, and F⁴ and F⁵, and the ring B formed by linking between groups represented by a pair selected from F⁸ and F⁹, F¹⁰ and F¹¹, F¹² and F¹³, and F¹⁴ and F⁷ can be selected from the examples of Ar¹¹ and Ar¹²
Examples of F¹ to F¹⁴, and the alkyl group, the cycloalkyl group, and the aryl group each of which the unsaturated rings that may be formed by linking among them each have include the same examples as those described for B¹ to B¹².

General formula (VIII)

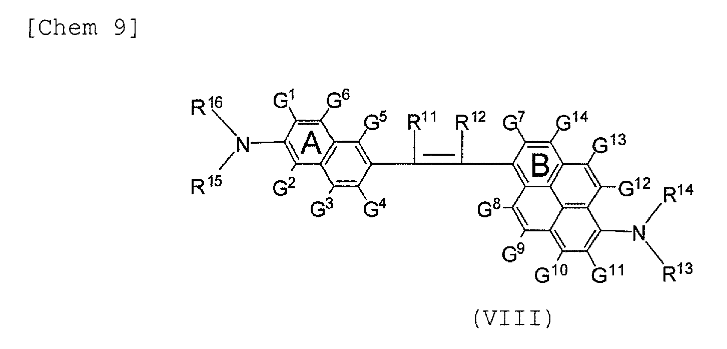

In the formula:
R¹¹ to R¹⁶ each independently have the same meaning as that described above;
a ring A and a ring B each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms;
G¹ to G¹⁴ each independently represent a hydrogen atom, an alkyl group having 1 to 4 carbon atoms, a cycloalkyl group having 5 or 6 carbon atoms, or an aryl group having 6 to 16 carbon atoms, G¹ and G⁶, G³ and G⁴, G⁸ and G⁹, G¹⁰ and G¹¹, G¹² and G¹³, or G¹⁴ and G⁷ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and R¹¹ and R¹² may be linked to each other to form a ring except an aromatic ring.
Examples of the ring A formed by linking between groups represented by a pair selected from G¹ and G⁶, G³ and G⁴, and G⁸ and G⁹, and the ring B formed by linking between groups represented by a pair selected from G¹⁰ and G¹¹, G¹² and G¹³, and G¹⁴ and G⁷ can be selected from the examples of Ar¹¹ and Ar¹².
Examples of G¹ to G¹⁴, and the alkyl group, the cycloalkyl group, and the aryl group each of which the unsaturated rings that may be formed by linking among them each have include the same examples as those described for B¹ to B¹².
General formula (IX)

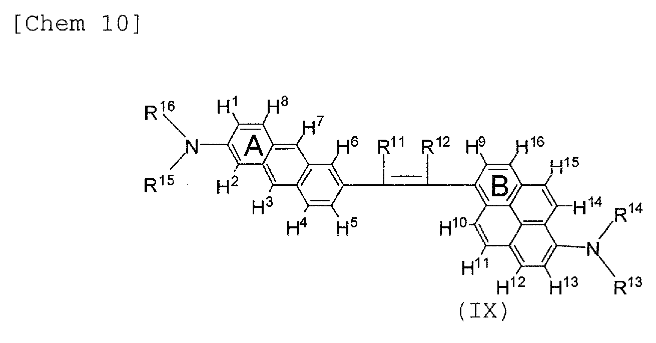

In the formula:
R¹¹ to R¹⁶ each independently have the same meaning as that described above;
a ring A and a ring B each independently represent a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms;
H¹ to H¹⁶ each independently represent a hydrogen atom, an alkyl group having 1 to 4 carbon atoms, a cycloalkyl group having 5 or 6 carbon atoms, or an aryl group having 6 to 16 carbon atoms, H¹ and H⁸, H⁴ and H⁵, H¹⁰ and H¹¹, H¹² and H¹³, H¹⁴ and H¹⁵, or H¹⁶ and H⁹ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.
Examples of the ring A formed by linking between groups represented by a pair selected from H¹ and H⁸, and H⁴ and H⁵, and the ring B formed by linking between groups represented by a pair selected from H¹⁰ and H¹¹, H¹² and H¹³, H¹⁴ and H¹⁵, and H¹⁶ and H⁹ can be selected from the examples of Ar¹¹ and Ar¹².
Examples of H¹ to H¹⁶, and the alkyl group, the cycloalkyl group, and the aryl group each of which the unsaturated rings that may be formed by linking among them each have include the same examples as those described for B¹ to B¹².

General formula (X)

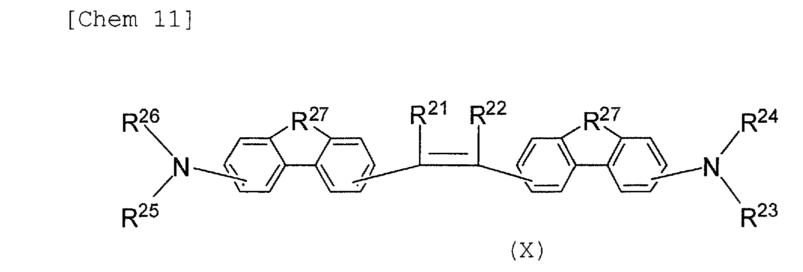

In the formula:
R²¹ and R²² each independently have the same meaning as that of each of R¹ and R²;
R²³ to R²⁶ each independently have the same meaning as that of each of R³ to R⁶;
R²⁷ represents -O-, -S-, -SO₂-, -SiR²⁸R²⁹-, or -NR³⁰- where R²⁸, R²⁹, and R³⁰ each independently represent a substituted or unsubstitutedalkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substituted or unsubstituted alkyl group having 1 to 10 carbon atoms, or a substituted or unsubstituted aryl group having 5 to 10 carbon atoms; and
R²¹ and R²² may be linked to each other to form a ring except an aromatic ring.
Examples of the alkyl group and the aryl group each represented by any one of R²⁸ to R³⁰ include the same examples as those described for R¹ to R⁶.

General formula (XI)

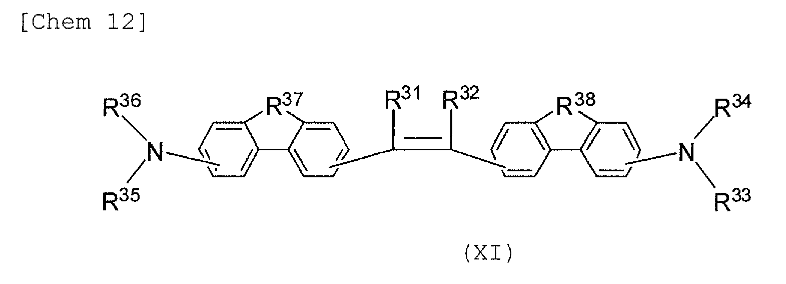

In the formula:
R³¹ and R³² each independently have the same meaning as that of each of R¹ and R²;
R³³ to R³⁶ each independently have the same meaning as that of each of R³ to R⁶;
R³⁷ and R³⁸ each represent -O-, -S-, -SO₂-, -SiR¹³¹R¹³²-, or -NR¹³³-where R¹³¹, R¹³², and R¹³³ each independently represent a substituted or unsubstituted alkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substitutedor unsubstituted alkyl group having 1 to 10 carbon atoms, or a substituted or unsubstituted aryl group having 5 to 10 carbon atoms, provided that R³⁷ and R³⁸ are different from each other; and
R³¹ and R³² may be linked to each other to form a ring except an aromatic ring.
Examples of the alkyl group and the aryl group each represented by any one of R¹³¹ and R¹³² include the same examples as those described for R¹ to R⁶.

General formula (XII)

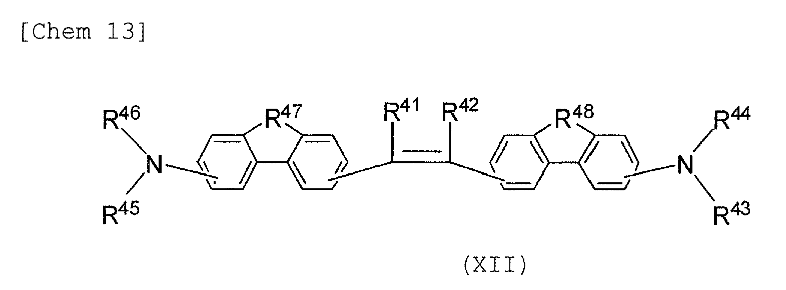

In the formula:
R⁴¹ and R⁴² each, independently have the same meaning as that of each of R¹ and R²;
R⁴³ to R⁴⁶ each independently have the same meaning as that of each of R³ to R⁶;
R⁴⁷ represents -O-, -S-, -SO₂-, -SiR¹⁴¹R¹⁴²-, or -NR¹⁴³- where R¹⁴¹, R¹⁴², and R¹⁴³ each independently represent a substituted or unsubstituted alkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substituted or unsubstituted alkyl group having 1 to 10 carbon atoms, or a substituted or unsubstituted aryl group having 5 to 10 carbon atoms;
R⁴⁸ represents a substituted or unsubstituted alkylene group having 1 to 50 carbon atoms, preferably an alkylene group having 1 to 20 carbon atoms, ormorepreferablya substitutedorunsubstituted alkylene group having 1 to 5 carbon atoms; and
R⁴¹ and R⁴² may be linked to each other to form a ring except an aromatic ring.
Examples of the alkyl group and the aryl group each represented by any one of R¹⁴¹ to R¹⁴³ include the same examples as those described for R¹ to R⁶, and examples of the alkylene group represented by R⁴⁸ include examples obtained by making the examples of the alkyl group described for R¹ to R⁶ divalent.

General formula (XIII)

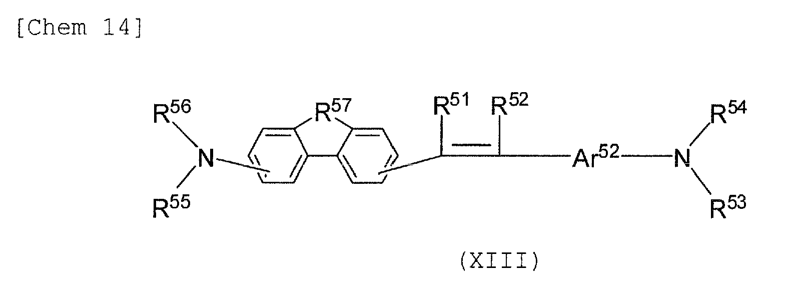

In the formula:
R⁵¹ and R⁵² each independently have the same meaning as that of each of R¹ and R²;
R⁵³ to R⁵⁶ each independently have the same meaning as that of each of R³ to R⁶;
R⁵⁷ represents -O-, -S-, -SO₂-, -SiR¹⁵¹R¹⁵²-, or -NR¹⁵³- where R¹⁵¹, R¹⁵², and R¹⁵³ each independently represent a substituted or unsubstitutedalkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substituted or unsubstituted alkyl group having 1 to 10 carbon atoms, or a substituted or unsubstituted aryl group having 5 to 10 carbon atoms;
Ar⁵² represents a substituted or unsubstituted fused ring group having 10 to 50 carbon atoms, preferably a substituted or unsubstituted fused ring group having 10 to 20 carbon atoms, or more preferably a substituted or unsubstituted fused ring group having 10 to 14 carbon atoms; and
R⁵¹ and R⁵² may be linked to each other to form a ring except an aromatic ring.
Examples of the alkyl group and the aryl group each represented by any one of R¹⁵¹ to R¹⁵³ include the same examples as those described for R¹ to R⁶, and examples of the fused ring group represented by Ar⁵² include the same examples as those described for Ar¹¹ and Ar¹².

General formula (XIV)

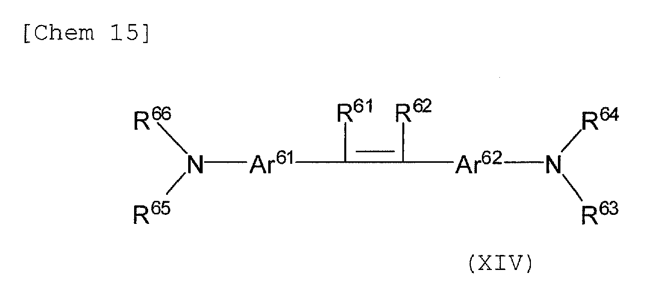

In the formula:
R⁶¹ and R⁶² each independently have the same meaning as that of each of R¹ and R²;
R⁶³ to R⁶⁶ each independently have the same meaning as that of each of R³ to R⁶;
Ar⁶¹ represents a substituted or unsubstituted fused ring group having 10 to 50 carbon atoms, and preferred examples thereof include a 1,4-naphthalene group, a 2,6-naphthalene group, a 2,7-naphthalene group, a 2,5-naphthalene group, a 1,5-naphthalene group, a 9,10-anthracene group, a 1,4-anthracene group, a 2,6-anthracene group, a 6,12-chrysene group, a 9,10-phenanthrene group, a 1,6-pyrene group, a 2,7-pyrene group, a 1,8-pyrene group, a 7,12-benzanthracene group, a 7,10-fluoranthene group, and a 7,12-benzo[k]fluoranthene group, all of which are divalent and substituted or unsubstituted groups, and more preferred examples thereof include a 1,4-naphthalene group, a 2,6-naphthalene group, a 2,7-naphthalene group, a 9,10-anthracene group, a 2, 6-anthracene group, a 6,12-chrysene group, a 1, 6-pyrene group, a 2, 7-pyrene group, a 1,8-pyrene group, a 7,12-benzanthracene group, a 1,5-anthracene group, a 1, 4-triphenylene group, and a 2, 7-triphenylene group, all of which are divalent and substituted or unsubstituted groups;
Ar⁶² represents a substituted or unsubstituted phenylene group, or preferably a 1,4-phenylene group; and
R⁶¹ and R⁶² may be linked to each other to form a ring except an aromatic ring.

General formula (XV)

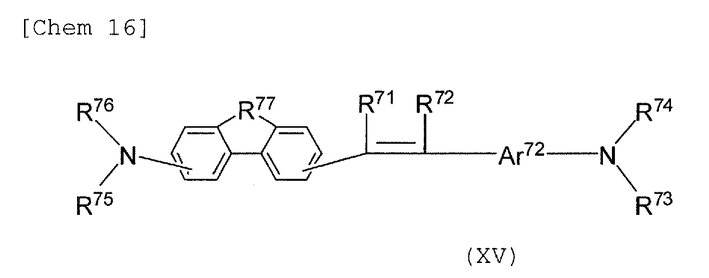

In the formula:
R⁷¹ and R⁷² each independently have the same meaning as that of each of R¹ and R²;
R⁷³ to R⁷⁶ each independently have the same meaning as that of each of R³ to R⁶;
R⁷⁷ represents -O-, -S-, -SO₂-, -SiR¹⁷¹R¹⁷²-, or -NR¹⁷³- where R¹⁷¹, R¹⁷², and R¹⁷³ each independently represent a substituted or unsubstituted alkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substituted or unsubstituted alkyl group having 1 to 10 carbon atoms, or a substituted or unsubstituted aryl group having 5 to 10 carbon atoms;
Ar⁷² represents a substituted or unsubstituted phenylene group, or preferably a 1,4-phenylene group; and
R⁷¹ and R⁷² may be linked to each other to form a ring except an aromatic ring.
Examples of the alkyl group and the aryl group each represented by any one of R¹⁷¹ to R¹⁷³ include the same examples as those described for R¹ to R⁶.

General formula (XVI)

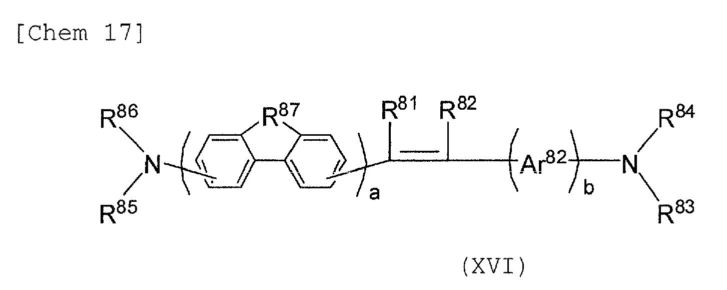

In the formula:
R⁸¹ and R⁸² each independently have the same meaning as that of each of R¹ and R²;
R⁸³ to R⁸⁶ each independently have the same meaning as that of each of R³ to R⁶;
R⁸⁷ represents a substituted or unsubstituted alkylene group having 1 to 50 carbon atoms, preferably an alkylene group having 1 to 20 carbon atoms, ormore preferably a substituted or unsubstituted alkylene group having 1 to 5 carbon atoms, -O-, -S-, -SO₂-, -SiR¹⁸¹R¹⁸²-, or -NR¹⁸³- where R¹⁸¹, R¹⁸², and R¹⁸³ each independently represent a substituted or unsubstituted alkyl group having 1 to 50 carbon atoms, or a substituted or unsubstituted aryl group having 5 to 50 carbon atoms, preferably a substituted or unsubstituted alkyl group having 1 to 20 carbon atoms, or a substituted or unsubstituted aryl group having 5 to 20 carbon atoms, or more preferably a substituted or unsubstituted alkyl group having 1 to 10 carbon atoms, or a substituted or unsubstituted aryl group having 5 to 10 carbon atoms;
Ar⁸² represents a substituted or unsubstituted arylene group having 5 to 50 carbon atoms, or a substituted or unsubstituted, divalent heterocyclic group having 5 to 50 carbon atoms;
a represents an integer of 1 to 3, or preferably 1, and b represents an integer of 2 or 3; and
R⁷¹ and R⁷² may be linked to each other to form a ring except an aromatic ring.
Examples of the alkylene group represented by R⁸⁷ include examples obtained by making the examples of the alkyl group described for R¹ to R⁶ divalent.
Examples of the alkyl group and the aryl group each represented by any one of R¹⁸¹ to R¹⁸³ include the same examples as those described for R¹ to R⁶, and examples of the arylene group and the heterocyclic group each represented by Ar⁵² include examples obtained by making the examples of the aryl group and the heterocyclic group described for R¹ to R⁶ divalent.

General formula (XVII)

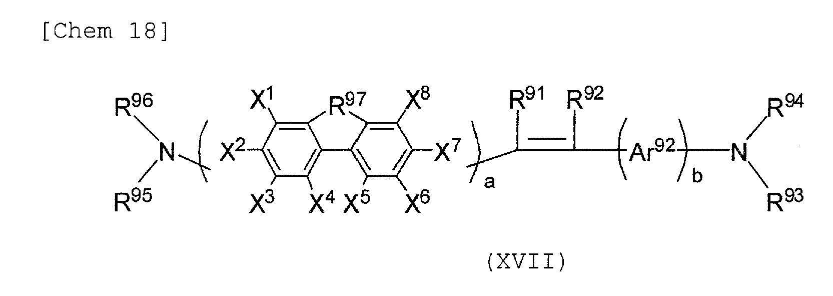

In the formula:
R⁹¹ and R⁹² each independently have the same meaning as that of each of R¹ and R²;
R⁹³ to R⁹⁶ each independently have the same meaning as that of each of R³ to R⁶;
R⁹⁷ represents a substituted or unsubstituted alkylene group having 1 to 50 carbon atoms, preferably an alkylene group having 1 to 20 carbon atoms, ormorepreferablya substitutedorunsubstituted alkylene group having 1 to 5 carbon atoms, -O-, -S-, -SO₂-, -SiR¹⁹¹R¹⁹²-, or -NR¹⁹³- where R¹⁹¹, R¹⁹², and R¹⁹³ each independently represent a substituted or unsubstituted alkyl group having 1 to 50 carbon atoms, or a substituted or unsubstituted aryl group having 5 to 50 carbon atoms;
X¹ to X⁸ each independently represent a single bond, a hydrogen atom, an alkyl group having 1 to 4 carbon atoms, a cycloalkyl group having 5 or 6 carbon atoms, or an aryl group having 6 to 16 carbon atoms, provided that groups represented by one or more pairs selected from X¹ and X², X² and X³, X³ and X⁴, X⁵ and X⁶, X⁶ and X⁷, and X⁷ and X⁸ are linked to each other to form unsaturated rings, and the unsaturated rings may each independently have a single bond, a hydrogen atom, an alkyl group having 1 to 4 carbon atoms, a cycloalkyl group having 5 or 6 carbon atoms, or an aryl group having 6 to 16 carbon atoms;
Ar⁹² represents a substituted or unsubstituted arylene group having 5 to 50 carbon atoms, or a substituted or unsubstituted, divalent heterocyclic group having 5 to 50 carbon atoms;
a represents an integer of 1 to 3, or preferably 1, and b represents an integer of 1 to 3, or preferably 1 or 2; and
R⁹¹ and R⁹² may be linked to each other to form a ring except an aromatic ring.
Examples of the alkylene group represented by R⁹⁷ include examples obtained by making the examples of the alkyl group described for R¹ to R⁶ divalent.
Examples of the alkyl group and the aryl group each represented by any one of R¹⁹¹ to R¹⁹³ include the same examples as those described for R¹ to R⁶, and examples of the arylene group and the heterocyclic group each represented by Ar⁹² include examples obtained by making the examples of the aryl group and the heterocyclic group described for R¹ to R⁶ divalent.

General formula (XVIII)

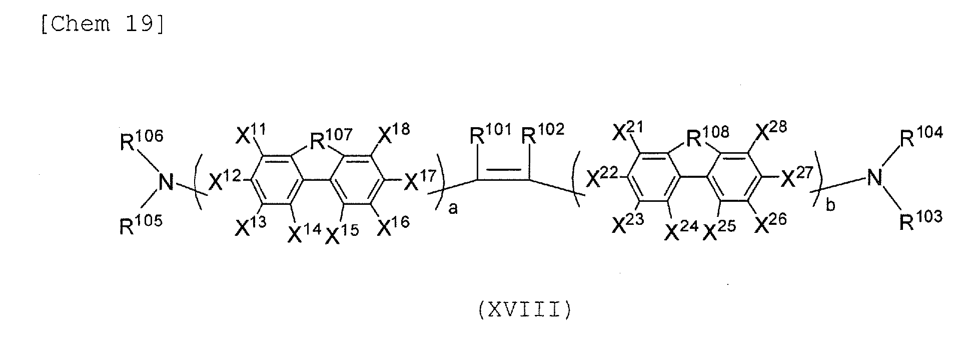

In the formula:
R¹⁰¹ and R¹⁰² each independently have the same meaning as that of each of R¹ and R²;
R¹⁰³ to R¹⁰⁶ each independently have the same meaning as that of each of R³ to R⁶;
R¹⁰⁷ and R¹⁰⁸ each independently represent a substituted or unsubstituted alkylene group having 1 to 50 carbon atoms, preferably an alkylene group having 1 to 20 carbon atoms, or more preferably a substituted or unsubstituted alkylene group having 1 to 5 carbon atoms, -O-, -S-, -SO₂-, -SiR¹⁹¹R¹⁹²-, or -NR¹⁹³- where R¹⁹¹, R¹⁹², and R¹⁹³ each independently represent a substituted or unsubstituted alkyl group having 1 to 50 carbon atoms, or a substituted or unsubstituted aryl group having 5 to 50 carbon atoms;
X¹¹ to X¹⁸ and X²¹ to X²⁸ each independently represent a single bond, a hydrogen atom, an alkyl group having 1 to 4 carbon atoms, a cycloalkyl group having 5 or 6 carbon atoms, or an aryl group having 6 to 16 carbon atoms, provided that groups represented by one or more pairs selected from X¹¹ and X¹², X¹² and X¹³, X¹³ and X¹⁴, X¹⁵ and X¹⁶, X¹⁶ and X¹⁷, X¹⁷ and X¹⁸, X²¹ and X²², X²² and X²³, X²³ and X²⁴, X²⁵ and X²⁶, X²⁶ and X²⁷, and X²⁷ and X²⁸ are linked to each other to form unsaturated rings, and the unsaturated rings may each independently have a single bond, a hydrogen atom, an alkyl group having 1 to 4 carbon atoms, a cycloalkyl group having 5 or 6 carbon atoms, or an aryl group having 6 to 16 carbon atoms;
a represents an integer of 1 to 3, or preferably 1, and b represents an integer of 1 to 3, or preferably 1; and
R¹⁰¹ and R¹⁰² may be linked to each other to form a ring except an aromatic ring.
Examples of the alkylene group represented by R¹⁰⁷ and R¹⁰⁸ include examples obtained by making the examples of the alkyl group described for R¹ to R⁶ divalent.
Examples of the alkyl group and the aryl group each represented by any one of R¹⁹¹ to R¹⁹³ include the same examples as those described for R¹ to R⁶.

R¹³ to R¹⁶, R²³ to R²⁶, R³³ to R³⁶, R⁴³ to R⁴⁶, R⁵³ to R⁵⁶, R⁶³ to R⁶⁶, R⁷³ to R⁷⁶, R⁸³ to R⁸⁶, R⁹³ to R⁹⁶, and R¹⁰³ to R¹⁰⁶ in the general formulae (II) to (XVIII) each preferably represent an unsubstituted phenyl group.

Examples of the substituent further substituting for each group in each of the general formulae (I) to (XVIII) include: an alkyl group (having preferably 1 to 30, more preferably 1 to 20, or particularly preferably 1 to 10 carbon atoms such as methyl, ethyl, isopropyl, t-butyl, n-octyl, n-decyl, or n-hexadecyl); a cycloalkyl group (having preferably 3 to 30, more preferably 3 to 20, or particularly preferably 3 to 10 carbon atoms, such as cyclopropyl, cyclopentyl, or cyclohexyl); an alkenyl group (having preferably 2 to 30, morepreferably2 to 20, orparticularlypreferably 2 to 10 carbon atoms, such as vinyl, allyl, 2-butenyl, or 3-pentenyl) ; an alkynyl group (having preferably 2 to 30, more preferably 2 to 20, or particularly preferably 2 to 10 carbon atoms, such as propargyl or 3-pentynyl), an aryl group (having preferably 6 to 30, more preferably 6 to 20, or particularly preferably 6 to 12 carbon atoms, such as phenyl, p-methylphenyl, naphthyl, or anthranyl); an amino group (having preferably 0 to 30, more preferably 0 to 20, or particularly preferably 0 to 10 carbon atoms, such as amino, methylamino, dimethylamino, diethylamino, dibenzylamino, diphenylamino, or ditolylamino); an alkoxy group (having preferably 1 to 30, more preferably 1 to 20, or particularly preferably 1 to 10 carbon atoms, such as methoxy, ethoxy, butoxy, or 2-ethylhexyloxy) ; an aryloxy group (having preferably 6 to 30, more preferably 6 to 20, or particularly preferably 6 to 12 carbon atoms, such as phenyloxy, 1-naphthyloxy, or 2-naphthyloxy); an heteroaryloxy group (having preferably 1 to 30, more preferably 1 to 20, or particularly preferably 1 to 12 carbon atoms, such as pyridyloxy, pyrazyloxy, pyrimidyloxy, or quinolyloxy) ; an acyl group (having preferably 1 to 30, more preferably 1 to 20, or particularly preferably 1 to 12 carbon atoms, such as acetyl, benzoyl, formyl, or pivaloyl); an alkoxycarbonyl group (having preferably 2 to 30, more preferably 2 to 20, or particularly preferably 2 to 12 carbon atoms, such as methoxycarbonyl or ethoxycarbonyl); an aryloxycarbonyl group (having preferably 7 to 30, more preferably 7 to 20, or particularly preferably 7 to 12 carbon atoms, such as phenyloxycarbonyl); an acyloxy group (having preferably 2 to 30, more preferably 2 to 20, or particularly preferably 2 to 10 carbon atoms, such as acetoxy or benzoyloxy); an acylamino group (having preferably 2 to 30, more preferably 2 to 20, orparticularlypreferably 2 to 10 carbon atoms, such as acetylamino or benzoylamino); an alkoxycarbonylamino group (having preferably 2 to 30, more preferably 2 to 20, or particularly preferably 2 to 12 carbon atoms, such as methoxycarbonylamino); an aryloxycarbonylamino group (having preferably 7 to 30, more preferably 7 to 20, or particularly preferably 7 to 12 carbon atoms, such as phenyloxycarbonylamino) ; a sulfonylamino group (having preferably 1 to 30, more preferably 1 to 20, or particularly preferably 1 to 12 carbon atoms, such as methanesulfonylamino or benzenesulfonylamino); a sulfamoyl group (having preferably 0 to 30, more preferably 0 to 20, or particularly preferably 0 to 12 carbon atoms, such as sulfamoyl, methylsulfamoyl, dimethylsulfamoyl, or phenylsulfamoyl); a carbamoyl group (having preferably 1 to 30, more preferably 1 to 20, orparticularlypreferably 1 to 12 carbon atoms, such as carbamoyl, methylcarbamoyl, diethylcarbamoyl, or phenylcarbamoyl); an alkylthio group (having preferably 1 to 30, more preferably 1 to 20, orparticularlypreferably 1 to 12 carbon atoms, such as methylthio or ethylthio) ; an arylthio group (having preferably 6 to 30, more preferably 6 to 20, or particularly preferably 6 to 12 carbon atoms, such as phenylthio) ; a heteroarylthio group (having preferably 1 to 30, more preferably 1 to 20, or particularly preferably 1 to 12 carbon atoms, such as pyridylthio, 2-benzimizolylthio, 2-benzoxazolylthio, or 2-benzthiazolylthio); a sulfonyl group (having preferably 1 to 30, more preferably 1 to 20, or particularly preferably 1 to 12 carbon atoms, such as mesyl or tosyl) ; a sulfinyl group (having preferably 1 to 30, more preferably 1 to 20, or particularly preferably 1 to 12 carbon atoms, such as methanesulfinyl or benzenesulfinyl); a ureido group (having preferably 1 to 30, more preferably 1 to 20, or particularly preferably 1 to 12 carbon atoms, such as ureido, methylureido, or phenylureido); a phosphoric acid amide group (having preferably 1 to 30, more preferably 1 to 20, or particularly preferably 1 to 12 carbon atoms, such as diethylphosphoric acid amide or phenylphosphoric acid amide) ; a hydroxyl group; a mercapto group; a halogen atom (such as a fluorine atom, a chlorine atom, a bromine atom, or an iodine atom); a cyano group; a sulfo group; a carboxyl group; a nitro group; a hydroxamic acid group; a sulfino group; a hydrazino group; an imino group; a heterocyclic group (having preferably 1 to 30 or more preferably 1 to 12 carbon atoms and containing, as a hetero atom, for example, a nitrogen atom, an oxygen atom, or a sulfur atom, and specific examples include imidazolyl, pyridyl, quinolyl, furyl, thienyl, piperidyl, morpholino, benzoxazolyl, benzimidazolyl, and benzthiazolyl); and a silyl group (having preferably 3 to 40, more preferably 3 to 30, or particularly preferably 3 to 24 carbon atoms, such as trimethylsilyl or triphenylsilyl). Each of those substituents may be additionally substituted.

Specific examples of the aromatic amine derivative represented by the general formula (I) of the present invention are shown below and in synthesis examples. However, the present invention is not limited to those exemplified compounds.

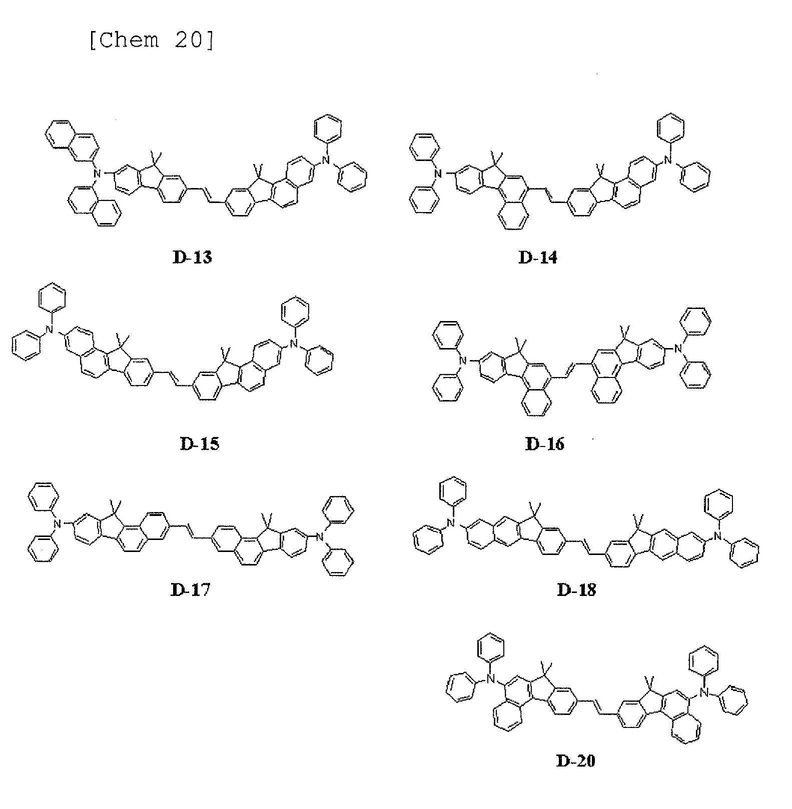

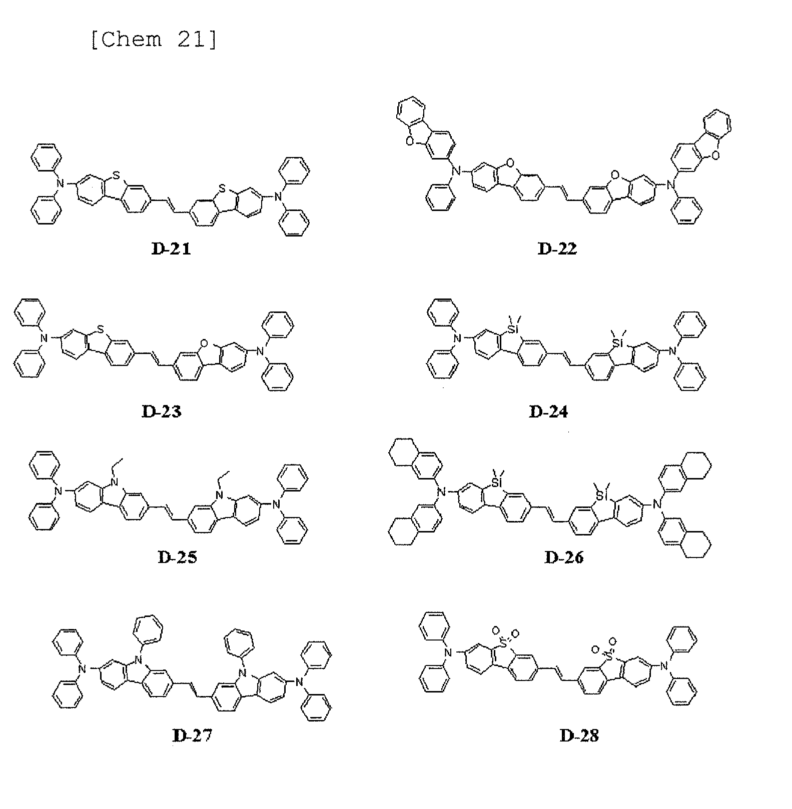

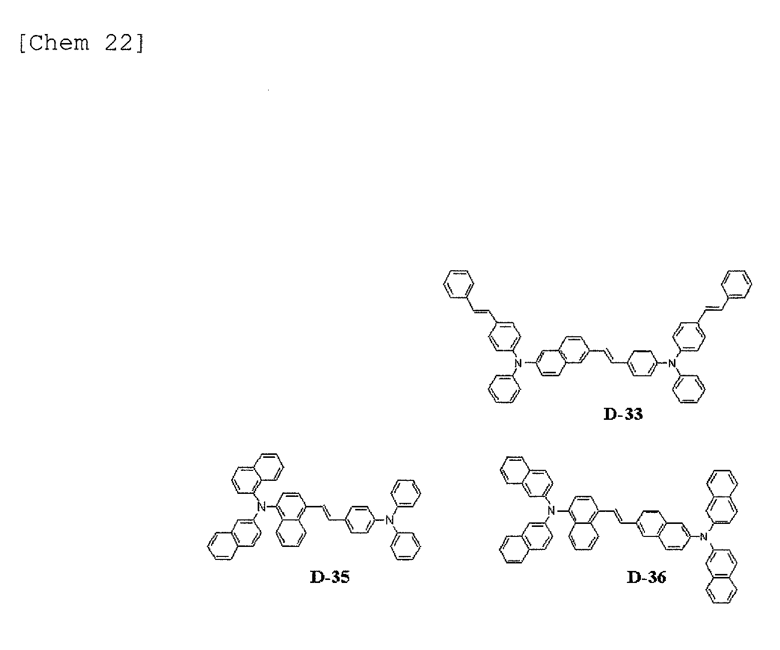

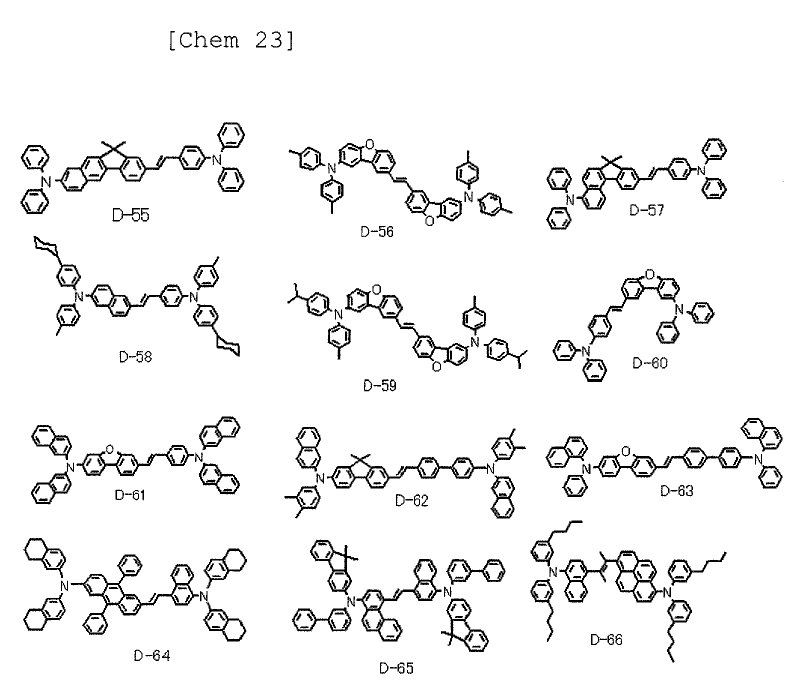

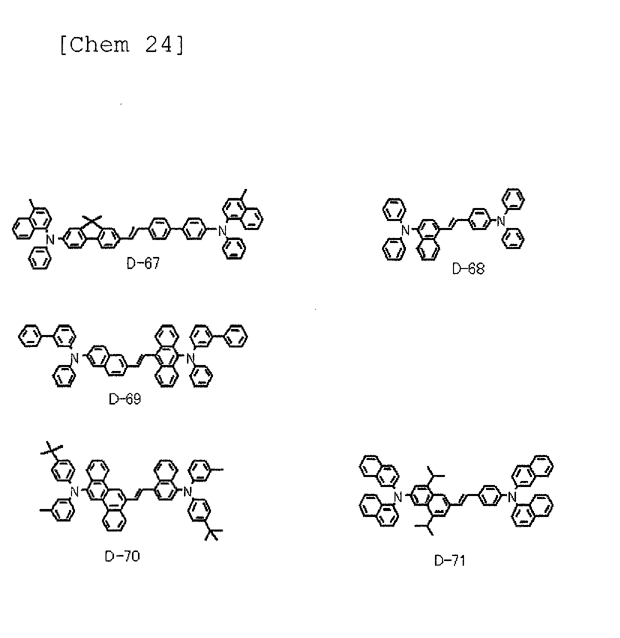

Next, a method of producing the aromatic amine derivative of the present invention is described.
The method of producing the aromatic amine derivative represented by the general formula (I) of the present invention is not particularly limited, and the derivative has only to be produced by a known method. For example, the derivative is produced by a coupling reaction between an amine derivative and an aromatic halide with a copper catalyst described in Tetrahedron 40 (1984), 1435 to 1456 or a palladium catalyst described in Journal of American Chemical Society 123 (2001), 7727 to 7729.

The aromatic amine derivative of the present invention is preferably used as a material for an organic EL device, and is more preferably used as a light emitting material for an organic EL device, in particular, a doping material for an organic EL device.
The organic EL device of the present invention is an organic electroluminescence device having an organic compound layer formed of one or more layers including at least a light emitting layer and interposed between a pair of electrodes, in which at least one layer of the organic compound layer contains at least one kind of aromatic amine derivative of the present invention.
In the organic EL device of the present invention, the light emitting layer preferably contains at least one kind of aromatic amine derivative, and the content of the aromatic amine derivative of the present invention in the light emitting layer is preferably 0. 01 to 20 wt%, more preferably 0.5 to 20 wt%, particularly preferably 1 to 20 wt%, or most preferably 5 to 20 wt%.
In addition, when the aromatic amine derivative of the present invention is used as a light emitting material of an organic EL device, the light emitting layer preferably contains at least one kind of aromatic amine derivative and at least one kind of compound selected from the compounds represented by the following general formulae (2a) to (2d), and the at least one kind of compound selected from the compounds represented by the following general formulae (2a) to (2d) is preferably a host material.

Hereinafter, the general formulae (2a) to (2d) are described.
General formula (2a)

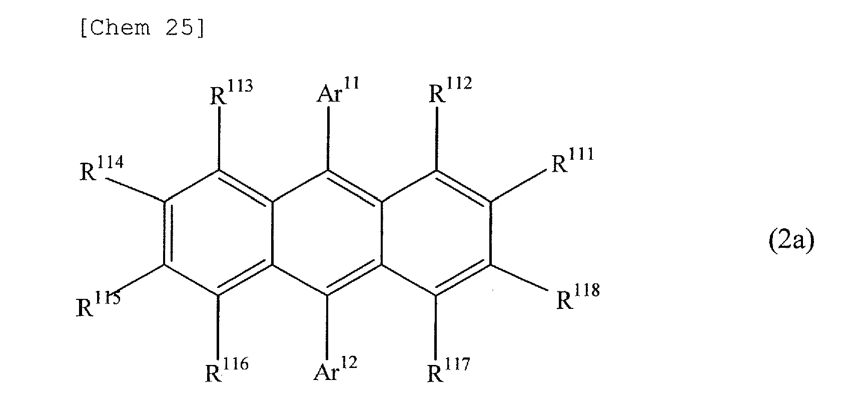

In the formula (2a), Ar¹¹ and Ar¹² each independently represent a group derived from a substituted or unsubstituted aromatic ring having 6 to 20 carbon atoms, and the aromatic ring may be substituted by one or two or more substituents. The substituent of the aromatic ring is selected from a substituted or unsubstituted aryl group having 6 to 50 carbon atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group (the aryl portion having 6 to 50 carbon atoms and the alkyl portion having 1 to 5 carbon atoms), a substituted or unsubstituted aryloxy group having 6 to 50 carbon atoms, a substituted or unsubstituted arylthio group having 6 to 50 carbon atoms, a substituted or unsubstituted alkoxycarbonyl group (the alkoxy portion having 1 to 50 carbon atoms), a substituted or unsubstituted silyl group, a carboxyl group, a halogen atom, a cyano group, a nitro group, and a hydroxyl group, and specific examples of any one of R¹¹¹ to R¹¹⁸ are selected from the groups described below. When the aromatic ring is substituted by two or more substituents, the substituents may be identical to or different from each other, and adjacent substituents may be bonded to each other to form a saturated or unsaturated cyclic structure. Ar¹¹ and Ar¹² are preferably different from each other. In addition, at least one of Ar¹¹ and Ar¹² preferably represents a substituent having a substituted or unsubstituted fused ring group having 10 to 30 carbon atoms, or more preferably represents a substituent having a substituted or unsubstituted naphthyl group.

Examples of the group derived from a substituted or unsubstituted aromatic ring having 6 to 20 carbon atoms represented by any one of Ar¹¹ and Ar¹² include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, and a 4"-t-butyl-p-terphenyl-4-yl group. A group derived from a substituted or unsubstituted aromatic ring having 10 to 14 ring carbon atoms is preferred. In particular, a 1-naphthyl group, a 2-naphthyl group, and a 9-phenanthryl group are preferred.

R¹¹¹ to R¹¹⁸ are each independently selected from a hydrogen atom, a substituted or unsubstituted aryl group having 6 to 50 carbon atoms, a substituted or unsubstituted heteroaryl group having 4 to 50 carbon atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group (the aryl portion having 6 to 50 carbon atoms and the alkyl portion having 1 to 50 carbon atoms), a substituted or unsubstituted aryloxy group having 5 to 50 carbon atoms, a substituted or unsubstituted arylthio group having 5 to 50 carbon atoms, a substituted or unsubstituted alkoxycarbonyl group (the alkyl portion having 1 to 50 carbon atoms), a substituted or unsubstituted silyl group, a carboxyl group, a halogen atom, a cyano group, a nitro group, and a hydroxyl group.

Examples of the substituted or unsubstituted aryl group having 6 to 50 carbon atoms represented by any one of R¹¹¹ to R¹¹⁸ in the formula (2a) include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, a 9,9-dimethylfluorene-1-yl group, a 9,9-dimethylfluorene-2-yl group, a 9,9-dimethylfluorene-3-yl group, and a 9,9-dimethylfluorene-4-yl group. Further examples include a substituent which is a combination of a phenyl group, a phenylene group, a naphthyl group, and a napthalene group (such as a phenylnaphthyl group, a naphthylphenyl group, a naphthylnaphthyl group, a naphthylnaphthylnaphthyl group, a phenylphenylnaphthyl group, a naphthylnaphthylphenyl group, a naphthylphenylnaphthyl group, a naphthylphenylphenyl group, a phenylnaphthylnaphthyl group, and a phenylnaphthylphenyl group).

Examples of the substituted or unsubstituted heteroaryl group having 4 to 50 carbon atoms represented by any one of R¹¹¹ to R¹¹⁸ in the formula (2a) include a 1-pyrrolyl group, a 2-pyrrolyl group, a 3-pyrrolyl group, a pyrazinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrolin-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1,10-phenanthrolin-3-yl group, a 1, 10-phenanthrolin-4-yl group, a 1, 10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a 1-phenazinyl group, a 2-phenazinyl group, a 1-phenothiazinyl group, a 2-phenothiazinyl group, a 3-phenothiazinyl group, a 4-phenothiazinyl group, a 10-phenothiazinyl group, a 1-phenoxazinyl group, a 2-phenoxazinyl group, a 3-phenoxazinyl group, a 4-phenoxazinyl group, a 10-phenoxazinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienylgroup, a 2-methylpyrrol-1-ylgroup, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t- butyl-1-indolyl group, a 2-t-butyl-3-indolyl group, and a 4-t- butyl-3-indolyl group.

Examples of the substituted or unsubstituted alkyl group having 1 to 50 carbon atoms represented by any one of R¹¹¹ to R¹¹⁸ in the formula (2a) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1, 2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, and a 1,2,3-trinitropropyl group.

Examples of the substituted or unsubstituted cycloalkyl group having 3 to 50 carbon atoms represented by any one of R¹¹¹ to R¹¹⁸ in the formula (2a) and as the substituent of the above aromatic ring include a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

The substituted or unsubstituted alkoxy group having 1 to 50 carbon atoms represented by any one of R¹¹¹ to R¹¹⁸ in the formula (2a) is a group represented by -OY, and Y is selected from the above substituted or unsubstituted alkyl groups having 1 to 50 carbon atoms represented by any one of R¹¹¹ to R¹¹⁸.

Examples of the substituted or unsubstituted aralkyl group (the aryl portion having 6 to 50 carbon atoms and the alkyl portion having 1 to 50 carbon atoms) as the substituents represented by any one of R¹¹¹ to R¹¹⁸ in the formula (2a) include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, an m-methylbenzyl group, an o-methylbenzyl group, a p-chlorobenzyl group, an m-chlorobenzyl group, an o-chlorobenzyl group, a p-bromobenzyl group, an m-bromobenzyl group, ano-bromobenzylgroup, ap-iodobenzylgroup, an m-iodobenzylgroup, an o-iodobenzyl group, a p-hydroxybenzyl group, an m-hydroxybenzyl group, an o-hydroxybenzyl group, a p-aminobenzyl group, an m-aminobenzyl group, an o-aminobenzyl group, a p-nitrobenzyl group, an m-nitrobenzyl group, an o-nitrobenzyl group, a p-cyanobenzyl group, an m-cyanobenzyl group, an o-cyanobenzyl group, a 1-hydroxy-2-phenylisopropyl group, and a 1-chloro-2-phenylisopropyl group.

The substituted or unsubstituted aryloxy group having 6 to 50 atoms, and the substituted or unsubstituted arylthio group having 6 to 50 atoms represented by any one of R¹¹¹ to R¹¹⁸ in the formula (2a) are represented by -OY' and -SY", respectively. Each of Y' and Y" are selected from the above substituted or unsubstituted aryl groups having 6 to 50 atoms represented by any one of R¹¹¹ to R¹¹⁸.

The substituted or unsubstituted alkoxycarbonyl group (the alkyl portion having 1 to 50 carbon atoms) represented by any one of R¹¹¹ to R¹¹⁸ in the formula (2a) is represented by -COOZ. Z is selected from the above substituted or unsubstituted alkyl groups having 1 to 50 carbon atoms represented by any one of R¹¹¹ to R¹¹⁸

Examples of the substituted silyl group represented by any one of R¹¹¹ to R¹¹⁸ in the formula (2a) include a trimethylsilyl group, a triethylsilyl group, a t-butyldimethylsilyl group, a vinyldimethylsilyl group, a propyldimethylsilyl group, and a triphenylsilyl group.

Examples of the halogen atom represented by any one of R¹¹¹ to R¹¹⁸ in the formula (2a) include fluorine, chlorine, bromine, and iodine.
The substituent of an aromatic ring represented by any one of R¹¹¹ to R¹¹⁸ and/or by any one of Ar¹¹ and Ar¹² may be further substituted by a halogen atom, a hydroxyl group, a nitro group, a cyano group, an alkyl group, an aryl group, a cycloalkyl group, an alkoxy group, an aromatic heterocyclic group, an aralkyl group, an aryloxy group, an arylthio group, an alkoxycarbonyl group, a carboxyl group, or the like.

The anthracene derivative represented by the formula (2a) is preferably a compound having a structure represented by the following formula (2a'),

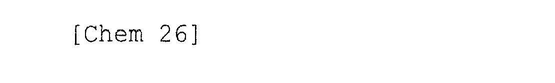

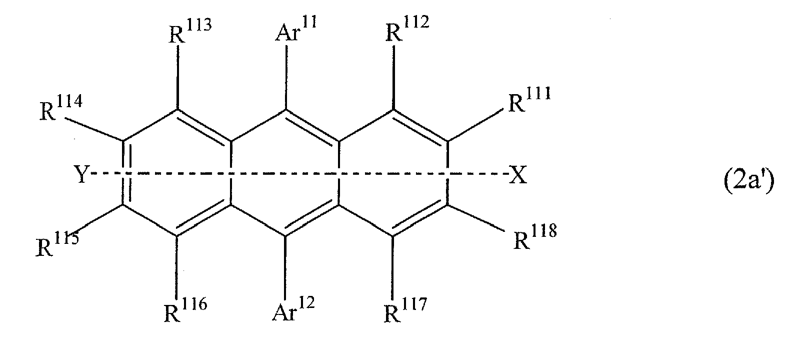

(In the formula (2a'), Ar¹¹ and Ar¹², and R¹¹¹ to R¹¹⁸ are as defined in the formula (2a), provided that the substituents A⁹ and A¹⁰ at the 9- and 10-positions of the anthracene structure are asymmetric with respect to the X-Y axis.)

Specific examples of the anthracene derivative represented by the general formula (2a) to be used in the organic EL device of the present invention include various known anthracene derivatives such as an anthracene derivative having two anthracene skeletons in of its molecule described in paragraphs [0043] to [0063] of JP 2004-356033 A and a compound having one anthracene skeleton described in p. 27 and 28 of WO 2005/061656. Representative specific examples are shown below.

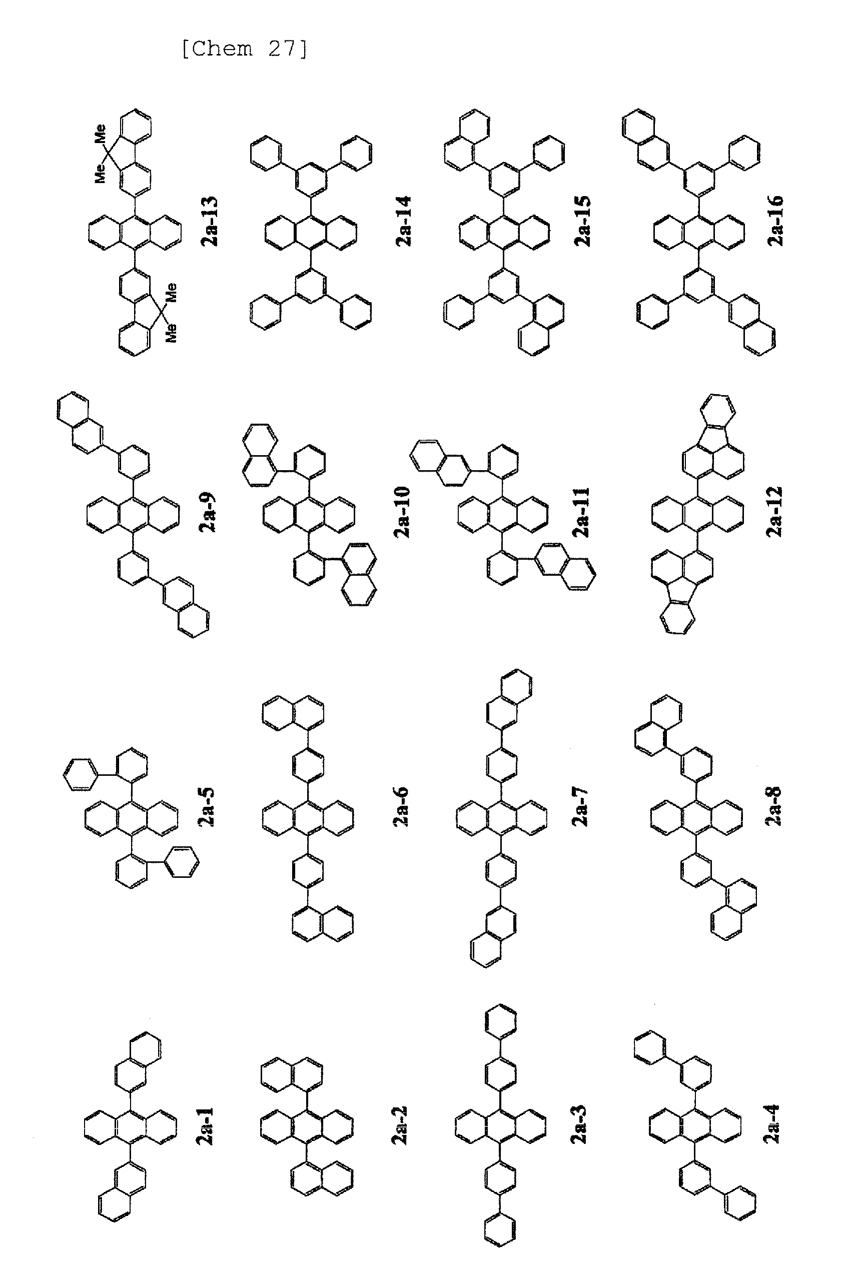

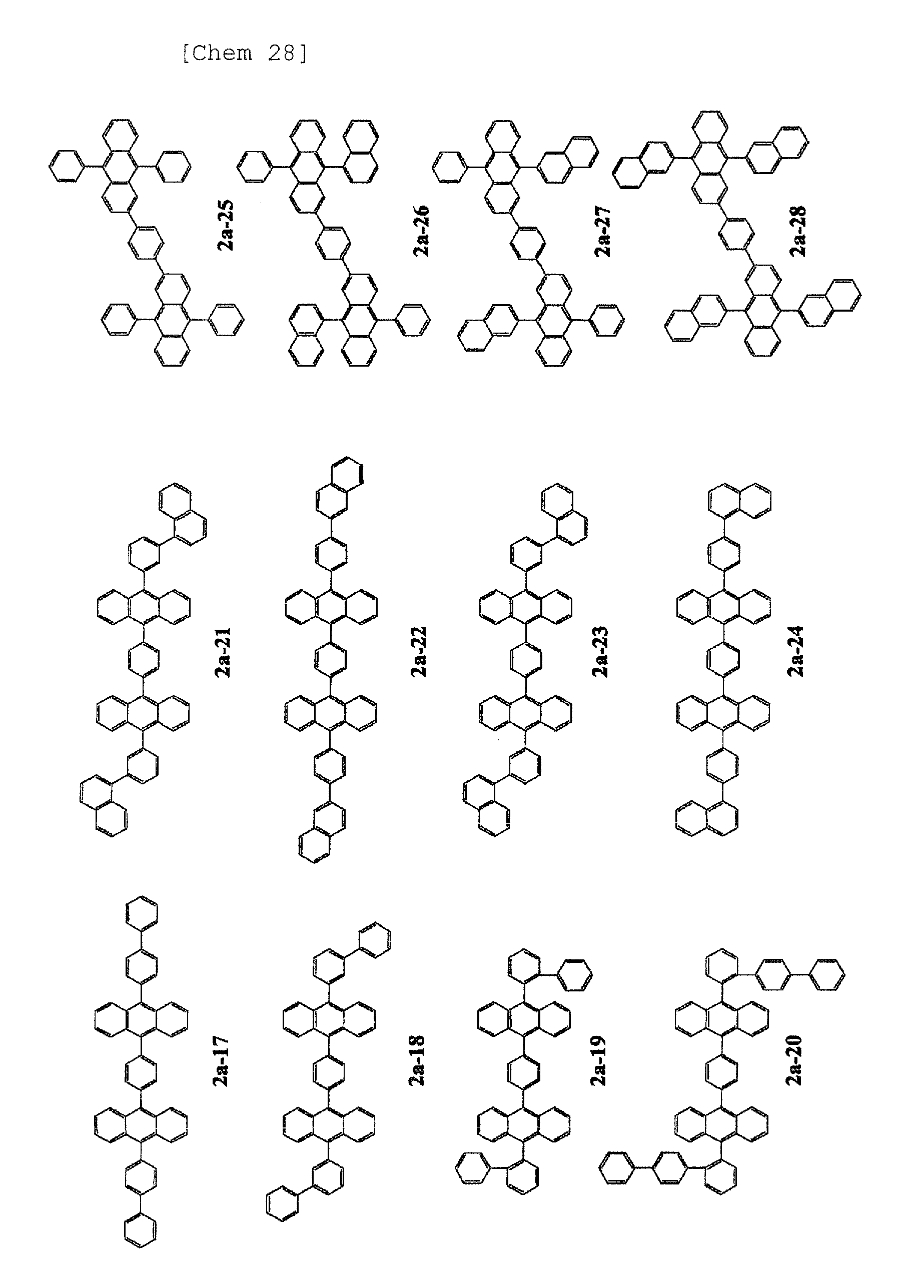

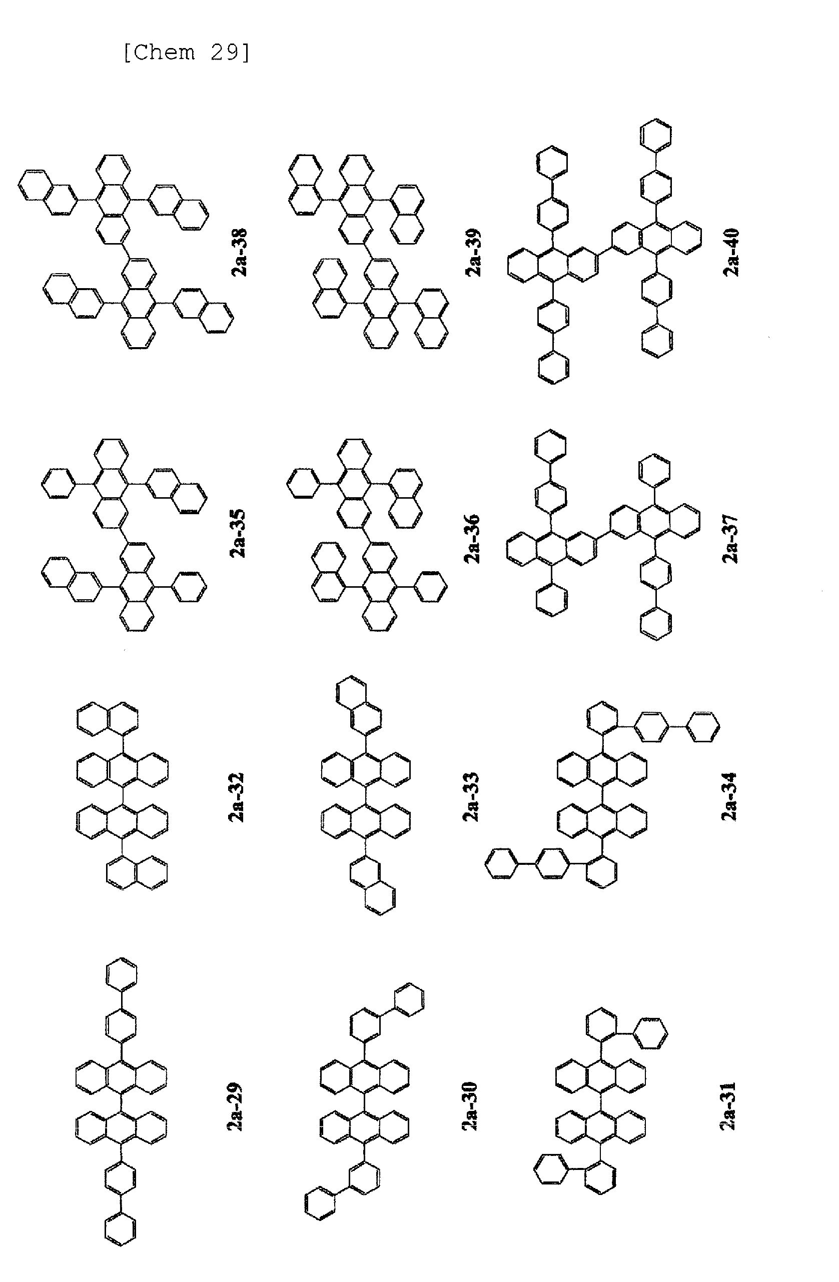

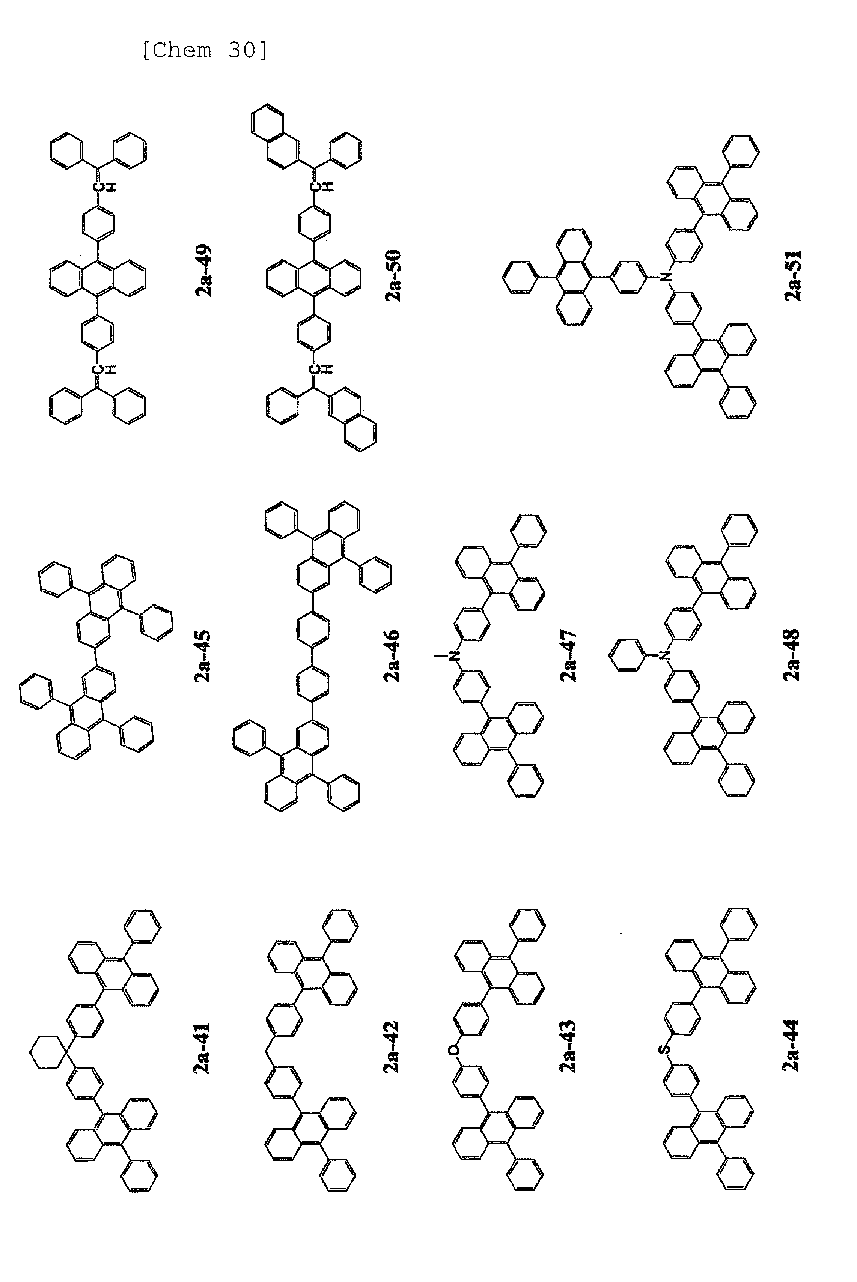

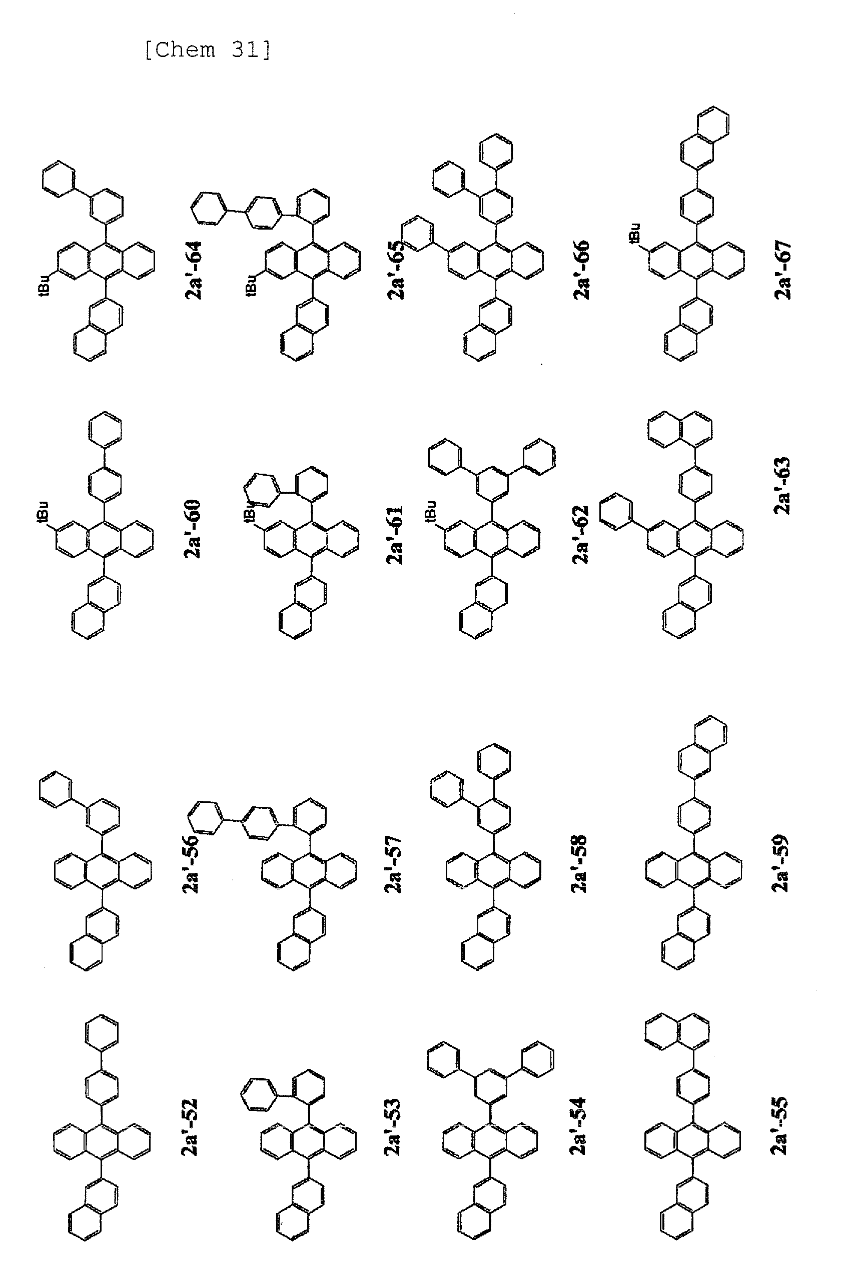

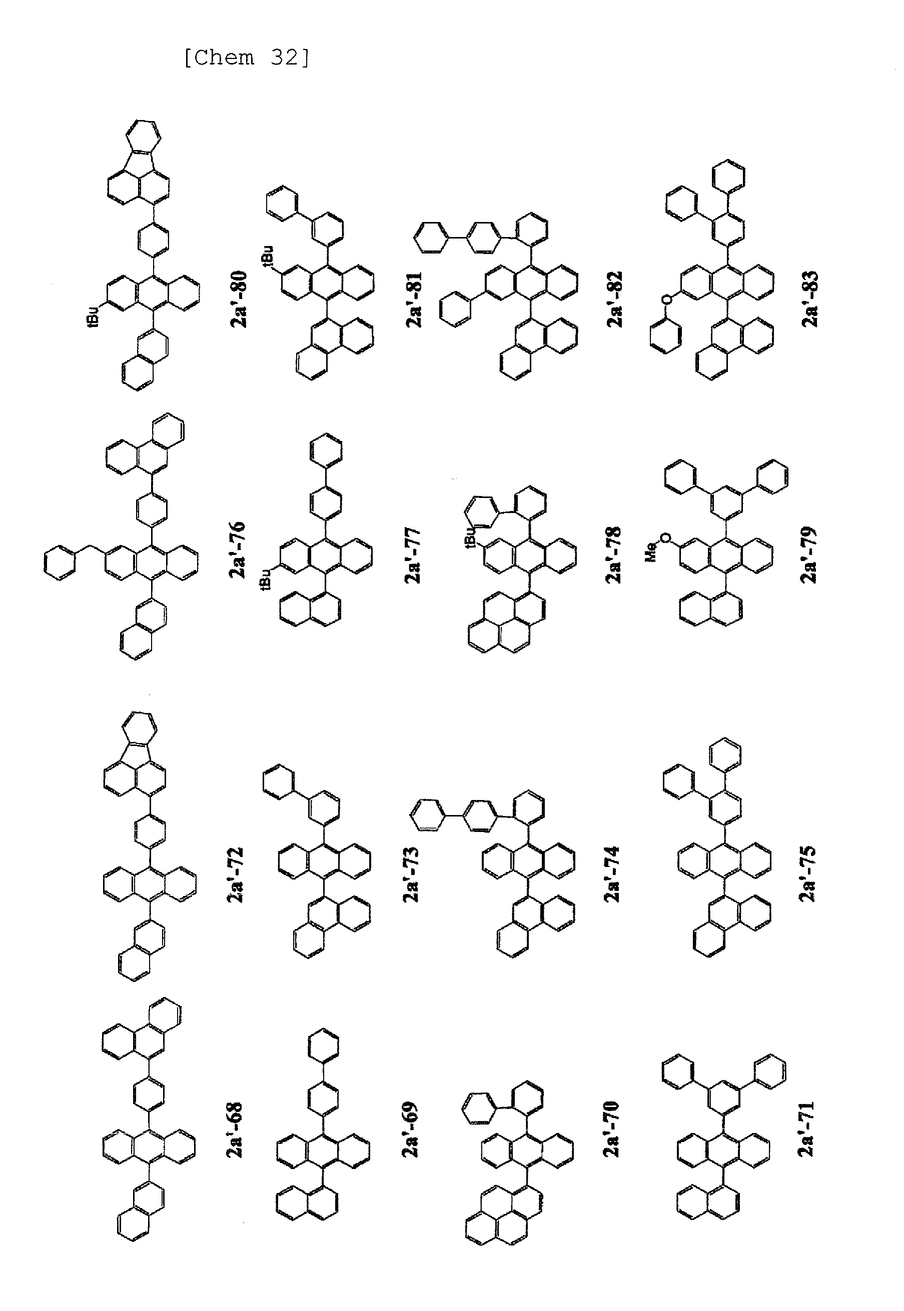

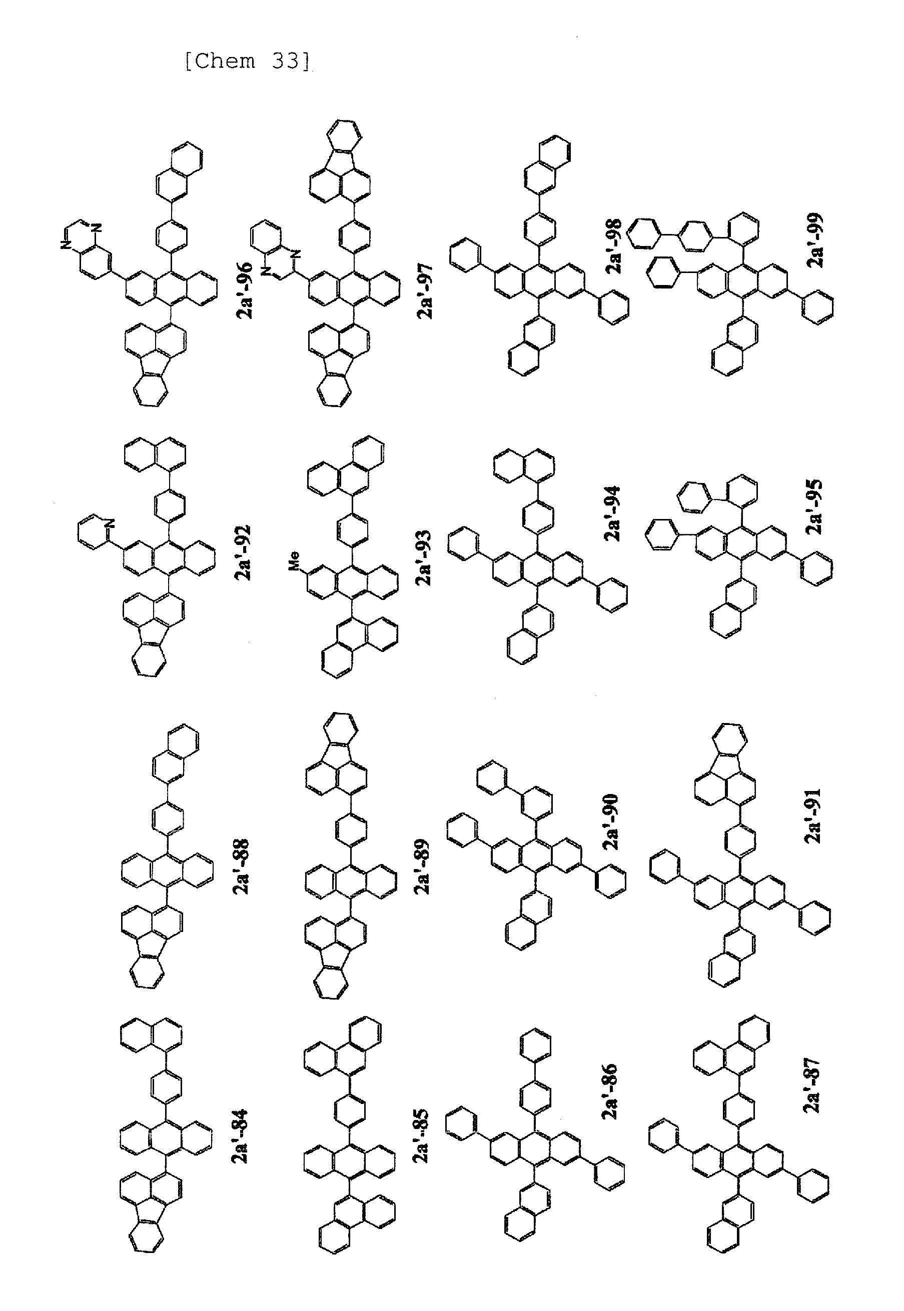

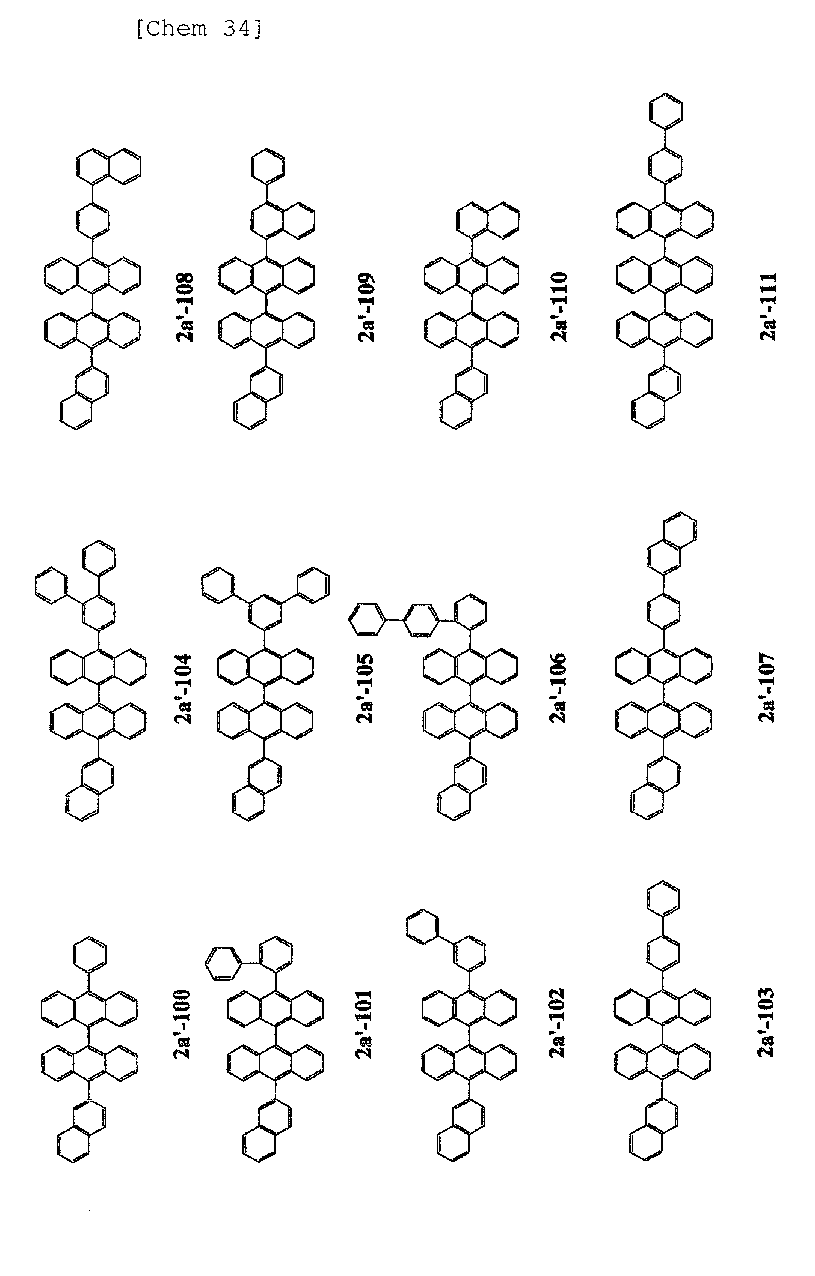

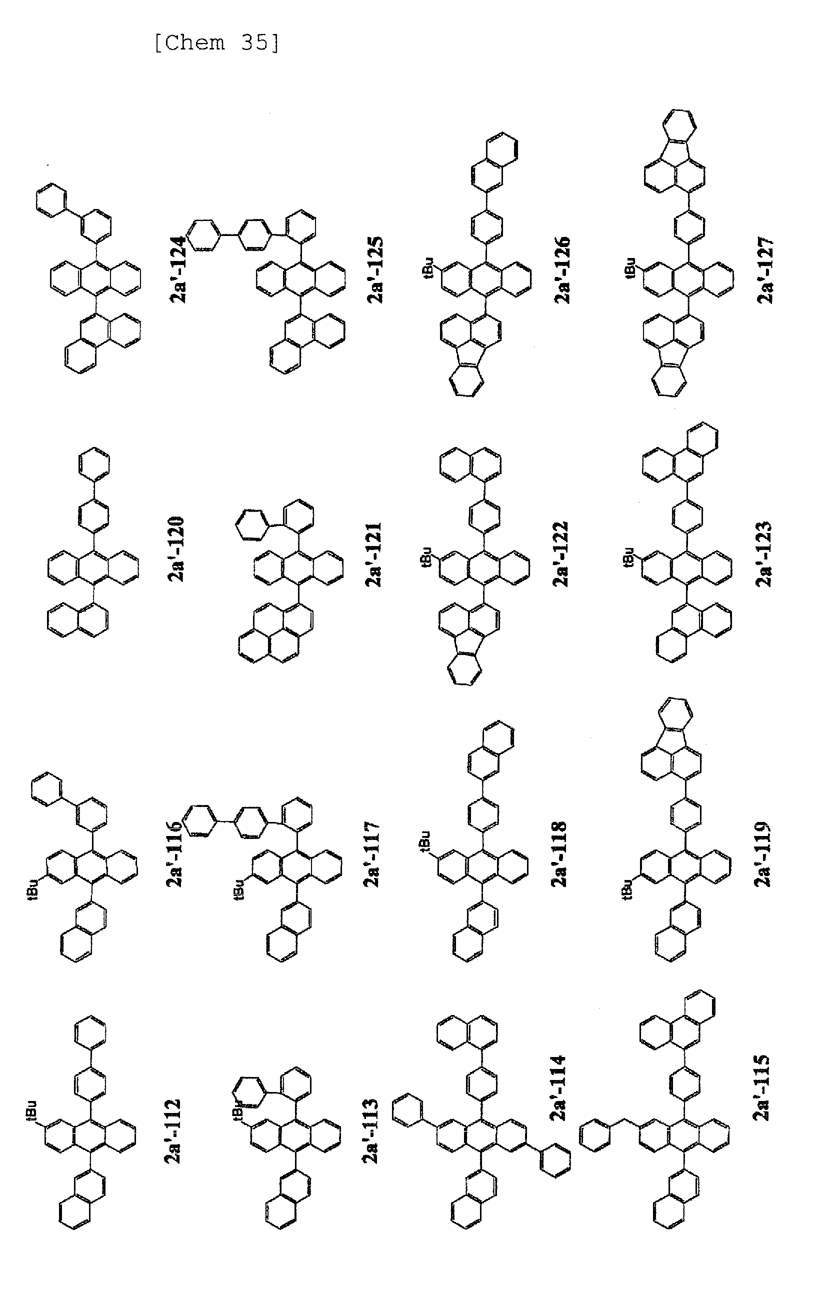

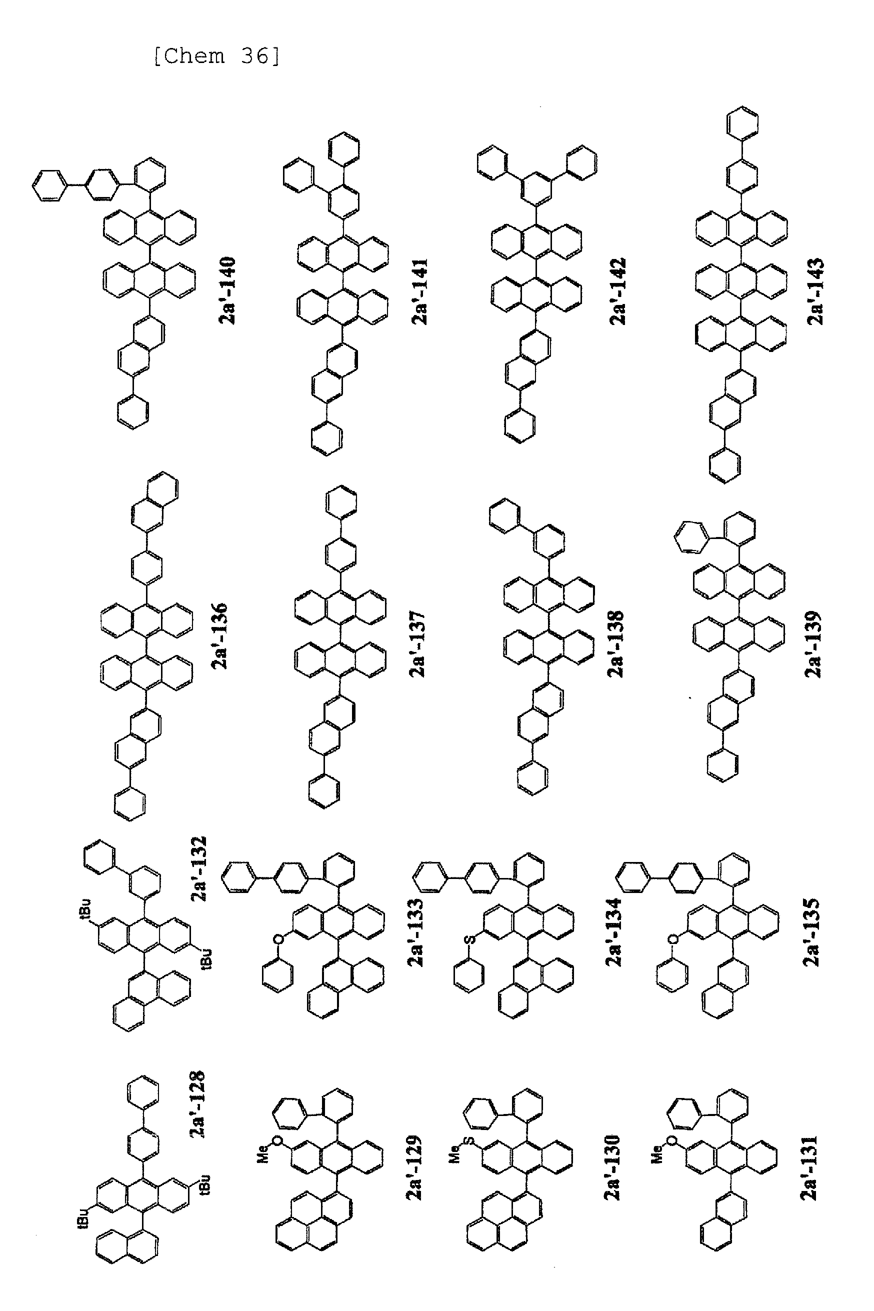

General formula (2b)

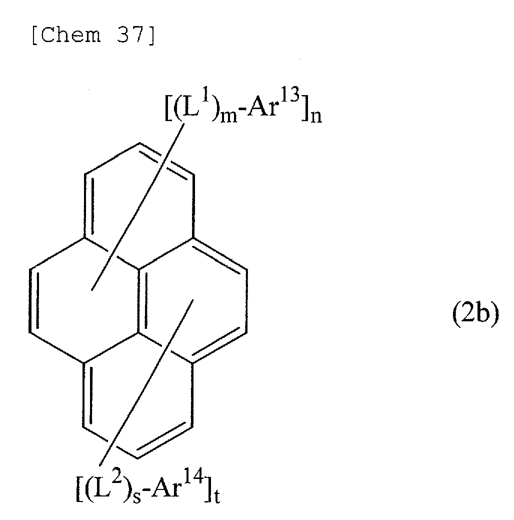

In the formula (2b): Ar¹³ and Ar¹⁴ each independently represent a substituted or unsubstituted aryl group having 6 to 50 ring carbon atoms; L¹ and L² each independently represent a group selected from a substituted or unsubstituted phenylene group, a substituted or unsubstituted naphthalenylene group, a substituted or unsubstituted fluorenylene group, and a substituted or unsubstituted dibenzosilolylene group; m represents an integer of 0 to 2, n represents an integer of 1 to 4, s represents an integer of 0 to 2, and t represents an integer of 0 to 4; and L¹ or Ar¹³ is bonded to any one of 1- to 5-positions of pyrene, and L² or Ar¹⁴ is bonded to any one of 6- to 10-positions of pyrene.

Examples of the aryl group having 6 to 50 ring carbon atoms represented by Ar¹³ and Ar¹⁴ in the formula (2b) include a phenyl group, a 1-naphthyl group, a 2-naphthyl group, a 1-anthryl group, a 2-anthryl group, a 9-anthryl group, a 9-(10-phenyl)anthryl group, a 9-(10-naphthyl-1-yl)anthryl group, a 9-(10-naphthyl-2-yl)anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-3-yl group, a p-terphenyl-2-yl group, an m-terphenyl-4-yl group, an m-terphenyl-3-yl group, anm-terphenyl-2-yl group, an o-tolyl group, an m-tolyl group, a p-tolyl group, a p-t-butylphenyl group, a 3-methyl-2-napthyl group, a 4-methyl-1-napthyl group, and a 4-methyl-1-anthryl group. An aromatic ring group having 6 to 16 ring carbon atoms is preferred. Particularly preferred are a phenyl group, a 1-naphthyl group, a 2-naphthyl group, a 9-(10-phenyl)anthryl group, a 9-(10-naphthyl-1-yl)anthryl group, a 9-(10-naphthyl-2-yl)anthryl group, a 9-phenanthryl group, a 1-pyrenyl group, a 2-pyrenyl group, a 4-pyrenyl group, a 2-biphenylyl group, a 3-biphenylyl group, a 4-biphenylyl group, an o-tolyl group, an m-tolyl group, a p-tolyl group, and a p-t-butylphenyl group.

Further, the aryl group may be further substituted by a substituent. Examples of the substituent include: an alkyl group (such as a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group) ; alkoxy groups each having 1 to 6 carbon atoms (such as an ethoxy group, a methoxy group, an i-propoxy group, an n-propoxy group, an s-butoxy group, a t-butoxy group, a pentoxy group, a hexyloxy group, a cyclopentoxy group, and a cyclohexyloxy group) ; aryl groups each having 6 to 40 atoms; amino groups each substituted by an aryl group having 6 to 40 atoms; ester groups each having an aryl group having 6 to 40 atoms; ester groups each having an alkyl group having 1 to 6 carbon atoms; a cyano group; a nitro group; and a halogen atom.

L¹ and L² in the formula (2b) are each preferably selected from a substituted or unsubstituted phenylene group and a substituted or unsubstituted fluorenylene group. The substituent is selected from the groups exemplified as the substituent for the above aryl group.
m in the formula (2b) preferably represents an integer of 0 or 1. n preferably represents an integer of 1 or 2. s preferably represents an integer of 0 or 1. t preferably represents an integer of 0 to 2.

Specific examples of the pyrene derivative represented by the general formula (2b) to be used in the organic EL device of the present invention include an asymmetric pyrene derivative described in paragraphs [0020] to [0023] of WO 2005/115950. Alternatively, a symmetric pyrene derivative can also be used as a material for the organic EL device of the present invention. Representative specific examples are shown below.

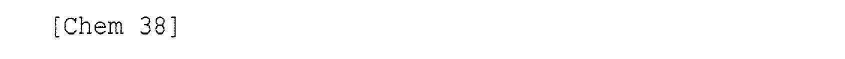

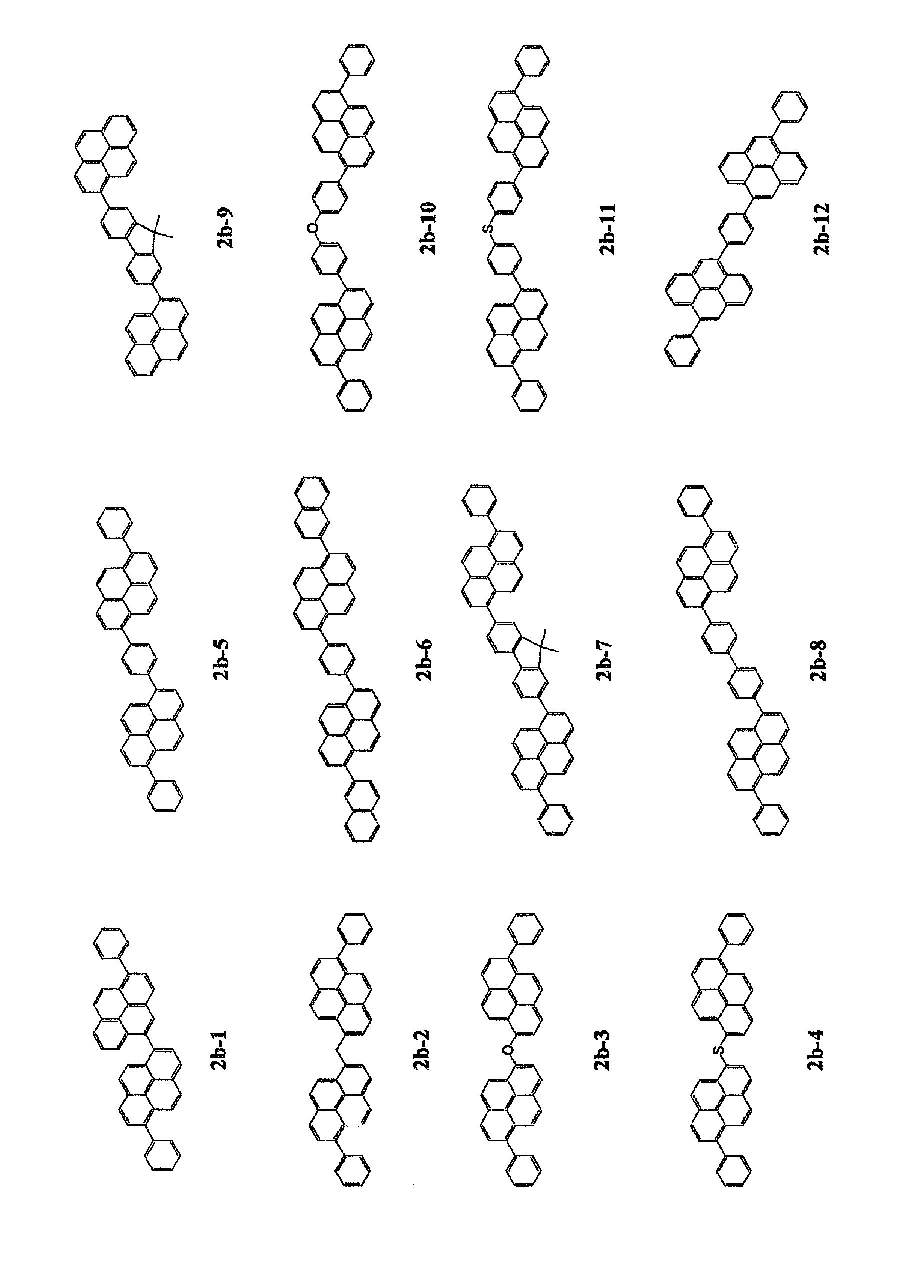

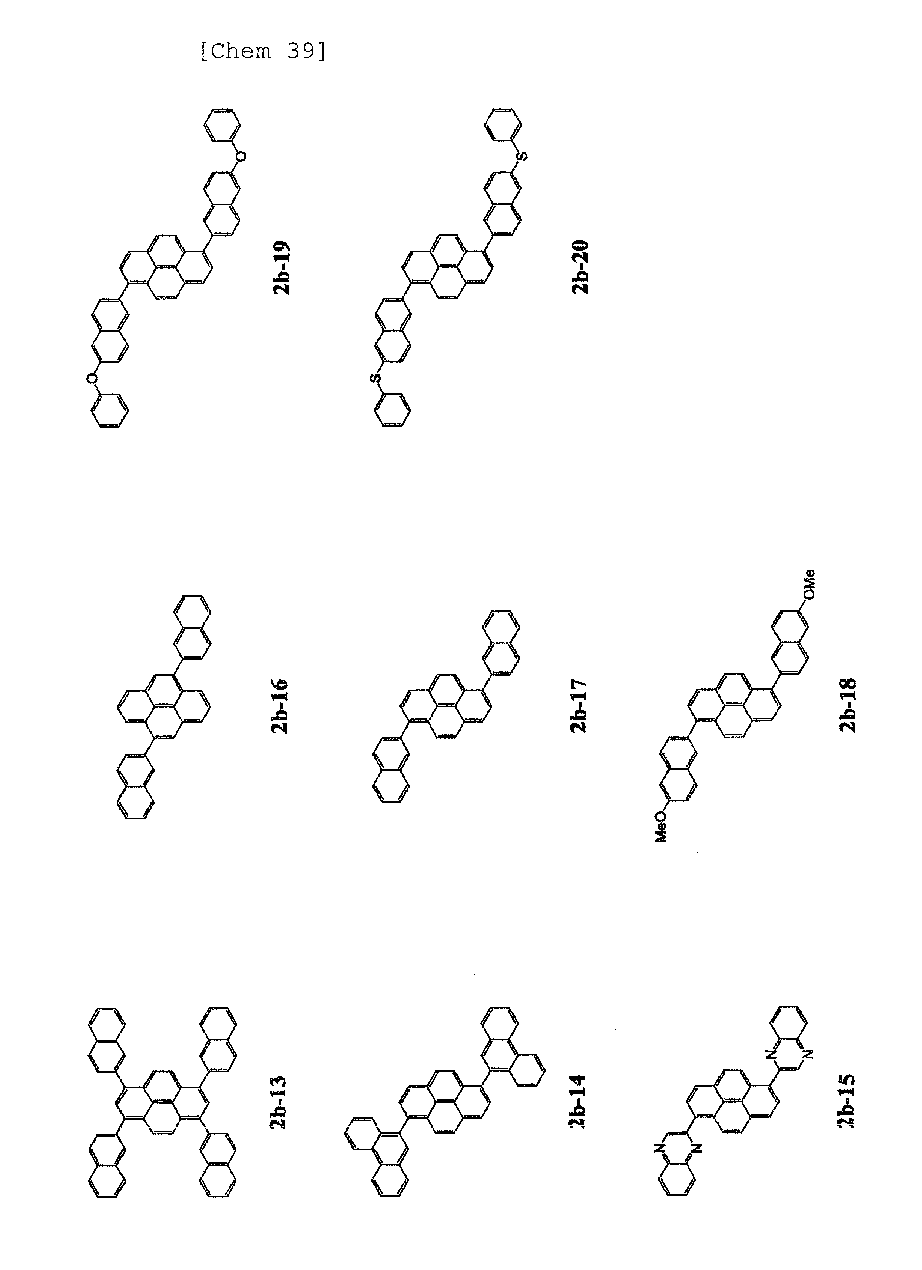

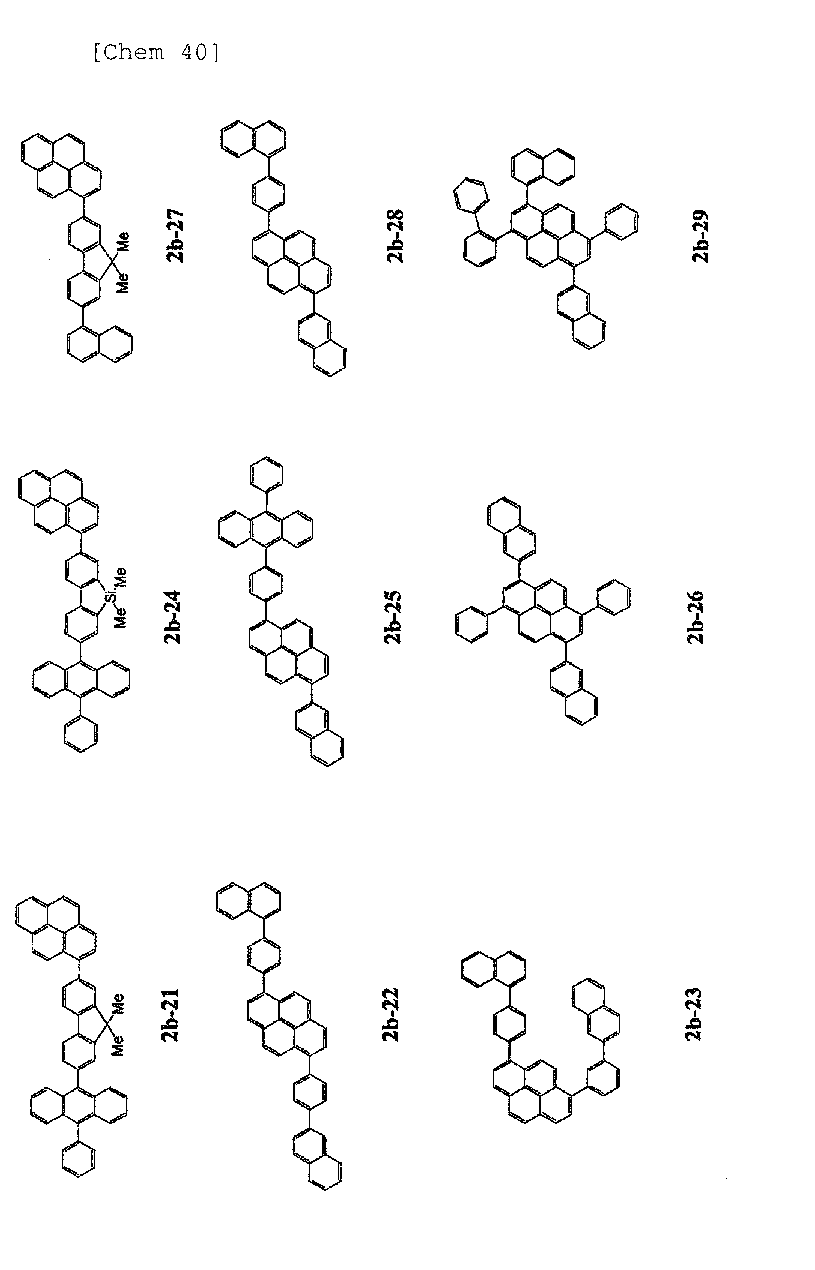

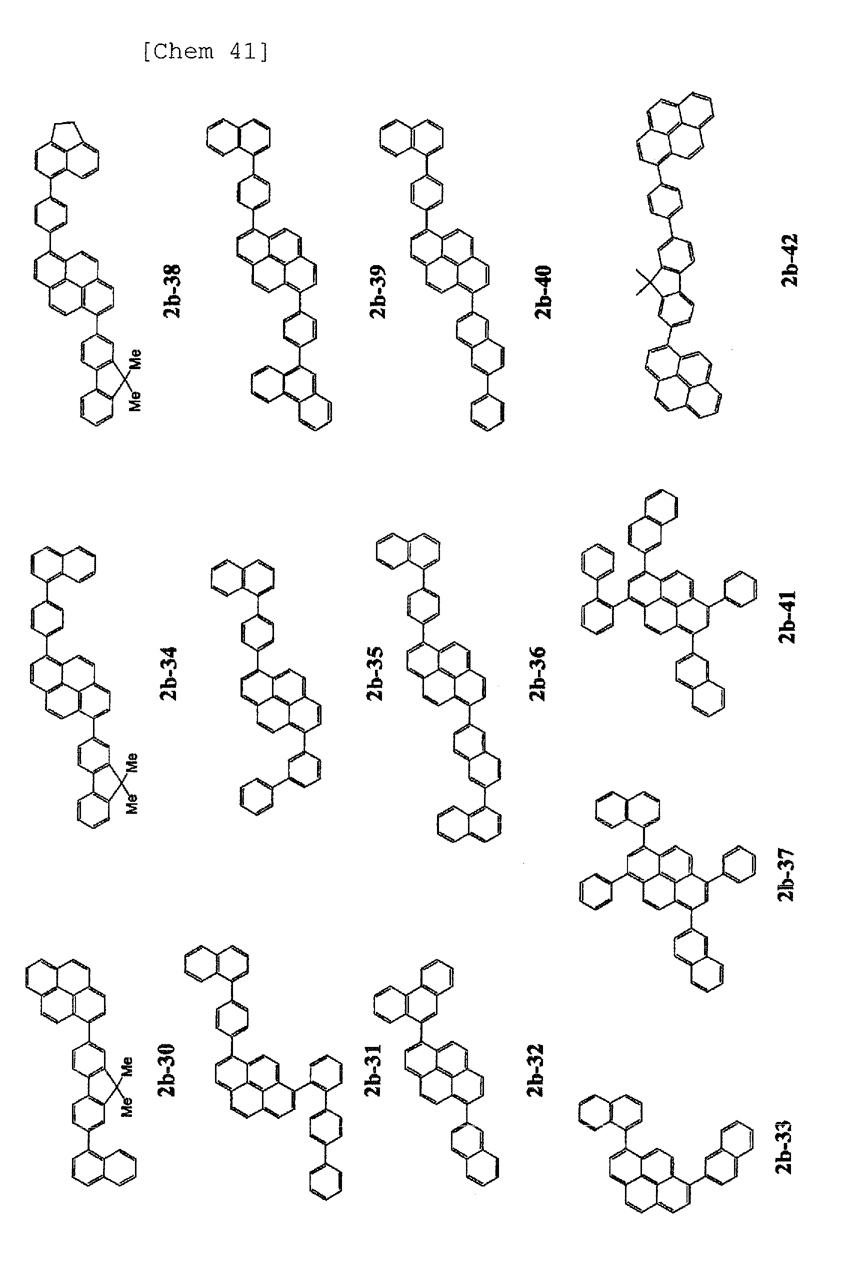

General formula (2c)

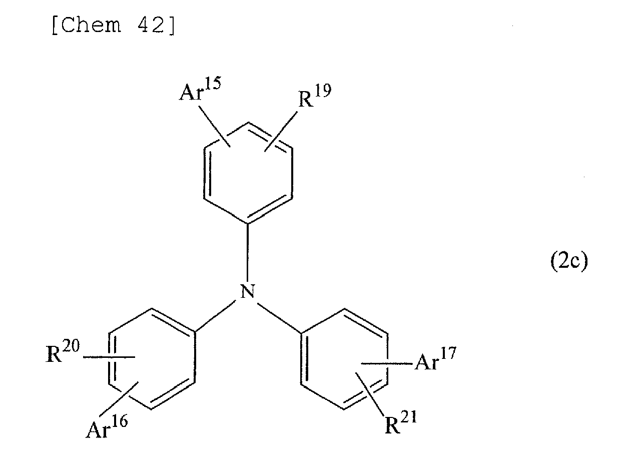

In the formula (2c) : Ar¹⁵ to Ar¹⁷ are each independently selected from a group having an anthracene structure, a group having a phenanthrene structure, and a group having a pyrene structure; and R¹⁹ to R²¹ each independently represent a hydrogen atom or a substituent.

Ar¹⁵ to Ar¹⁷ in the formula (2c) are each selected from preferably a substituted or unsubstituted anthrylphenyl group, an anthryl group, a phenanthrenyl group, a perylenyl group, and a pyrenyl group, more preferably an alkyl-substituted or unsubstituted anthrylphenyl group, a phenanthryl group, and a pyrenyl group, or particularly preferably a pyrenyl group and a phenanthryl group.

Examples of any one of R¹⁹ to R²¹ in the formula (2c) include: a hydrogen atom; an alkyl group (having preferably 1 to 30, more preferably 1 to 20, or particularly preferably 1 to 10 carbon atoms, such as methyl, ethyl, isopropyl, t-butyl, n-octyl, n-decyl, n-hexadecyl, cyclopropyl, cyclopentyl, or cyclohexyl); an alkenyl group (having preferably 2 to 30, more preferably 2 to 20, or particularly preferably 2 to 10 carbon atoms, such as vinyl, allyl, 2-butenyl, or 3-pentenyl); an alkynyl group (having preferably 2 to 30, more preferably 2 to 20, orparticularlypreferably 2 to 10 carbon atoms, such as propargyl or 3-pentynyl); an aryl group (having preferably 6 to 30, more preferably 6 to 20, or particularly preferably 6 to 12 carbon atoms, such as phenyl, p-methylphenyl, naphthyl, or anthranyl); an amino group (having preferably 0 to 30, more preferably 0 to 20, or particularly preferably 0 to 10 carbon atoms, such as amino, methylamino, dimethylamino, diethylamino, dibenzylamino, diphenylamino, or ditolylamino); an alkoxy group (having preferably 1 to 30, more preferably 1 to 20, or particularly preferably 1 to 10 carbon atoms, such as methoxy, ethoxy, butoxy, or 2-ethylhexyloxy); an aryloxy group (having preferably 6 to 30, more preferably 6 to 20, or particularlypreferably 6 to 12 carbon atoms, such as phenyloxy, 1-naphthyloxy, or 2-naphthyloxy) ; an heteroaryloxy group (having preferably 1 to 30, more preferably 1 to 20, or particularly preferably 1 to 12 carbon atoms, such as pyridyloxy, pyrazyloxy, pyrimidyloxy, or quinolyloxy); an acyl group (having preferably 1 to 30, more preferably 1 to 20, or particularly preferably 1 to 12 carbon atoms, such as acetyl, benzoyl, formyl, or pivaloyl); an alkoxycarbonyl group (having preferably 2 to 30, more preferably 2 to 20, or particularly preferably 2 to 12 carbon atoms, such as methoxycarbonyl or ethoxycarbonyl); an aryloxycarbonyl group (having preferably 7 to 30, more preferably 7 to 20, or particularly preferably 7 to 12 carbon atoms, such as phenyloxycarbonyl); an acyloxy group (having preferably 2 to 30, more preferably 2 to 20, or particularly preferably 2 to 10 carbon atoms, such as acetoxy or benzoyloxy) ; an acylamino group (having preferably 2 to 30, more preferably 2 to 20, or particularly preferably 2 to 10 carbon atoms, such as acetylamino or benzoylamino); an alkoxycarbonylamino group (having preferably 2 to 30, more preferably 2 to 20, or particularly preferably 2 to 12 carbon atoms, such as methoxycarbonylamino); an aryloxycarbonylamino group (having preferably 7 to 30, more preferably 7 to 20, or particularly preferably 7 to 12 carbon atoms, such as phenyloxycarbonylamino); a sulfonylamino group (having preferably 1 to 30, morepreferably 1 to 20, orparticularlypreferably 1 to 12 carbon atoms, such as methanesulfonylamino or benzenesulfonylamino); a sulfamoyl group (having preferably 0 to 30, more preferably 0 to 20, or particularly preferably 0 to 12 carbon atoms, such as sulfamoyl, methylsulfamoyl, dimethylsulfamoyl, or phenylsulfamoyl); a carbamoyl group (having preferably 1 to 30, more preferably 1 to 20, or particularly preferably 1 to 12 carbon atoms, such as carbamoyl, methylcarbamoyl, diethylcarbamoyl, or phenylcarbamoyl); an alkylthio group (having preferably 1 to 30, more preferably 1 to 20, or particularly preferably 1 to 12 carbon atoms, such as methylthio or ethylthio) ; an arylthio group (having preferably 6 to 30, more preferably 6 to 20, orparticularlypreferably 6 to 12 carbon atoms, such as phenylthio); a heteroarylthio group (having preferably 1 to 30, more preferably 1 to 20, or particularly preferably 1 to 12 carbon atoms, such as pyridylthio, 2-benzimizolylthio, 2-benzoxazolylthio, or 2-benzthiazolylthio); a sulfonyl group (having preferably 1 to 30, more preferably 1 to 20, or particularly preferably 1 to 12 carbon atoms, such as mesyl or tosyl); a sulfinyl group (havingpreferably 1 to 30, morepreferably 1 to 20, or particularly preferably 1 to 12 carbon atoms, such as methanesulfinyl or benzenesulfinyl); a ureido group (having preferably 1 to 30, more preferably 1 to 20, orparticularlypreferably 1 to 12 carbon atoms, such as ureido, methylureido, or phenylureido) ; a phosphoric acid amide group (having preferably 1 to 30, more preferably 1 to 20, or particularly preferably 1 to 12 carbon atoms, such as diethylphosphoric acid amide or phenylphosphoric acid amide); a hydroxyl group; a mercapto group; a halogen atom (such as a fluorine atom, a chlorine atom, a bromine atom, or an iodine atom) ; a cyano group; a sulfo group; a carboxyl group; a nitro group; a hydroxamic acid group; a sulfino group; a hydrazino group; an imino group; a heterocyclic group (having preferably 1 to 30 or more preferably 1 to 12 carbon atoms and containing, as a hetero atom, for example, a nitrogen atom, an oxygen atom, or a sulfur atom, and specific examples include imidazolyl, pyridyl, quinolyl, furyl, thienyl, piperidyl, morpholino, benzoxazolyl, benzimidazolyl, and benzthiazolyl); and a silyl group (having preferably 3 to 40, more preferably 3 to 30, orparticularlypreferably 3 to 24 carbon atoms, such as trimethylsilyl or triphenylsilyl). Each of those substituents may be additionally substituted.
The substituents R¹⁹ to R²¹ in the formula (2c) are each preferably selected from an alkyl group and an aryl group.

Specific examples of the amine derivative represented by the general formula (2c) to be used in the organic EL device of the present invention include various known amine derivatives such as an amine derivative described in paragraphs [0079] to [0083] of JP 2002-324678 A. Representative specific examples are shown below.

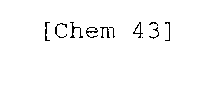

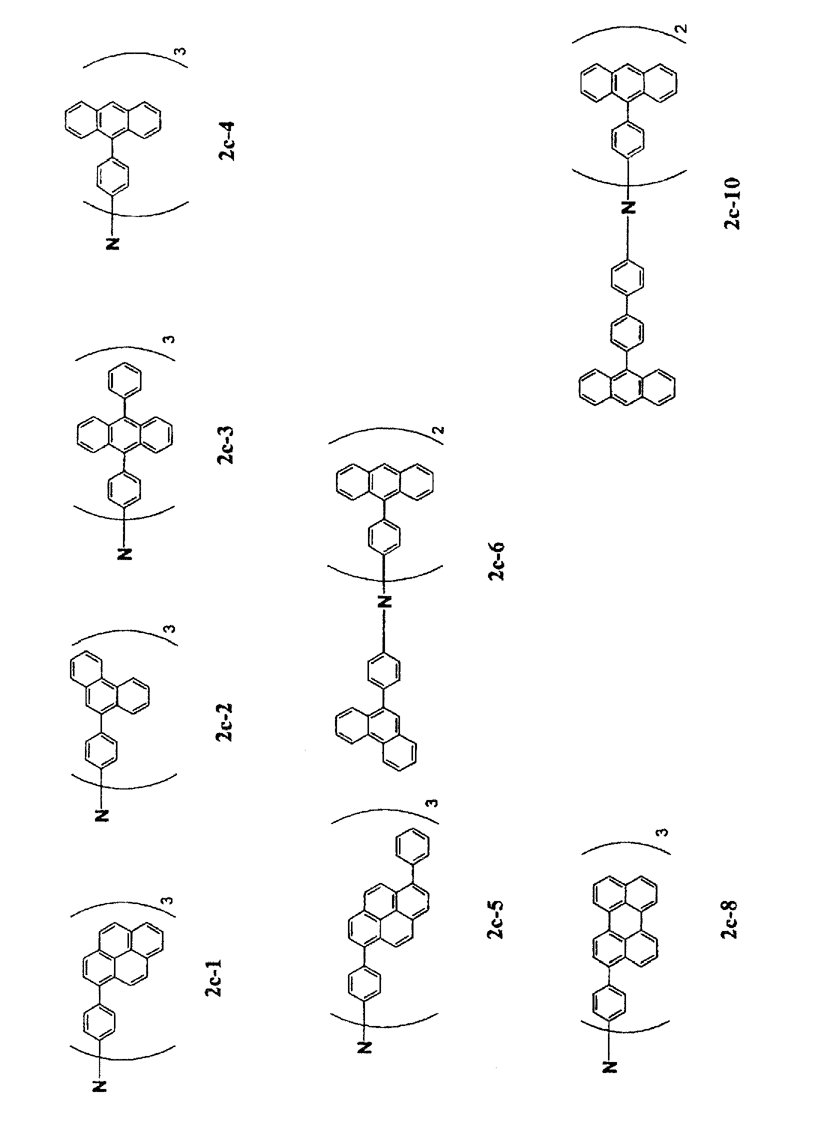

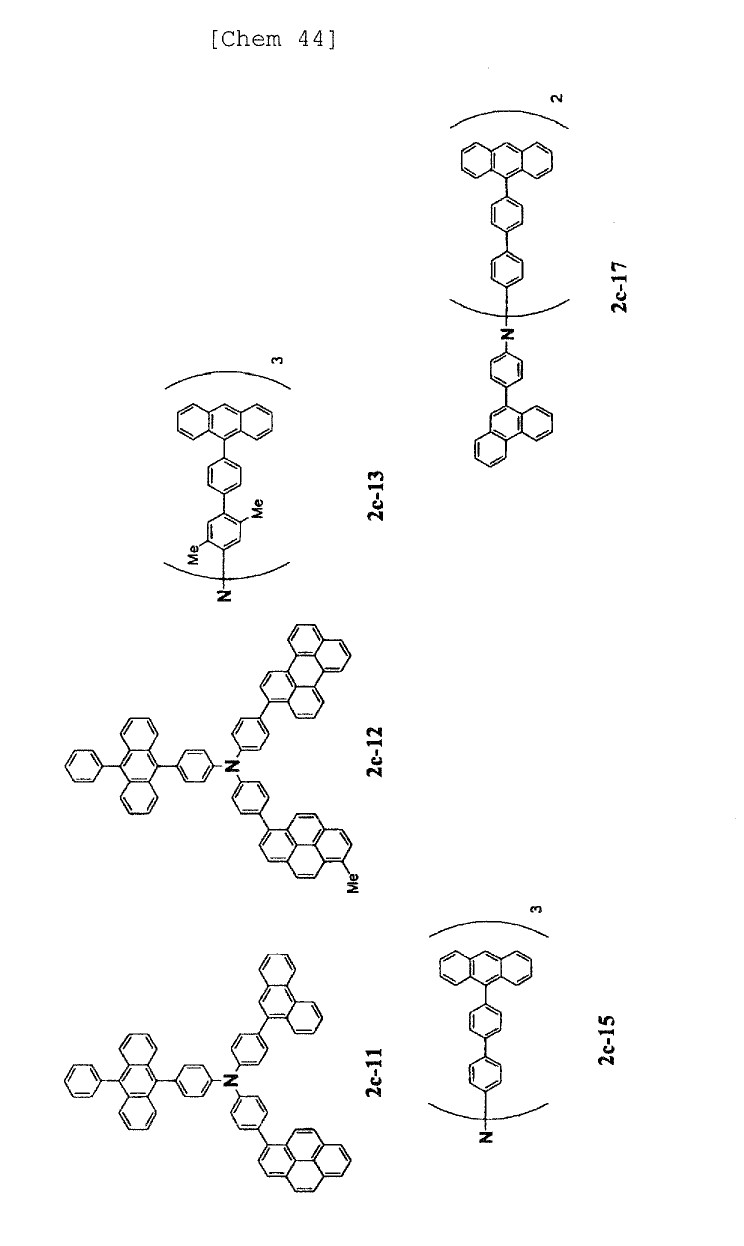

General formula (2d)

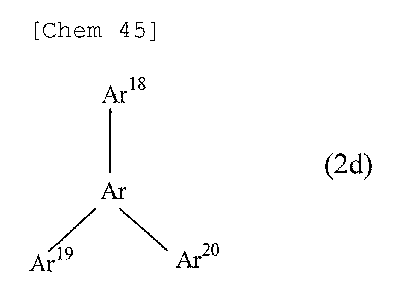

In the formula (2d): Ar¹⁸ to Ar²⁰ each independently represent an aryl group having 6 to 50 ring carbon atoms, and the aryl group may be substituted by one or two or more substituents; at least one of Ar¹⁸ to Ar²⁰ and substituents possessed by those aryl groups has a fused ring aryl structure having 10 to 20 ring carbon atoms, or a fused ring heteroaryl structure having 6 to 20 ring carbon atoms; and Ar represents a trivalent group derived from an aromatic ring or from a heterocyclic aromatic ring.

The aryl group having 6 to 50 ring carbon atoms represented by any one of Ar¹⁸ to Ar²⁰ in the formula (2d) has preferably 6 to 30, more preferably 6 to 20, or still more preferably 6 to 16 ring carbon atoms. Examples of the aryl group include a phenyl group, a naphthyl group, an anthryl group, a phenanthrenyl group, a pyrenyl group, a perylenyl group, a fluorenyl group, a biphenylyl group, a terphenylyl group, a rubrenyl group, a chrysenyl group, a triphenylenyl group, a benzoanthryl group, a benzophenanthrenyl group, and a diphenylanthryl group. Each of those aryl groups may additionally have a substituent.

Examples of the substituent in aryl groups include: an alkyl group (having preferably 1 to 30, more preferably 1 to 20, or particularly preferably 1 to 10 carbon atoms, such as methyl, ethyl, isopropyl, t-butyl, n-octyl, n-decyl, n-hexadecyl, cyclopropyl, cyclopentyl, or cyclohexyl); an alkenyl group (having preferably 2 to 30, more preferably 2 to 20, orparticularlypreferably 2 to 10 carbon atoms, such as vinyl, allyl, 2-butenyl, or 3-pentenyl) ; an alkynyl group (having preferably 2 to 30, more preferably 2 to 20, or particularly preferably 2 to 10 carbon atoms, such as propargyl or 3-pentynyl); an aryl group (having preferably 6 to 30, more preferably 6 to 20, or particularly preferably 6 to 12 carbon atoms, such as phenyl, p-methylphenyl, naphthyl, or anthranyl); an amino group (having preferably 0 to 30, more preferably 0 to 20, or particularly preferably 0 to 10 carbon atoms, such as amino, methylamino, dimethylamino, diethylamino, dibenzylamino, diphenylamino, or ditolylamino); an alkoxy group (having preferably 1 to 30, more preferably 1 to 20, or particularly preferably 1 to 10 carbon atoms, such as methoxy, ethoxy, butoxy, or 2-ethylhexyloxy) ; an aryloxy group (having preferably 6 to 30, more preferably 6 to 20, or particularly preferably 6 to 12 carbon atoms, such as phenyloxy, 1-naphthyloxy, or 2-naphthyloxy); a heteroaryloxy group (having preferably 1 to 30, more preferably 1 to 20, or particularly preferably 1 to 12 carbon atoms, such as pyridyloxy,pyrazyloxy,pyrimidyloxy,or quinolyloxy);an acylgroup (having preferably 1 to 30, more preferably 1 to 20, or particularly preferably 1 to 12 carbon atoms, such as acetyl, benzoyl, formyl, or pivaloyl); an alkoxycarbonyl group (having preferably 2 to 30, more preferably 2 to 20, or particularly preferably 2 to 12 carbon atoms, such as methoxycarbonyl or ethoxycarbonyl); an aryloxycarbonyl group (having preferably 7 to 30, more preferably 7 to 20, or particularly preferably 7 to 12 carbon atoms, such as phenyloxycarbonyl); an acyloxy group (having preferably 2 to 30, more preferably 2 to 20, or particularly preferably 2 to 10 carbon atoms, such as acetoxy or benzoyloxy); an acylamino group (having preferably 2 to 30, more preferably 2 to 20, or particularly preferably 2 to 10 carbon atoms, such as acetylamino or benzoylamino); an alkoxycarbonylamino group (having preferably 2 to 30, more preferably 2 to 20, or particularly preferably 2 to 12 carbon atoms, such as methoxycarbonylamino); an aryloxycarbonylamino group (having preferably 7 to 30, more preferably 7 to 20, or particularly preferably 7 to 12 carbon atoms, such as phenyloxycarbonylamino); a sulfonylamino group (having preferably 1 to 30, more preferably 1 to 20, or particularly preferably 1 to 12 carbon atoms, such as methanesulfonylamino or benzenesulfonylamino); a sulfamoyl group (having preferably 0 to 30, more preferably 0 to 20, or particularly preferably 0 to 12 carbon atoms, such as sulfamoyl, methylsufamoyl, dimethylsulfamoyl, orphenylsulfamoyl); a carbamoyl group (having preferably 1 to 30, morepreferably 1 to 20, orparticularlypreferably 1 to 12 carbon atoms, such as carbamoyl, methylcarbamoyl, diethylcarbamoyl, or phenylcarbamoyl); an alkylthio group (having preferably 1 to 30, more preferably 1 to 20, or particularlypreferably 1 to 12 carbon atoms, such as methylthio or ethylthio) ; an arylthio group (having preferably 6 to 30, more preferably 6 to 20, or particularly preferably 6 to 12 carbon atoms, such as phenylthio); a heteroarylthio group (having preferably 1 to 30, more preferably 1 to 20, or particularly preferably 1 to 12 carbon atoms, such as pyridylthio, 2-benzimizolylthio, 2-benzoxazolylthio, or 2-benzthiazolylthio); a sulfonyl group (having preferably 1 to 30, more preferably 1 to 20, or particularly preferably 1 to 12 carbon atoms, such as mesyl or tosyl) ; a sulfinyl group (having preferably 1 to 30, more preferably 1 to 20, or particularly preferably 1 to 12 carbon atoms, such as methanesulfinyl or benzenesulfinyl); a ureido group (having preferably 1 to 30, more preferably 1 to 20, or particularly preferably 1 to 12 carbon atoms, such as ureido, methylureido, or phenylureido); a phosphoric acid amide group (having preferably 1 to 30, more preferably 1 to 20, or particularly preferably 1 to 12 carbon atoms, such as diethylphosphoric acid amide or phenylphosphoric acid amide) ; a hydroxyl group; a mercapto group; a halogen atom (such as a fluorine atom, a chlorine atom, a bromine atom, or an iodine atom); a cyano group; a sulfo group; a carboxyl group; a nitro group; a hydroxamic acid group; a sulfino group; a hydrazino group; an imino group; a heterocyclic group (having preferably 1 to 30 or more preferably 1 to 12 carbon atoms and containing, as a hetero atom, for example, a nitrogen atom, an oxygen atom, or a sulfur atom, and specific examples include imidazolyl, pyridyl, quinolyl, furyl, thienyl, piperidyl, morpholino, benzoxazolyl, benzimidazolyl, benzthiazolyl, a carbazolyl group, or an azepinyl group); and a silyl group (having preferably 3 to 40, more preferably 3 to 30, or particularly preferably 3 to 24 carbon atoms, such as trimethylsilyl or triphenylsilyl). Each of those substituents may be additionally substituted.

Examples of the fused ring aryl structure having 10 to 20 ring carbon atoms possessed by at least one of Ar¹⁸ to Ar²⁰ in the formula (2d) and substituents possessed by those aryl groups include a naphthalene structure, an anthracene structure, a phenanthrene structure, a pyrene structure, and a perylene structure. Of those, a naphthalene structure, an anthracene structure, a pyrene structure, or a phenanthrene structure is preferred, a phenanthrene structure or an aryl structure with four or more rings is more preferred, and a pyrene structure is particularly preferred.
Examples of the fused ring heteroaryl structure having 6 to 20 ring carbon atoms possessed by at least one of Ar¹⁸ to Ar²⁰ in the formula (2d) and substituents possessed by those aryl groups include a quinoline structure, a quinoxaline structure, a quinazoline structure, an acridine structure, a phenanthridine structure, a phthalazine structure, and a phenanthroline structure. Of those, a quinoline structure, a quinoxaline structure, a quinazoline structure, a phthalazine structure, or a phenanthroline structure is preferred.

The trivalent group derived from an aromatic ring represented by Ar in the general formula (2d) has preferably 6 to 30, more preferably 6 to 20, or still more preferably 6 to 16 carbon atoms. Specific examples of the trivalent group include trivalent groups each derived from benzene, naphthalene, anthracene, phenanthrene, pyrene, or triphenylene.
The trivalent group derived from a heterocyclic aromatic ring represented by Ar in the general formula (2d) contains, as a hetero atom, preferably an atom selected from a nitrogen atom, a sulfur atom, and an oxygen atom, or more preferably a nitrogen atom. In addition, the trivalent group has preferably 2 to 30, more preferably 3 to 20, or still more preferably 3 to 16 carbon atoms. Specific examples of the trivalent group include trivalent groups each derived from pyridine, pyrazine, thiopyran, quinoline, quinoxaline, or triazine. Each of those trivalent groups each derived from an aromatic ring or from a heterocyclic aromatic ring may have a substituent. Examples of the substituent include groups exemplified for a substituent on the aryl group represented by the substituent Ar¹⁸. Ar preferably represents a trivalent group derived frombenzenetriyl, naphthalenetriyl, anthracenetriyl, pyrenetriyl, or triphenylene, more preferably represents benzenetriyl, or still more preferably represents an unsubstituted (Ar¹⁸, Ar¹⁹, and Ar²⁰ are each substituted) benzenetriyl or an alkyl-substituted benzenetriyl group.

Specific examples of the benzene derivative represented by the general formula (2d) to be used in the organic EL device of the present invention include various known benzene derivatives such as a benzene derivative described in paragraphs [0079] to [0083] of JP 2002-324678 A. Representative specific examples are shown below.

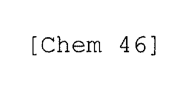

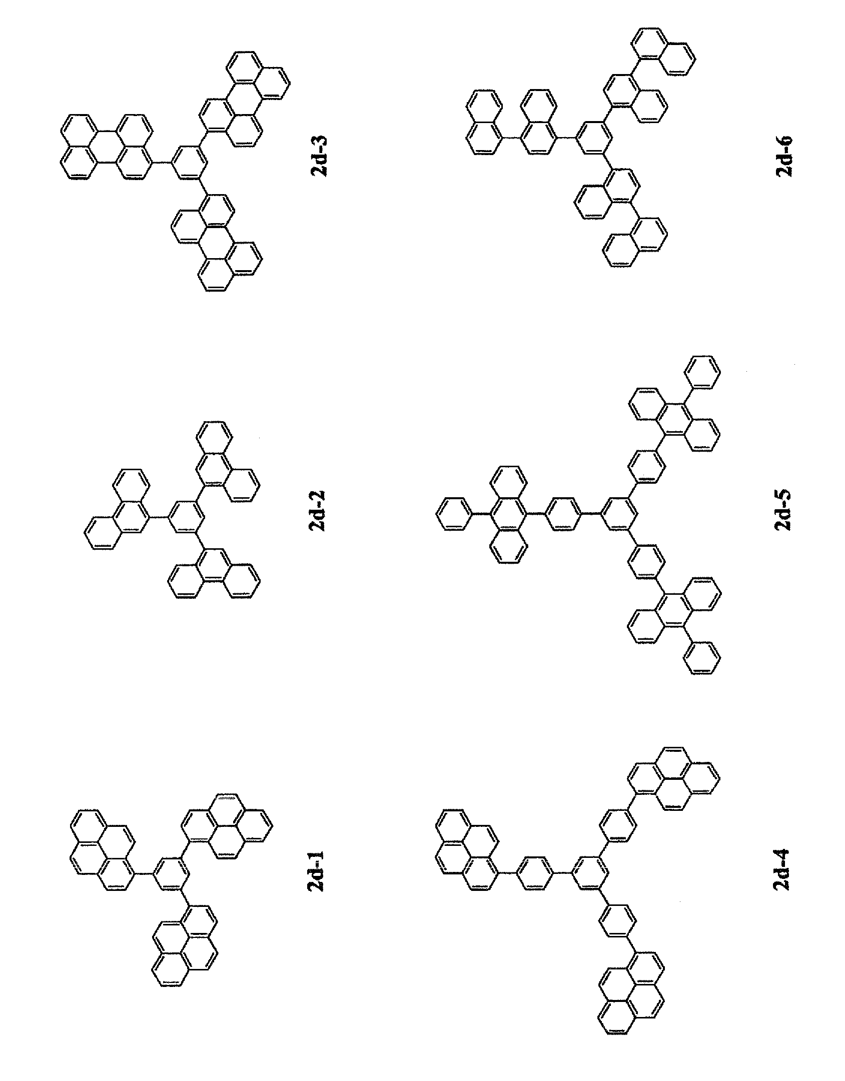

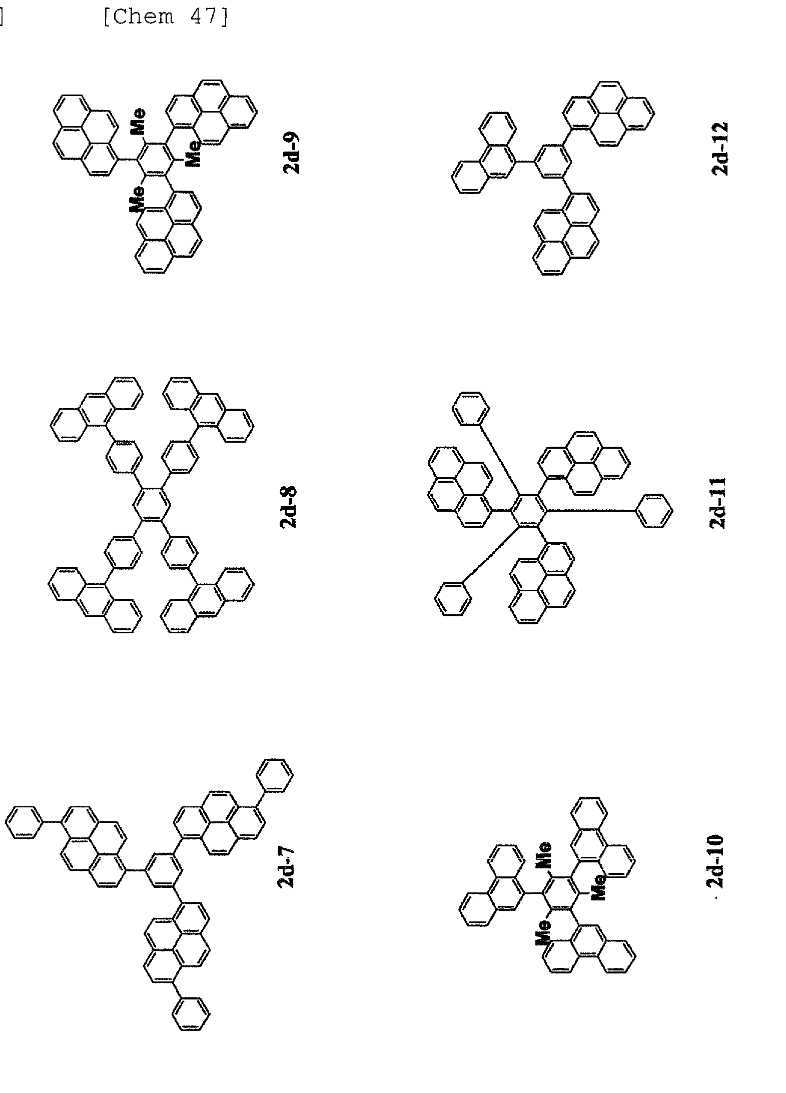

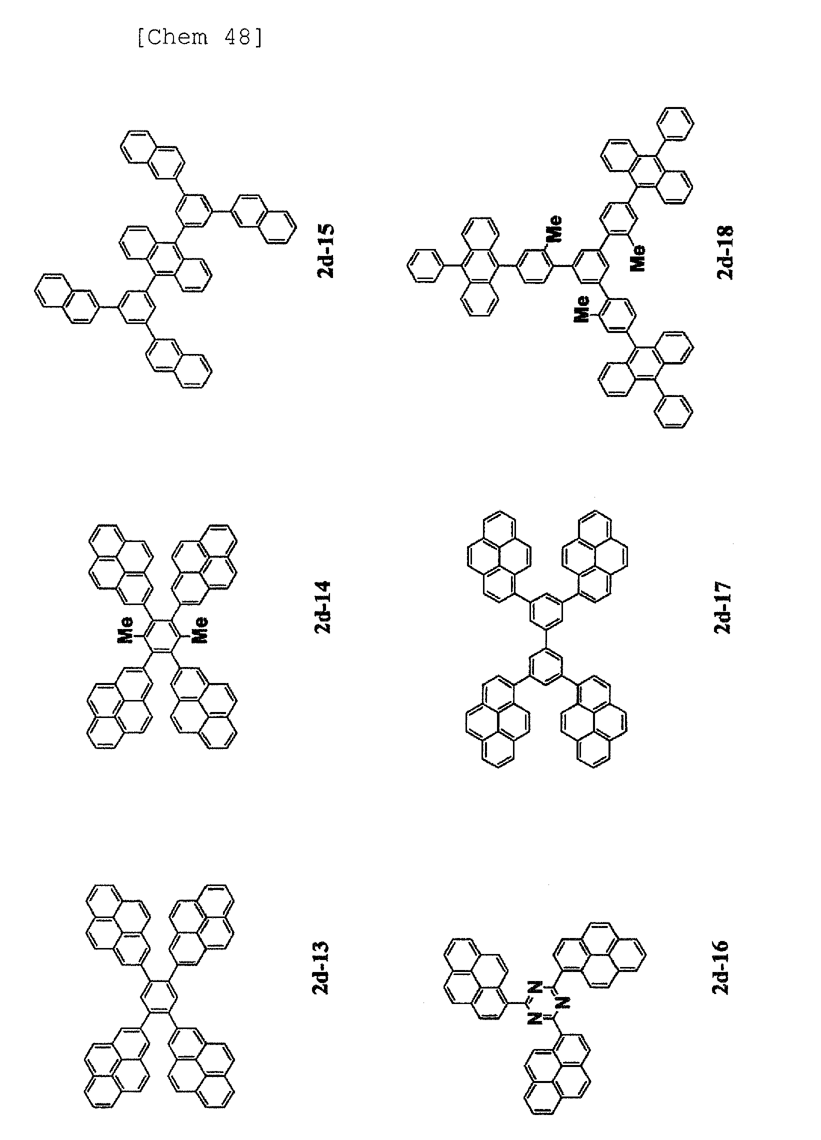

In the present invention, the organic EL device having multiple organic thin film layers is a laminate having, for example, an (anode/hole injecting layer/light emitting layer/cathode), (anode/light emitting layer/electron injecting layer/cathode), or (anode/hole injecting layer/light emitting layer/electron injecting layer/cathode) constitution.
In addition to the aromatic amine derivative of the present invention, an additional known light emitting material, doping material, hole injecting material, or electron injecting material can be used as required in the multiple layers. When the organic EL device has a constitution of the multiple organic thin film layers, a reduction in luminance or lifetime due to quenching can be prevented.
If needed, a light emitting material, a doping material, a hole injecting material, and an electron injecting material can be used in combination. In addition, a doping material can provide improvements in emission luminance and luminous efficiency, and red or blue light emission. In addition, each of the hole injecting layer, the light emitting layer, and the electron injecting layer may be formed of a layer constitution having two or more layers. At that time, in the case of the hole injecting layer, a layer for injecting a hole from the electrode is referred to as a hole injecting layer, and a layer for receiving the hole from the hole injecting layer and transporting the hole to the light emitting layer is referred to as a hole transporting layer. In the same manner, in the case of the electron injecting layer, a layer for injecting an electron from the electrode is referred to as an electron injecting layer, and a layer for receiving the electron from the electron injecting layer and transporting the electron to the light emitting layer is referred to as an electron transporting layer. Each of those layers is selected and used depending on factors such as the energy level of a material, heat resistance, and adhesiveness between the layer and an organic layer or a metal electrode.

Examples of a host material or a doping material other than those represented by the above general formulae (2a) to (2d) which can be used in the light emitting layer together with the aromatic amine derivative of the present invention include, but are not limited to: polyfused aromatic compounds such as naphthalene, phenanthrene, rubrene, anthracene, tetracene, pyrene, perylene, chrysene, decacyclene, coronene, tetraphenylcyclopentadiene, pentaphenylcyclopentadiene, fluorene, spirofluorene, 9,10-diphenylanthracene, 9,10-bis(phenylethinyl)anthracene, and 1,4-bis(9'-ethinylanthracene)benzene and derivatives thereof; organic metal complexes such as tris(8-quinolinolato)aluminum and bis-(2-methyl-8-quinolinolato)-4-(phenylphenolato)aluminum; a triarylamine derivative; a styrylamine derivative; a stilbene derivative; a coumarin derivative; a pyrane derivative; an oxazone derivative; a benzothiazole derivative; a benzoxazole derivative; a benzimidazole derivative; a pyrazine derivative; a cinnamate derivative; a diketopyrrolopyrrole derivative; an acridone derivative; and a quinacridone derivative.

A compound having an ability of transporting a hole, having a hole injection effect from an anode and an excellent hole injection effect to a light emitting layer or a light emittingmaterial, having an ability of preventing the migration of an exciton generated in the light emitting layer to an electron injecting layer or an electron injecting material, and having excellent thin film-formability is preferred as a hole injecting material. Specific examples of the compound include, but are not limited to, a phthalocyanine derivative, a naphthalocyanine derivative, a porphyrin derivative, oxazole, oxadiazole, triazole, imidazole, imidazolone, imidazolethione, pyrazoline, pyrazolone, tetrahydroimidazole, oxazole, oxadiazole, hydrazone, acylhydrazone, polyarylalkane, stilbene, butadiene, benzidine type triphenylamine, styrylamine type triphenylamine, diamine type triphenylamine, derivatives thereof, and polymer materials such as polyvinyl carbazole, polysilane, and a conductive polymer.
Of the hole injecting materials that can be used in the organic EL device of the present invention, more effective hole injecting materials are an aromatic tertiary amine derivative and a phthalocyanine derivative.
Examples of the aromatic tertiary amine derivative include, but are not limited to, triphenylamine, tritolylamine, tolyldiphenylamine, N,N'-diphenyl-N,N'-(3-methylphenyl)-1,1'-biphenyl-4,4'-diamine, N,N,N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)-phenanthrene-9,10-d iamine, N,N-bis(4-di-4-tolylaminophenyl)-4-phenyl-cyclohexane, and an oligomer or a polymer having one of the aromatic tertiary amine skeletons.

Examples of the phthalocyanine (Pc) derivative include, but are not limited to, phthalocyanine derivatives such as H₂Pc, CuPc, CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc, and GaPc-O-GaPc, and naphthalocyanine derivatives.
In addition, the organic EL device of the present invention is preferably formed of a layer containing each of those aromatic tertiary amine derivatives and/or each of phthalocyanine derivatives, for example, the hole transporting layer or the hole injecting layer between a light emitting layer and an anode.

A compound having an ability of transporting electrons, having an electron injection effect from a cathode and an excellent electron injection effect to a light emitting layer or a light emitting material, having an ability of preventing the migration of an exciton generated in the light emitting layer to the hole injecting layer, and having excellent thin film-formability is preferred as an electron injecting material. Specific examples of the compound include fluorenone, anthraquinodimethane, diphenoquinone, thiopyranedioxide, oxazole, oxadiazole, triazole, imidazole, perylenetetracarboxylic acid, fluorenylidenemethane, anthraquinodimethane, anthrone, and derivatives thereof, but the compound is not limited thereto. In addition, an electron-accepting substance can be added to the hole injecting material or an electron-donating substance can be added to the electron injecting material to thereby sensitize the hole injecting material or the electron injecting material, respectively.

In the organic EL device of the present invention, more effective electron injecting materials are a metal complex compound and a nitrogen-containing five-membered ring derivative.
Examples of the metal complex compound include, but are not limited to, 8-hydroxyquinolinatolithium, bis (8-hydroxyquinolinato) zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, and bis(2-methyl-8-quinolinato)(2-naphtholato)gallium.

Examples of the nitrogen-containing five-memebered derivative preferably include, an oxazole derivative, a thiazole derivative, an oxadiazole derivative, a thiadiazole derivative, and a triazole derivative. Specific examples of the derivative include, but are not limited to, 2,5-bis(1-phenyl)-1,3,4-oxazole, dimethylPOPOP, 2,5-bis(1-phenyl)-1,3,4-thiazole, 2,5-bis(1-phenyl)-1,3,4-oxadiazole, 2-(4'-tert-butylphenyl)-5-(4"-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4''-biphenyl)-1,3,4-thiadiazole, 2,5-bis(1-naphthyl)-1,3,4-thiadiazole, 1,4-bis[2-(5-phenylthiadiazolyl)]benzene, 2-(4'-tert-butylphenyl)-5-(4''-biphenyl)-1,3,4-triazole, 2,5-bis(1-naphthyl)-1,3,4-triazole, and 1,4-bis[2-(5-phenyltriazolyl)]benzene.

In the organic EL device of the present invention, in addition to at least one kind selected from the aromatic amine derivatives represented by the general formula (I), at least one kind of light emitting material, doping material, hole injecting material, and electron injecting material may be incorporated into the light emitting layers. In addition, the surface of the organic EL device obtained according to the present invention can be provided with a protective layer, or the entire device can be protected with silicone oil, a resin, or the like with a view to improving the stability of the device against temperature, humidity, an atmosphere, or the like.
A conductive material having a work function larger than 4 eV is suitably used in the anode of the organic EL device of the present invention. Examples of the conductive material to be used include, but are not limited to: carbon, aluminum, vanadium, iron, cobalt, nickel, tungsten, silver, gold, platinum, palladium, and alloys thereof; metal oxides such as tin oxide and indium oxide to be used in an ITO substrate and an NESA substrate; and further, organic conductive resins such as polythiophene and polypyrrole. A conductive substance having a work function smaller than 4 eV is suitably used in the cathode. Examples of the conductive substance to be used include, but are not limited to, magnesium, calcium, tin, lead, titanium, yttrium, lithium, ruthenium, manganese, aluminum, lithium fluoride, and alloys thereof. Representative examples of the alloys include, but are not limited to, a magnesium/silver alloy, a magnesium/indium alloy, and a lithium/aluminum alloy. A ratio between the components of the alloy is controlled depending on, for examples, the temperature of a deposition source, an atmosphere, and the degree of vacuum, and is selected appropriately. Each of the anode and the cathode may be formed in a layer constitution having two or more layers if needed.

It is desirable that at least one surface of the organic EL device of the present invention is sufficiently transparent in the emission wavelength region of the device so that the device can efficiently emit light. A substrate is also desirably transparent. A transparent electrode is formed by using any one of the above conductive materials, and is set by a method such as deposition or sputtering in such a manner that desired translucency is secured. The light transmittance of an electrode on a light emitting surface is desirably 10% or more. The substrate is not limited as long as it has mechanical strength, thermal strength, and transparency. Examples of the substrate include a glass substrate and a transparent resin film. Examples of the transparent resin film include polyethylene, anethylene-vinyl acetate copolymer, an ethylene-vinyl alcohol copolymer, polypropylene, polystyrene, polymethyl methacrylate, polyvinyl chloride, polyvinyl alcohol, polyvinyl butyral, nylon, polyether ether ketone, polysulfone, polyether sulfone, a tetrafluoroethylene-perfluoroalkyl vinyl ether copolymer, polyvinyl fluoride, a tetrafluoroethylene-ethylene copolymer, a tetrafluoroethylene-hexafluoropropylene copolymer, polychlorotrifluoroethylene, polyvinylidene fluoride, polyester, polycarbonate, polyurethane, polyimide, polyether imide, polyimide, and polypropylene.

Any one of dry film forming methods such as vacuum deposition, sputtering, plasma, and ion plating, and wet film forming methods such as spin coating, dipping, and flow coating is applicable to the formation of each layer of the organic EL device of the present invention. The thickness of each layer is not particularly limited, but must be set to an appropriate thickness. An excessively large thickness requires an increased applied voltage for obtaining certain optical output, resulting in poor efficiency. An excessively small thickness causes a pin hole or the like, with the result that sufficient emission luminance cannot be obtained even when an electric field is applied. In general, the thickness is in the range of preferably 5 nm to 10 µm, or more preferably 10 nm to 0.2 µm.

In the case of a wet film forming method, a material of which each layer is formed is dissolved or dispersed into an appropriate solvent such as ethanol, chloroform, tetrahydrofuran, or dioxane, to thereby form a thin film. At that time, any one of the above solvents may be used.
An organic EL material-containing solution containing the aromatic amine derivative of the present invention as an organic EL material and a solvent can be used as a solution suitable for such wet film forming method.
In addition, it is preferred that the organic EL material contain a host material and a dopant material, the dopant material be the aromatic amine derivative of the present invention, and the host material be at least one kind selected from the compounds represented by the general formulae (2a), (2b), (2c), and (2d).
In addition, an appropriate resin or additive may be used in each of the organic thin film layers for, for example, improving film formability or preventing a pin hole in the layer.
Examples of an available resin include: insulating resins such as polystyrene, polycarbonate, polyallylate, polyester, polyamide, polyurethane, polysulfone, polymethyl methacrylate, polymethyl acrylate, and cellulose, and copolymers thereof; photoconductive resins such as poly-N-vinylcarbazole and polysilane; and conductive resins such as polythiophene and polypyrrole. Examples of the additive include an antioxidant, a UV absorber, and a plasticizer.

The organic EL device of the present invention can find use in applications including a flat luminous body such as the flat panel display of a wall hanging television, a light source for the backlight, meters, or the like of a copying machine, a printer, or a liquid crystal display, a display panel, and a signal lamp. In addition, the material of the present invention can be used in not only the field of an organic EL device but also the fields of an electrophotographic photosensitive member, a photoelectric conversion device, a solar cell, an image sensor, and the like.

### Examples

Hereinafter, the present invention is described in detail by way of synthesis examples and examples.
Synthesis processes for Compounds D-41 to D-54 synthesized in the synthesis examples are shown.

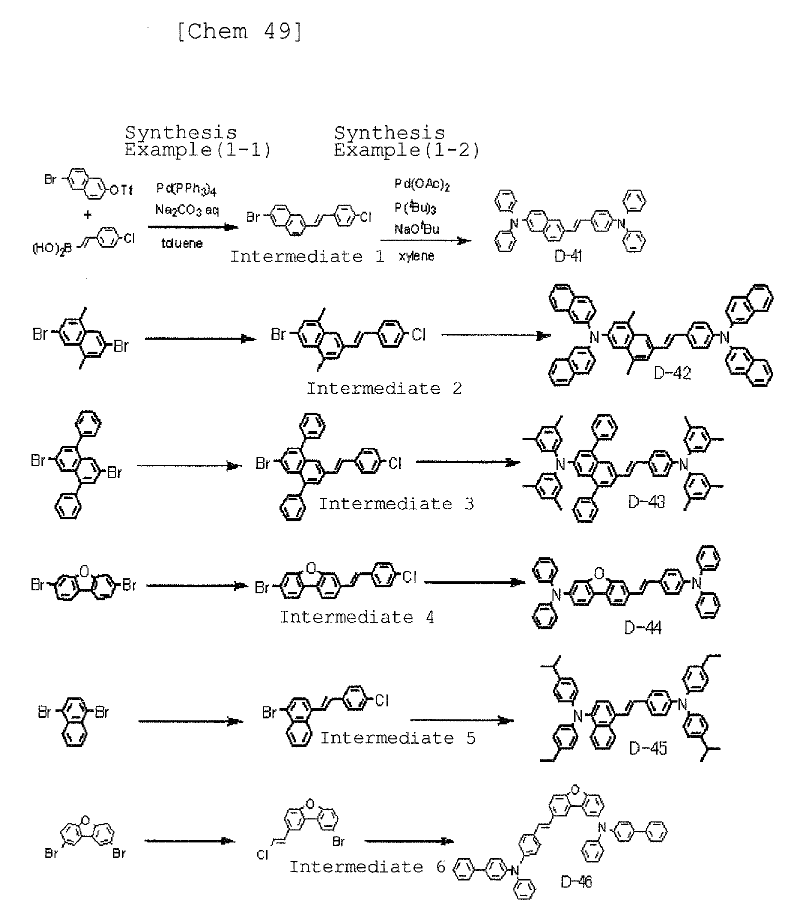

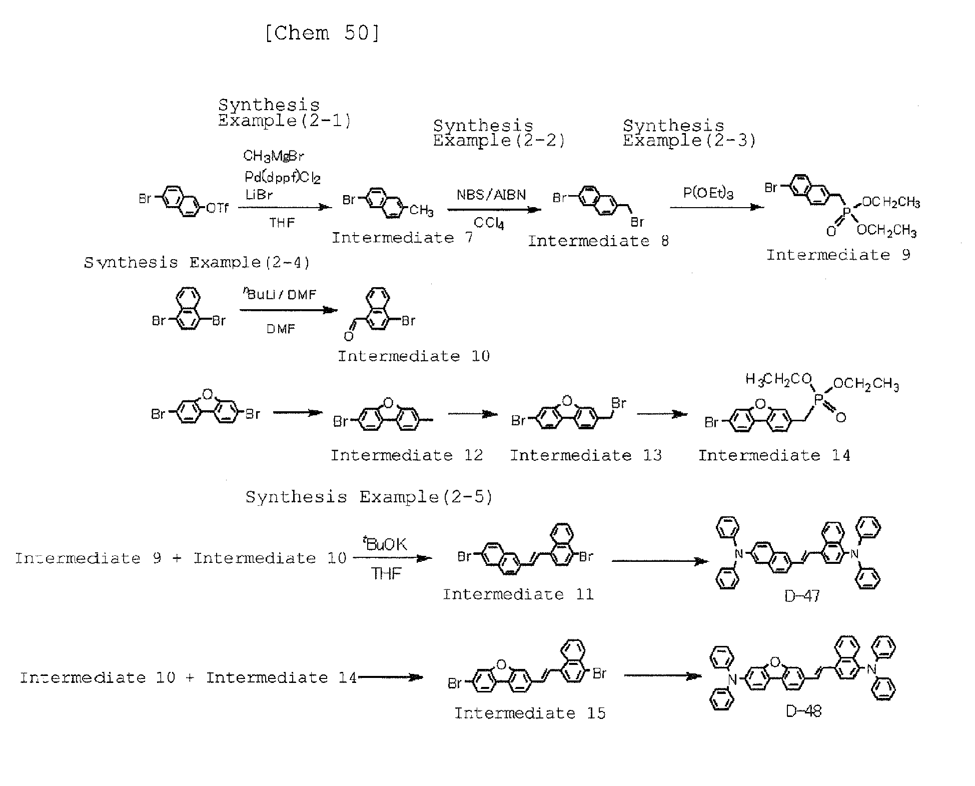

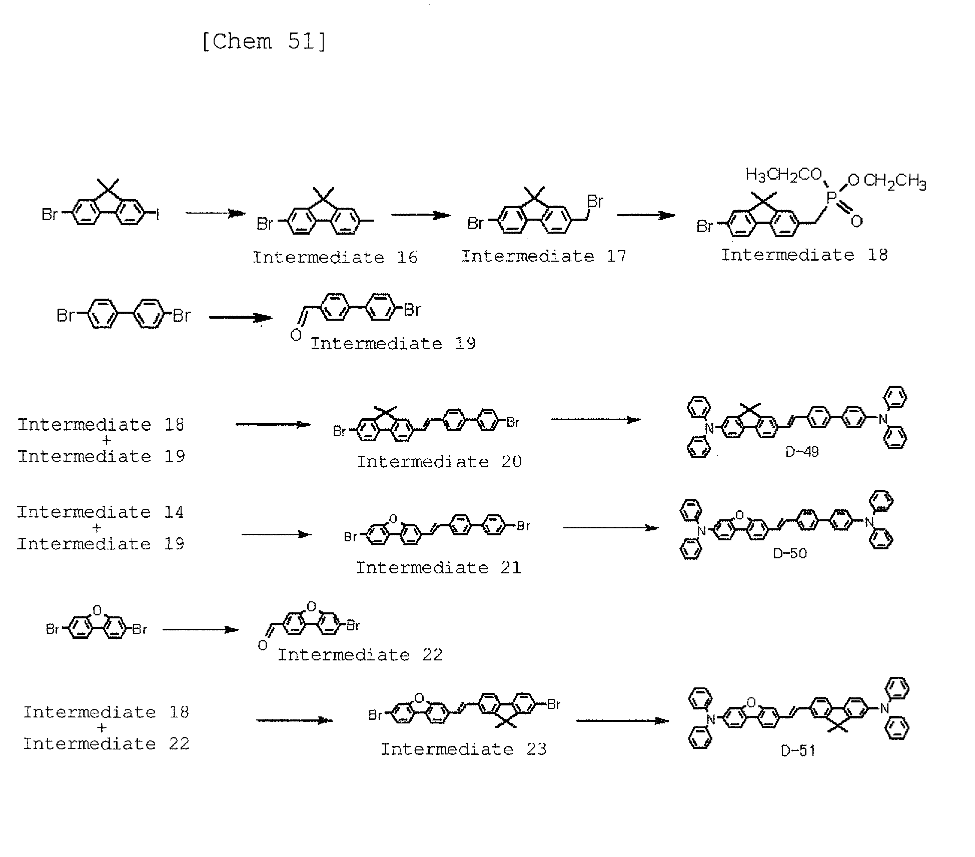

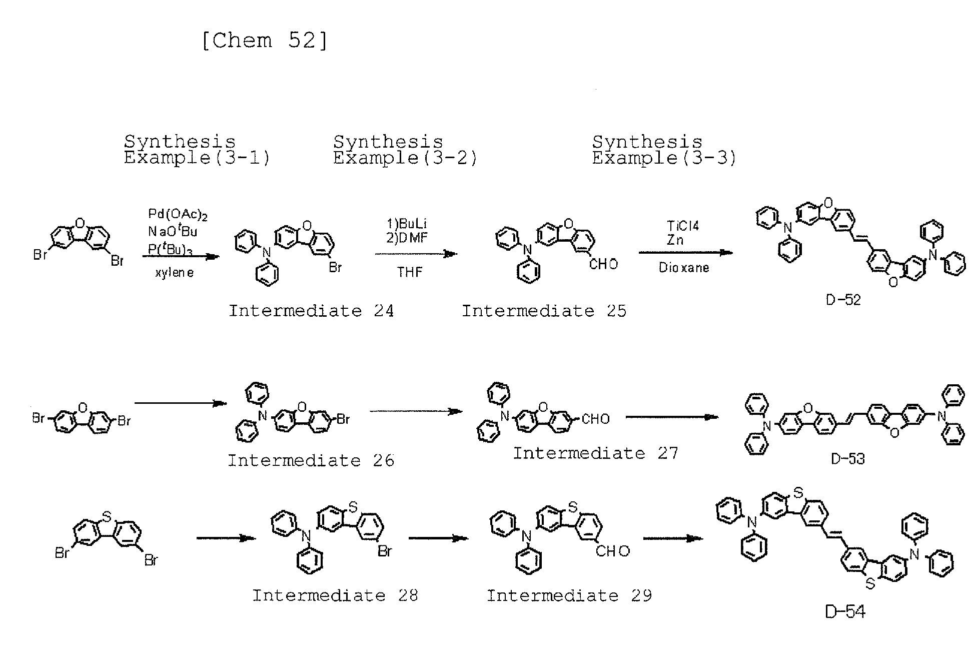

### Synthesis Example 1: synthesis of D-41

### Synthesis Example (1-1)

14.2 g of 6-bromo-2-naphthyl trifluoromethanesulfonate, 8.0 g of trans-2-(4-chlorophenyl)vinylboronic acid, 1.4 g of Pd(PPh₃)₄, 12.8 g (60 mL of clean water) of sodium carbonate, and toluene were loaded into a 500-mL eggplant flask, and then the mixture was subj ected to a reaction in a stream of argon under reflux for 8 hours.
After having been cooled, the reaction solution was filtrated, and the resultant solid was washed with clean water and methanol. The resultant coarse product was dissolved in hot toluene, and the solution was purified by silica gel chromatography (toluene). The resultant solid was dried under reduced pressure. As a result, 6. 6 g of a yellowish white solid were obtained. The solid was identified as Intermediate 1 by field desorption mass spectrometry (FD-MS).

### Synthesis Example (1-2)

In a stream of argon, 6. 6 g of Intermediate 1, 260 mg of Pd(OAc)₂, 3.7 g of t-butoxy sodium, 138 µL of P^{t}Bu₃, 8.2 g of diphenylamine, and dehydrated toluene were loaded into a 300-mL eggplant flask, and the mixture was subjected to a reaction under reflux for 8 hours.
After having been cooled, the reaction solution was filtrated, and the resultant solid was washed with clean water and methanol. The resultant coarse product was dissolved in hot toluene, and the solution was purified by silica gel chromatography (toluene). The resultant solid was recrystallized (toluene) twice. The resultant solid was washed with n-hexane and dried under reduced pressure. As a result, 2.3 g of a yellow solid were obtained. The solid was identified as D-41 by FD-MS.

### Synthesis Example 2: synthesis of D-42

Synthesis was performed in the same manner as in Synthesis Example (1-1) except that 2,6-dibromo-4,8-dimethylnaphthalene was used instead of 6-bromo-2-naphthyl trifluoromethanesulfonate. The resultant was identified as Intermediate 2 by FD-MS.
Synthesis was performed in the same manner as in Synthesis Example (1-2) except that Intermediate 2 was used instead of Intermediate 1 and bis(β-naphthyl)amine was used instead of diphenylamine. The resultant was identified as D-42 by FD-MS.

### Synthesis Example 3: synthesis of D-43

Synthesis was performed in the same manner as in Synthesis Example (1-1) except that 2,6-dibromo-4,8-diphenylnaphthalene was used instead of 6-bromo-2-naphthyltrifluoromethanesulfonate. The resultant was identified as Intermediate 3 by FD-MS.
Synthesis was performed in the same manner as in Synthesis Example (1-2) except that Intermediate 3 was used instead of Intermediate 1 and bis(3,5-dimethylphenyl)amine was used instead of diphenylamine. The resultant was identified as D-43 by FD-MS.

### Synthesis Example 4: synthesis of D-44

Synthesis was performed in the same manner as in Synthesis Example (1-1) except that 3,7-dibromodibenzofuran was used instead of 6-bromo-2-naphthyl trifluoromethanesulfonate. The resultant was identified as Intermediate 4 by FD-MS.
Synthesis was performed in the same manner as in Synthesis Example (1-2) except that Intermediate 4 was used instead of Intermediate 1. The resultant was identified as D-44 by field desorption mass spectrometry (FD-MS).

### Synthesis Example 5: synthesis of D-45

Synthesis was performed in the same manner as in Synthesis Example (1-1) except that 1,4-dibromonaphthalene was used instead of 6-bromo-2-naphthyl trifluoromethanesulfonate. The resultant Was identified as Intermediate 5 by FD-MS.
Synthesis was performed in the same manner as in Synthesis Example (1-2) except that Intermediate 5 was used instead of Intermediate 1 and 4-isopropylphenyl-4-ethylphenylamine was used instead of diphenylamine. The resultant was identified as D-45 by FD-MS.

### Synthesis Example 6: synthesis of D-46

Synthesis was performed in the same manner as in Synthesis Example (1-1) except that 2,8-dibromodibenzofuran was used instead of 6-bromo-2-naphthyl trifluoromethanesulfonate. The resultant was identified as Intermediate 6 by FD-MS.
Synthesis was performed in the same manner as in Synthesis Example (1-2) except that Intermediate 6 was used instead of Intermediate 1 and biphenylamine was used instead of diphenylamine. The resultant was identified as D-46 by FD-MS.

### Synthesis Example 7: synthesis of D-47

### Synthesis Example (2-1)

In a stream of argon, 48.0 g of 6-bromo-2-naphthyl trifluoromethanesulfonate, 5.5 g of [1,1'-bis(diphenylphosphino)ferrocene]dichloropalladium, 11.7 g of lithium bromide, and dehydrated THF were loaded into a 1, 000-mL eggplant flask, and the mixture was cooled to -20°C. Then, 135 mL of a solution (1 mol/L) of methylmagnesium bromide in THF were added to the flask. The temperature of the resultant mixture was gradually increased, and the mixture was subjected to a reaction at room temperature for 2 hours and then at 80°C for 4 hours.
After the resultant had been cooled, a 10% aqueous solution of hydrochloric acid and toluene were added to the resultant for separation and extraction. After that, an organic layer was washed with baking soda water and a saturated salt solution, and was dried with magnesium sulfate. A coarse product obtained by concentrating the dried product was purified by silica gel chromatography (methylene chloride and hexane). The resultant solid was further purified by silica gel chromatography (hexane). The resultant solid was dried under reduced pressure. As a result, 15.0 g of a white solid were obtained. The solid was identified as Intermediate 7 by FD-MS.

### Synthesis Example (2-2)

In a stream of argon, 15.0 g of Intermediate 7, 12.1 g of N-bromosuccinimide, 100 mg of azobisisobutyronitrile, and carbon tetrachloride were loaded into a 300-mL eggplant flask, and the mixture was subjected to a reaction under reflux for 3 hours.
After the resultant had been cooled, clean water and methylene chloride were added to the resultant for separation and extraction. After that, an organic layer was washed with clean water and a saturated salt solution, and was dried with sodium sulfate. A coarse product obtained by concentrating the dried product was purified by silica gel chromatography (methylene chloride and hexane). The resultant solid was dried under reduced pressure. As a result, 16.2 g of a white solid were obtained. The solid was identified as Intermediate 8 by FD-MS.

### Synthesis Example (2-3)

In a stream of argon, 16.0 g of Intermediate 8 and 14 g of triethyl phosphite were loaded into a 300-mL eggplant flask, and the temperature of the mixture was increased. Ethyl bromide produced in the foregoing was removed by distillation, and then unreacted triethyl phosphite was removed by distillation under reduced pressure. As a result, 19.2 g of a white solid were obtained. The solid was identified as Intermediate 9 by field desorption mass spectrometry (FD-MS).

### Synthesis Example (2-4)

In a stream of argon, 30.0 g of 1,4-dibromonaphthalene and 300 mL of dehydrated THF were loaded into a 1,000-mL eggplant flask, and the mixture was cooled to -65°C. After that, 72 mL of a solution (1.6 M) of n-butyllithium in hexane were charged into the flask, and the resultant mixture was subj ected to a reaction for 30 minutes. After that, 25 mL of dehydrated N,N-dimethylformamide were dropped into the flask, and the temperature of the resultant mixture was gradually increased. Then, the mixture was subjected to a reaction at room temperature for 4 hours.
Then, 4N hydrochloric acid and toluene were added to the resultant for separation and extraction. After that, an organic layer was washed with clean water and a saturated salt solution, and was dried with sodium sulfate. A coarse product obtained by concentrating the dried product was purified by silica gel chromatography (toluene), and the resultant solid was dried under reduced pressure. As a result, 20. 8 g of a white solid were obtained. The solid was identified as Intermediate 10 by FD-MS.

### Synthesis Example (2-5)

In a stream of argon, 7.1 g of Intermediate 9, 4.7 g of Intermediate 10, and dehydrated THF were loaded into a 500-mL eggplant flask, and the mixture was cooled to -50°C. After that, 2.5 g of t-butoxy potassium were added to the flask, and the temperature of the resultant mixture was gradually increased to room temperature. Then, the mixture was subjected to a reaction for 7 hours.
The reaction solution was filtrated, and the resultant solid was washed with clean water and methanol and dried under reduced pressure. As a result, 10.8 g of a white solid were obtained. The solid was identified as Intermediate 11 by FD-MS.
Synthesis was performed in the same manner as in Synthesis Example (1-2) except that Intermediate 11 was used instead of Intermediate 1. The resultant was identified as D-47 by FD-MS.

### Synthesis Example 8: synthesis of D-48

Synthesis was performed in the same manner as in Synthesis Example (2-1) except that 3,7-dibenzofuran was used instead of 6-bromo-2-naphthyl trifluoromethanesulfonate. The resultant was identified as Intermediate 12 by FD-MS.
Synthesis was performed in the same manner as in Synthesis Example (2-2) except that Intermediate 12 was used instead of Intermediate 7. The resultant was identified as Intermediate 13 by FD-MS.
Synthesis was performed in the same manner as in Synthesis Example (2-3) except that Intermediate 13 was used instead of Intermediate 8. The resultant was identified as Intermediate 14 by FD-MS.
Synthesis was performed in the same manner as in Synthesis Example (2-5) except that Intermediate 14 was used instead of Intermediate 9. The resultant was identified as Intermediate 15 by FD-MS.
Synthesis was performed in the same manner as in Synthesis Example (1-2) except that Intermediate 15 was used instead of Intermediate 1. The resultant was identified as D-48 by FD-MS.

### Synthesis Example 9: synthesis of D-49

Synthesis was performed in the same manner as in Synthesis Example (2-1) except that 2-bromo-7-iodo-9,9-dimethylfluorene was used instead of 6-bromo-2-naphthyl trifluoromethanesulfonate. The resultant was identified as Intermediate 16 by FD-MS.
Synthesis was performed in the same manner as in Synthesis Example (2-2) except that Intermediate 16 was used instead of Intermediate 7. The resultant was identified as Intermediate 17 by FD-MS.
Synthesis was performed in the same manner as in Synthesis Example (2-3) except that Intermediate 17 was used instead of Intermediate 8. The resultant was identified as Intermediate 18 by FD-MS.
Synthesis was performed in the same manner as in Synthesis Example (2-4) except that 4,4'-dibromobiphenyl was used instead of 1,4-dibromonaphthalene. The resultant was identified as Intermediate 19 by FD-MS.
Synthesis was performed in the same manner as in Synthesis Example (2-5) except that Intermediate 18 was used instead of Intermediate 9 and Intermediate 19 was used instead of Intermediate 10. The resultant was identified as Intermediate 20 by FD-MS.
Synthesis was performed in the same manner as in Synthesis Example (1-2) except that Intermediate 20 was used instead of Intermediate 1. The resultant was identified as D-49 by FD-MS.

### Synthesis Example 10: synthesis of D-50

Synthesis was performed in the same manner as in Synthesis Example (2-5) except that Intermediate 14 was used instead of Intermediate 9 and Intermediate 19 was used instead of Intermediate 10. The resultant was identified as Intermediate 21 by FD-MS.
Synthesis was performed in the same manner as in Synthesis Example (1-2) except that Intermediate 21 was used instead of Intermediate 1. The resultant was identified as D-50 by FD-MS.

### Synthesis Example 11: synthesis of D-51

Synthesis was performed in the same manner as in Synthesis Example (2-4) except that 3,7-dibromobiphenyldibenzofuran was used instead of 1,4-dibromonaphthalene. The resultant was identified as Intermediate 22 by FD-MS.
Synthesis was performed in the same manner as in Synthesis Example (2-5) except that Intermediate 18 was used instead of Intermediate 9 and Intermediate 22 was used instead of Intermediate 10. The resultant was identified as Intermediate 23 by FD-MS.
Synthesis was performed in the same manner as in Synthesis Example (1-2) except that Intermediate 23 was used instead of Intermediate 1. The resultant was identified as D-51 by FD-MS.

### Synthesis Example 12: synthesis of D-52

### Synthesis Example (3-1)

13.04 g of 2,8-dibromodibenzofuran, 6.77 g of diphenylamine, 0.180 g of palladium(II) acetate, 4.610 g of sodium t-butoxide, and 700 ml of xylene were loaded into a 1-1 three-necked flask, and 32 ml of a 0.1-mol/l solution of tri-t-butylphosphine in xylene were slowly dropped into the flask while the mixture was stirred with a stirrer. Then, a nitrogen atmosphere was established in the container, and the resultant mixture was subjected to a reaction at 110°C for 7.5 hours. After that, the resultant was subjected to each of a separation treatment and purification by column chromatography three times. As a result, 10.23 g of a white solid were obtained. The solid was identified as Intermediate 24 by FD-MS.

### Synthesis Example (3-2)

10.23 g of Intermediate 24 thus obtained were loaded into a 1-L four-necked flask, and a pressure reduction and a pressure recovery with an argon gas were each repeated three times so that the air in the system was replaced with argon. Next, 300 ml of dehydrated tetrahydrofuran were added to the flask, and the mixture was stirred. After that, the mixture was cooled to around -65°C with a dry ice/acetone bath, and 28.5 ml of dehydrated dimethylformamide were dropped into the flask over about 30 minutes . The resultant mixture was continuously subjected to a reaction at -65°C for 2 hours. After that, the temperature of the mixture was increased to room temperature, and the mixture was subjected to a reaction for an additional two hours before the stirring was stopped. After the reaction liquid had been left at rest for about 18 hours, 100 ml of 1N hydrochloric acid were added to the reaction liquid. Next, the reaction liquid was extracted with ethyl acetate and water, and the resultant organic layer was concentrated. As a result, 8.3 g of a white solid were obtained. The solid was identified as Intermediate 25 by FD-MS.

### Synthesis Example (3-3)

First, 2. 73 g of zinc and 150 ml of dehydrated tetrahydrofuran were loaded into a 500-ml three-necked flask. While the reaction vessel was cooled in an ice bath, 2.34 ml of titanium tetrachloride and 11. 4 ml of pyridine were dropped into the vessel, and the mixture was stirred with a stirrer. Next, 3.27 g of Intermediate 25 were dissolved in 100 ml of dehydrated tetrahydrofuran, and the solution was dropped into the vessel over 30 minutes. After that, a nitrogen atmosphere was established in the reaction vessel, and the resultant mixture was subj ected to a reaction at room temperature for 20 minutes and at 60°C for 5 hours. A 10% aqueous solution of potassium carbonate was added to the resultant, and the mixture was subjected to each of a separation treatment and purification by column chromatography five times. As a result, 1.04 g of a yellowish white solid were obtained. The solid was identified as D-52 by FD-MS.

### Synthesis Example 13: synthesis of D-53

Synthesis was performed in the same manner as in Synthesis Example (3-1) except that 3,7-dibromodibenzofuran was used instead of 2,8-dibromodibenzofuran. The resultant was identified as Intermediate 26 by FD-MS.
Synthesis was performed in the same manner as in Synthesis Example (3-2) except that Intermediate 26 was used instead of Intermediate 24. The resultant was identified as Intermediate 27 by FD-MS.
Synthesis was performed in the same manner as in Synthesis Example (3-3) except that Intermediate 27 was used instead of Intermediate 25. The resultant was identified as D-53 by FD-MS.

### Synthesis Example 14: synthesis of D-54

Synthesis was performed in the same manner as in Synthesis Example (3-1) except that 2,8-dibromodibenzothiophene was used instead of 2,8-dibromodibenzofuran. The resultant was identified as Intermediate 28 by FD-MS.
Synthesis was performed in the same manner as in Synthesis Example (3-2) except that Intermediate 28 was used instead of Intermediate 24. The resultant was identified as Intermediate 29 by FD-MS.
Synthesis was performed in the same manner as in Synthesis Example (3-3) except that Intermediate 29 was used instead of Intermediate 25. The resultant was identified as D-54 by FD-MS.

### Example 1

A transparent electrode formed of indium tin oxide and having a thickness of 120 nm was provided on a glass substrate measuring 25 mm by 75 mm by 1.1 mm. The transparent electrode functions as an anode. Subsequently, the glass substrate was washed by being irradiated with UV and ozone. After that, the substrate was placed in a vacuum deposition apparatus.
First, N',N"-bis[4-(diphenylamino)phenyl]-N',N"-diphenylbiphenyl-4,4' -diamine was deposited from the vapor so as to serve as a hole injecting layer having a thickness of 60 nm. After that, N,N,N',N'-tetrakis(4-biphenyl)-4,4'-benzidine was deposited from the vapor onto the layer so as to serve as a hole transporting layer having a thickness of 20 nm. Next, the anthracene derivative (2a-1) as a host material and the aromatic amine derivative (D-44) as a doping material were simultaneously deposited from the vapor at a mass ratio of 40:2 so that a light emitting layer having a thickness of 40 nm might be formed.
Tris(8-hydroxyquinolinato)aluminum was deposited from the vapor onto the light emitting layer so as to serve as an electron injecting layer having a thickness of 20 nm.
Next, lithium fluoride was deposited from the vapor so as to have a thickness of 1 nm, and then aluminum was deposited from the vapor so as to have a thickness of 150 nm. Thus, an organic EL device was produced. It should be noted that the aluminum/lithium fluoride functions as a cathode.
The performance (emission luminance) of the organic EL device thus obtained when the device was driven at a current density of 10 mA/cm², and the half lifetime of the device at an initial luminance of 100 cd/cm² were measured. Table 1 shows the results.

### Examples 2 to 15

Organic EL devices were each produced in the same manner as in Example 1 except that any one of the aromatic amine derivatives shown in Tables 1 to 3 was used instead of the aromatic amine derivative (D-44), and the devices were each evaluated in the same manner as in Example 1. Tables 1 to 3 show the results.

### Examples 16 and 17

Organic EL devices were each produced in the same manner as in Example 1 except that any one of the compounds shown in Table 4 was used instead of the anthracene derivative (2a-1), and the devices were each evaluated in the same manner as in Example 1. Table shows the results.

### Comparative Examples 1 to 3

Organic EL devices were each produced in the same manner as in Example 1 except that any one of the following compounds was selected as shown in Table 4 instead of the compound of the aromatic amine derivative (D-44), and the devices were each evaluated in the same manner as in Example 1. Table 4 shows the results.

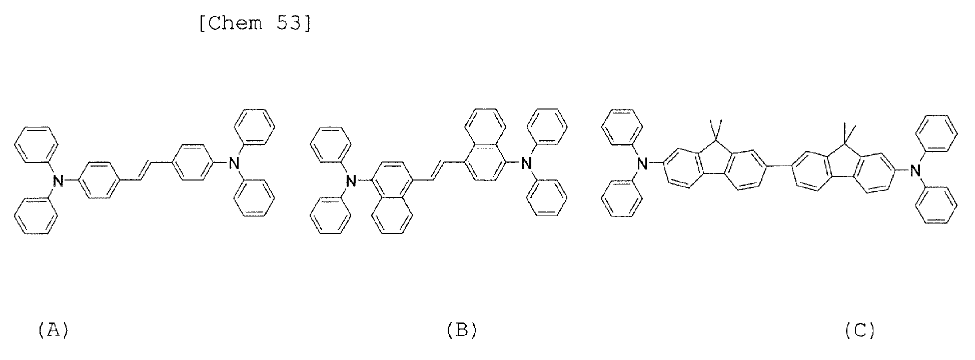

[Table 1]

**Table 1**

| | Example 1 | Example 2 | Example 3 | Example 4 | Example 5 |
|---|---|---|---|---|---|
| Host material | 2a-1 | 2a-1 | 2a-1 | 2a-1 | 2a-1 |
| Doping material | D-44 | D-45 | D-42 | D-49 | D-36 |
| Voltage at which device is driven (V) | 6.0 | 6.0 | 6.0 | 6.0 | 6.0 |
| Luminescent color | Blue | Blue | Blue | Blue | Blue |
| Emission luminance (cd/m²) | 680 | 700 | 720 | 590 | 650 |
| Half lifetime (hours) | >10,000 | >10,000 | >10,000 | >10,000 | >10,000 |

**Table 2**

| | Example 6 | Example 7 | Example 8 | Example 9 | Example 10 |
|---|---|---|---|---|---|
| Host material | 2a-1 | 2a-1 | 2a-1 | 2a-1 | 2a-1 |
| Doping material | D-51 | D-53 | D-52 | D-48 | D-27 |
| Voltage at which device is driven (V) | 6.0 | 6.0 | 6.0 | 6.0 | 6.0 |
| Luminescent color | Blue | Blue | Blue | Blue | Blue |
| Emission luminance (cd/m²) | 600 | 610 | 550 | 700 | 540 |
| Half lifetime (hours) | >10,000 | >10,000 | >10,000 | >10,000 | >10,000 |

[Table 2]

**Table 3**

| | Example 11 | Example 12 | Example 13 | Example 14 | Example 15 |
|---|---|---|---|---|---|
| Host material | 2a-1 | 2a-1 | 2a-1 | 2a-1 | 2a-1 |
| Doping material | D-13 | D-14 | D-68 | D-50 | D-43 |
| Voltage at which device is driven (V) | 6.0 | 6.0 | 6.0 | 6.0 | 6.0 |
| Luminescent color | Blue | Blue | Blue | Blue | Blue |
| Emission luminance (cd/m²) | 650 | 550 | 760 | 660 | 730 |
| Half lifetime (hours) | >10,000 | >10,000 | >10,000 | >10,000 | >10,000 |

**Table 4**

| | Example 16 | Example 17 | Comparative Example 1 | Comparative Example 2 | Comparative Example 3 |
|---|---|---|---|---|---|
| Host material | 2b-9 | 2d-1 | 2a-1 | 2a-1 | 2a-1 |
| Doping material | D-44 | D-44 | (A) | (B) | (C) |
| Voltage at which device is driven (V) | 6.0 | 6.0 | 6.0 | 6.0 | 6.0 |
| Luminescent color | Blue | Blue | Blue | Blue | Blue |
| Emission luminance (cd/m²) | 680 | 650 | 330 | 300 | 400 |
| Half lifetime (hours) | 8,000 | 7,000 | 4,000 | 3,000 | 5,000 |

### Industrial Applicability

As described above in detail, the organic EL device using the aromatic amine derivative of the present invention provides emission luminance sufficient for practical use even at a low applied voltage, has high luminous efficiency, hardly deteriorates even after long-term use, and has a long lifetime. Therefore, the organic EL device is useful as a light source such as a flat luminous body of a wall hanging television or backlight for a display.

## Claims

1. An aromatic amine derivative represented by the following general formula (I): 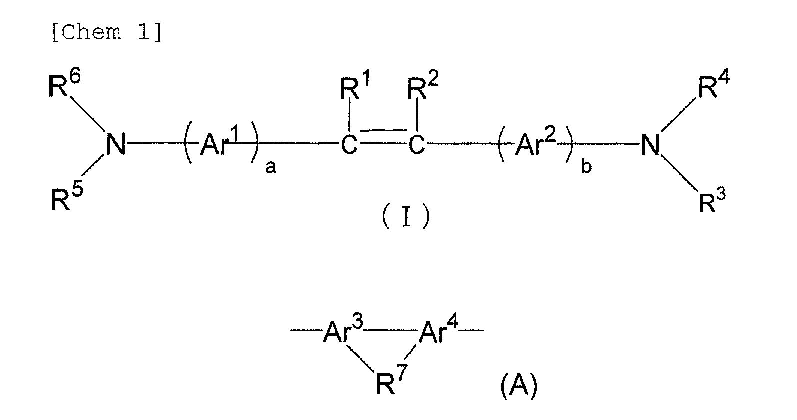 where:
R¹ and R² each independently represent a hydrogen atom, a substituted or unsubstituted alkyl group having 1 to 50 carbon atoms, or a substituted or unsubstituted aryl group having 5 to 50 carbon atoms;
R³ to R⁶ each independently represent a substituted or unsubstituted alkyl group having 1 to 50 carbon atoms, a substituted or unsubstituted aryl group having 5 to 50 carbon atoms, or a substituted or unsubstituted heterocyclic group having 5 to 50 carbon atoms;
Ar¹ and Ar² each independently represent a substituted or unsubstituted arylene group having 5 to 50 carbon atoms, a substituted or unsubstituted, divalent heterocyclic group having 5 to 50 carbon atoms, or a group represented by the above general formula (A) where Ar³ and Ar⁴ each independently represent a substituted or unsubstituted arylene group having 5 to 50 carbon atoms, or a substituted or unsubstituted, divalent heterocyclic group having 5 to 50 carbon atoms, and R⁷ represents a substituted or unsubstituted alkylene group having 1 to 50 carbon atoms, -O-, -S-, -SO₂-, -SiR⁸R⁹-, or -NR¹⁰- where R⁸, R⁹, and R¹⁰ each independently represent a substituted or unsubstituted alkyl group having 1 to 50 carbon atoms, or a substituted or unsubstituted aryl group having 5 to 50 carbon atoms;
a and b each independently represent an integer of 1 to 3, when a represents 2 or more, multiple Ar¹'s may be identical to or different from each other, and when b represents 2 or more, multiple Ar²'s may be identical to or different from each other; and
R¹ and R², R¹ and Ar¹, R² and Ar², R³ and R⁴, R⁵ and R⁶, Ar¹ and R⁵ and/or R⁶, or Ar² and R³ and/or R⁴ may be bonded and linked to each other to form a saturated or unsaturated ring,
provided that, when all R³ to R⁶ each represent an unsubstituted phenyl group and a=b=1, at least one of Ar¹ and Ar² represents a group except an unsubstituted 1,4-phenylene group and an unsubstituted 1,4-naphthylene group.

2. The aromatic amine derivative according to claim 1, wherein Ar¹ and Ar² in the general formula (I) each independently represent a group represented by the general formula (A).

3. The aromatic amine derivative according to claim 1, wherein Ar¹ and Ar² in the general formula (I) are identical to each other, and each represent a group represented by the general formula (A).

4. The aromatic amine derivative according to claim 1, wherein, when Ar¹ in the general formula (I) represents a substituted or unsubstituted arylene group having 5 to 50 carbon atoms, or a substituted or unsubstituted, divalent heterocyclic group having 5 to 50 carbon atoms, Ar¹ and Ar² represent different groups.

5. The aromatic amine derivative according to claim 1, wherein the general formula (I) satisfies the following conditions:
R¹ to R⁶, and Ar¹ and Ar² each independently have the same meaning as that described above;
in the formula (A), Ar³ and Ar⁴ each independently have the same meaning as that described above, and R⁷ represents -O-, -S-, -SO₂-, -SiR⁸R⁹-, or -NR¹⁰- where R⁸, R⁹, and R¹⁰ each independently represent a substituted or unsubstituted alkyl group having 1 to 50 carbon atoms, or a substituted or unsubstituted aryl group having 5 to 50 carbon atoms;
a and b each independently represent an integer of 1; and
R¹ and R² may be linked to each other to form a ring except an aromatic ring,
provided that at least one of Ar¹ and Ar² represents a group except a substituted or unsubstituted 1,4-phenylene group, a substituted or unsubstituted 1, 4-naphthylene group, a substituted or unsubstituted 2,6-naphthylene group, and a substituted or unsubstituted 9,10-anthracenylene group.

6. The aromatic amine derivative according to claim 1, wherein the general formula (I) satisfies the following conditions:
R¹ to R⁶ each independently have the same meaning as that described above;
Ar¹ represents a group represented by the above general formula (A) where Ar³, Ar⁴, and R⁷ each independently have the same meaning as that described above;
Ar²'s each independently represent a substituted or unsubstituted arylene group having 5 to 50 carbon atoms, or a substituted or unsubstituted, divalent heterocyclic group having 5 to 50 carbon atoms;
a represents an integer of 1 to 3, and b represents an integer of 2 or 3; and
R¹ and R² may be linked to each other to form a ring except an aromatic ring.

7. The aromatic amine derivative according to claim 1, wherein the aromatic amine derivative is represented by the following general formula (II): 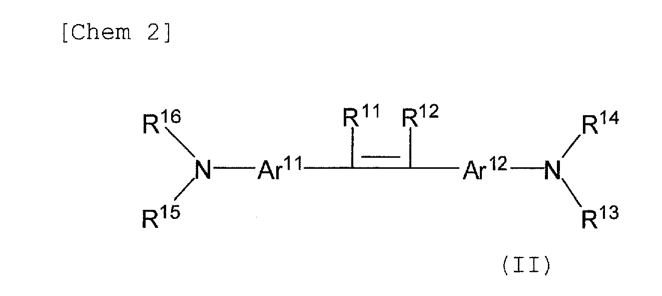 where :
R¹¹ and R¹² each independently have the same meaning as that of each of R¹ and R² ;
R¹³ to R¹⁶ each independently have the same meaning as that of each of R³ to R⁶;
Ar¹¹ and Ar¹² each independently represent a substituted or unsubstituted fused ring group having 10 to 50 carbon atoms, provided that Ar¹¹ and Ar¹² are different from each other; and
R¹¹ and R¹² may be linked to each other to form a ring except an aromatic ring.

8. The aromatic amine derivative according to claim 7, wherein the aromatic amine derivative is represented by the following general formula (III): 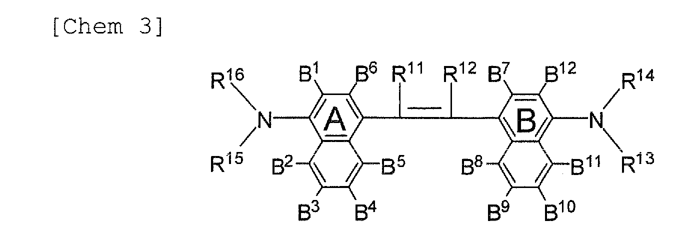 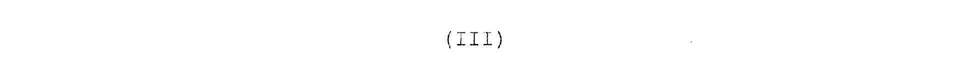 where:
R¹¹ to R¹⁶ each independently have the same meaning as that described above;
a ring A and a ring B each independently represent a substituted or unsubstituted fused ring group having 10 to 50 carbon atoms, the group having a skeleton of a 1,4-naphthalene residue;
B¹ to B¹² each independently represent a hydrogen atom, an alkyl group having 1 to 4 carbon atoms, a cycloalkyl group having 5 or 6 carbon atoms, or an aryl group having 6 to 16 carbon atoms, provided that
groups represented by one or more pairs selected from B¹ and B⁶, B² and B³, B³ and B⁴, B⁴ and B⁵, B⁷ and B¹², B⁸ and B⁹, B⁹ and B¹⁰, and B¹⁰ and B¹¹ are linked to each other to form unsaturated rings, and the unsaturated rings may each independently have a hydrogen atom, an alkyl group having 1 to 4 carbon atoms, a cycloalkyl group having 5 or 6 carbon atoms, or an aryl group having 6 to 16 carbon atoms, and
the ring A and the ring B are different from each other; and
R¹¹ and R¹² may be linked to each other to form a ring except an aromatic ring.

9. The aromatic amine derivative according to claim 7, wherein the aromatic amine derivative is represented by the following general formula (IV): 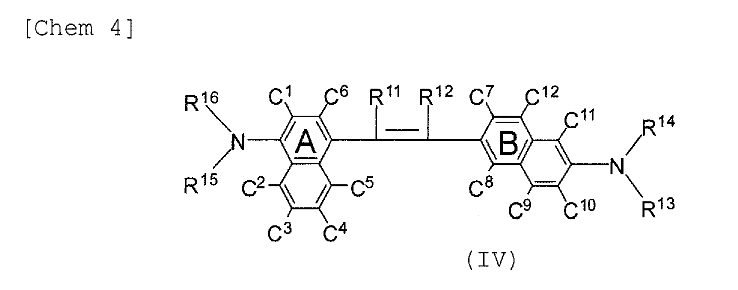 where:
R¹¹ to R¹⁶ each independently have the same meaning as that described above;
C¹ to C¹² each independently represent a hydrogen atom, an alkyl group having 1 to 4 carbon atoms, a cycloalkyl group having 5 or 6 carbon atoms, or an aryl group having 6 to 16 carbon atoms, C¹ and C⁶, C² and C³, C³ and C⁴, C⁴ and C⁵, C⁷ and C¹², or C⁹ and C¹⁰ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

10. The aromatic amine derivative according to claim 7, wherein the aromatic amine derivative is represented by the following general formula (V): 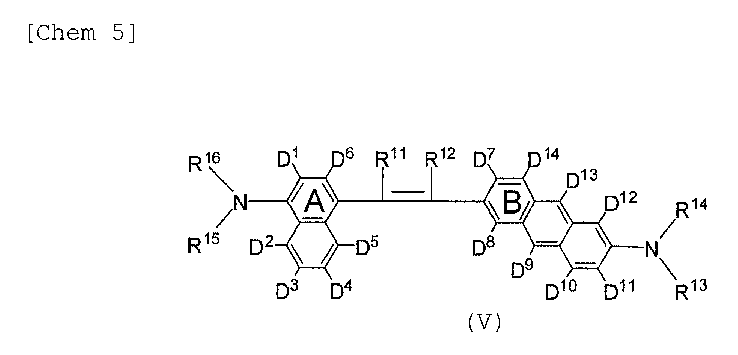 where:
R¹¹ to R¹⁶ each independently have the same meaning as that described above;
D¹ to D¹⁴ each independently represent a hydrogen atom, an alkyl group having 1 to 4 carbon atoms, a cycloalkyl group having 5 or 6 carbon atoms, or an aryl group having 6 to 16 carbon atoms, D¹ and D⁶, D² and D³, D³ and D⁴, D⁴ and D⁵, D⁷ and D¹⁴, or D¹⁰ and D¹¹ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

11. The aromatic amine derivative according to claim 7, wherein the aromatic amine derivative is represented by the following general formula (VI): 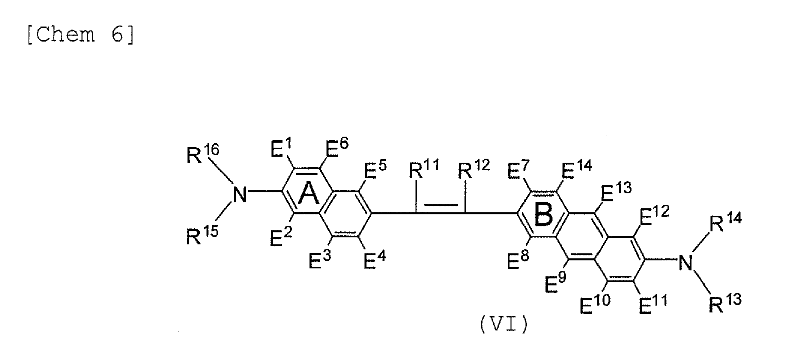 where:
R¹¹ to R¹⁶ each independently have the same meaning as that described above;
E¹ to E¹⁴ each independently represent a hydrogen atom, an alkyl group having 1 to 4 carbon atoms, a cycloalkyl group having 5 or 6 carbon atoms, or an aryl group having 6 to 16 carbon atoms, E¹ and E⁶, E³ and E⁴, E¹⁰ and E¹¹, or E⁷ and E¹⁴ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

12. The aromatic amine derivative according to claim 7, wherein the aromatic amine derivative is represented by the following general formula (VII): 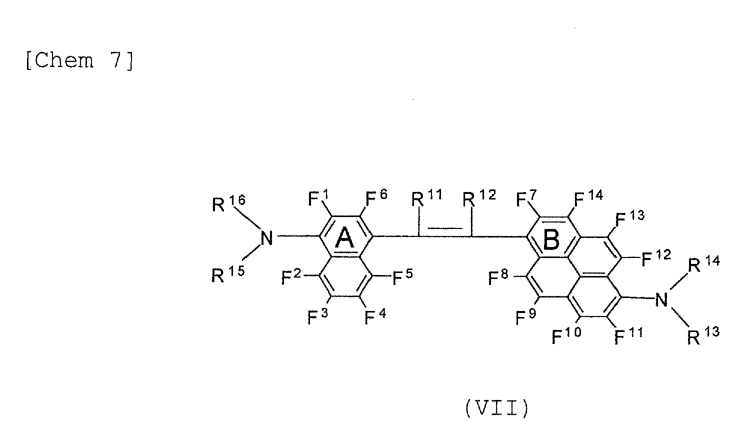 where:
R¹¹ to R¹⁶ each independently have the same meaning as that described above;
F¹ to F¹⁴ each independently represent a hydrogen atom, an alkyl group having 1 to 4 carbon atoms, a cycloalkyl group having 5 or 6 carbon atoms, or an aryl group having 6 to 16 carbon atoms, F¹ and F⁶ F² and F³ F³ and F⁴ F⁴ and F⁵, F⁸ and F⁹, F¹⁰ and F¹¹, F¹² and F¹³, or F¹⁴ and F⁷ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

13. The aromatic amine derivative according to claim 7, wherein the aromatic amine derivative is represented by the following general formula (VIII): 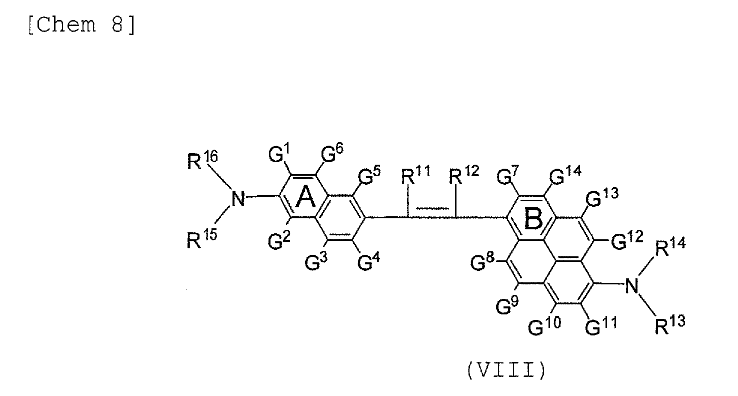 where:
R¹¹ to R¹⁶ each independently have the same meaning as that described above;
G¹ to G¹⁴ each independently represent a hydrogen atom, an alkyl group having 1 to 4 carbon atoms, a cycloalkyl group having 5 or 6 carbon atoms, or an aryl group having 6 to 16 carbon atoms, G¹ and G⁶, G³ and G⁴, G⁸ and G⁹, G¹⁰ and G¹¹, G¹² and G¹³, or G¹⁴ and G⁷ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

14. The aromatic amine derivative according to claim 7, wherein the aromatic amine derivative is represented by the following general formula (IX): 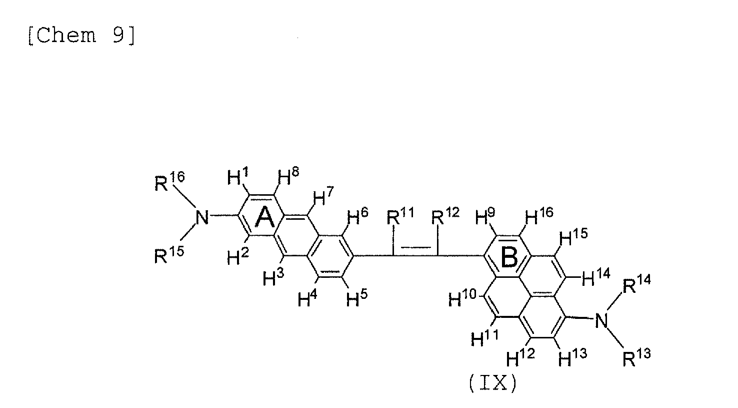 were:
R¹¹ to R¹⁶ each independently have the same meaning as that described above;
H¹ to H¹⁶ each independently represent a hydrogen atom, an alkyl group having 1 to 4 carbon atoms, a cycloalkyl group having 5 or 6 carbon atoms, or an aryl group having 6 to 16 carbon atoms, H¹ and H⁸ H⁴ and H⁵, H¹⁰ and H¹¹, H¹² and H¹³, H¹⁴ and H¹⁵, or H¹⁶ and H⁹ may be linked to each other to form an unsaturated ring, and the unsaturated rings may each independently have a hydrogen atom, an alkyl group having 1 to 4 carbon atoms, a cycloalkyl group having 5 or 6 carbon atoms, or an aryl group having 6 to 16 carbon atoms; and
R¹¹ and R¹² may be linked to each other to form a ring except an aromatic ring.

15. The aromatic amine derivative according to claim 1, wherein the aromatic amine derivative is represented by the following general formula (X): 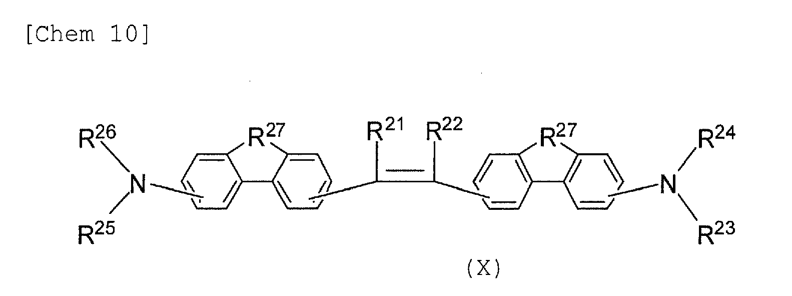 where:
R²¹ and R²² each independently have the same meaning as that of each of R¹ and R²;
R²³ to R²⁶ each independently have the same meaning as that of each of R³ to R⁶;
R²⁷ represents -O-, -S-, -SO₂-, -SiR²⁸R²⁹-, or -NR³⁰- where R²⁸, R²⁹, and R³⁰ each independently represent a substituted or unsubstituted alkyl group having 1 to 50 carbon atoms, or a substituted or unsubstituted aryl group having 5 to 50 carbon atoms; and
R²¹ and R²² may be linked to each other to form a ring except an aromatic ring.

16. The aromatic amine derivative according to claim 1, wherein the aromatic amine derivative is represented by the following general formula (XI): 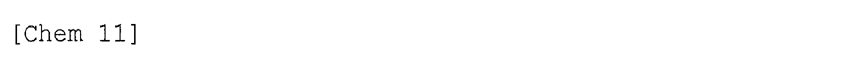 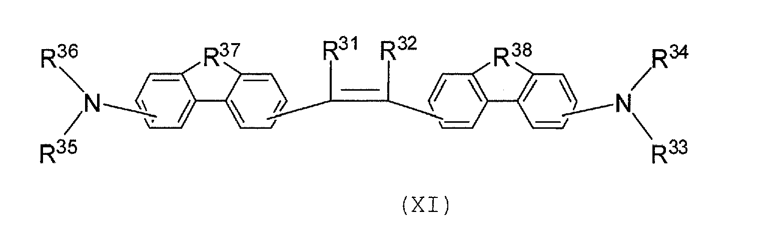 where:
R³¹ and R³² each independently have the same meaning as that of each of R¹ and R²;
R³³ to R³⁶ each independently have the same meaning as that of each of R³ to R⁶;
R³⁷ and R³⁸ each represent -O-, -S-, -SO₂-, -SiR¹³¹R¹³²-, or -NR¹³³- where R¹³¹, R¹³², and R¹³³ each independently represent a substituted or unsubstituted alkyl group having 1 to 50 carbon atoms, or a substituted or unsubstituted aryl group having 5 to 50 carbon atoms, provided that R³⁷ and R³⁸ are different from each other; and
R³¹ and R³² may be linked to each other to form a ring except an aromatic ring.

17. The aromatic amine derivative according to claim 1, wherein the aromatic amine derivative is represented by the following general formula (XII): 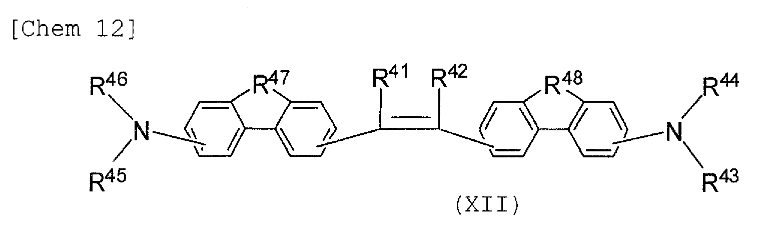 where:
R⁴¹ and R⁴² each independently have the same meaning as that of each of R¹ and R²;
R⁴³ to R⁴⁶ each independently have the same meaning as that of each of R³ to R⁶;
R⁴⁷ represents -O-, -S-, -SO₂-, -SiR¹⁴¹R¹⁴²-, or -NR¹⁴³- where R¹⁴¹ R¹⁴², and R¹⁴³ each independently represent a substituted or unsubstituted alkyl group having 1 to 50 carbon atoms, or a substituted or unsubstituted aryl group having 5 to 50 carbon atoms;
R⁴⁸ represents a substituted or unsubstituted alkylene group having 1 to 50 carbon atoms; and
R⁴¹ and R⁴² may be linked to each other to form a ring except an aromatic ring.

18. The aromatic amine derivative according to claim 1, wherein the aromatic amine derivative is represented by the following general formula (XIII): 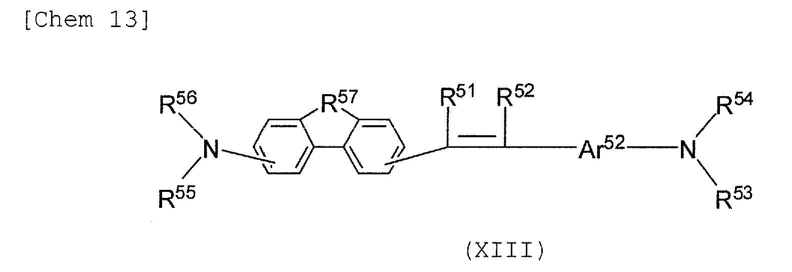 where:
R⁵¹ and R⁵² each independently have the same meaning as that of each of R¹ and R²;
R⁵³ to R⁵⁶ each independently have the same meaning as that of each of R³ to R⁶;
R⁵⁷ represents -O-, -S-, -SO₂-, -SiR¹⁵¹R¹⁵²-, or -NR¹⁵³- where R¹⁵¹ R¹⁵², and R¹⁵³ each independently represent a substituted or unsubstitutedalkyl group having 1 to 50 carbon atoms, or a substituted or unsubstituted aryl group having 5 to 50 carbon atoms;
Ar⁵² represents a substituted or unsubstituted fused ring group having 10 to 50 carbon atoms; and
R⁵¹ and R⁵² may be linked to each other to form a ring except an aromatic ring.

19. The aromatic amine derivative according to claim 1, wherein the aromatic amine derivative is represented by the following general formula (XIV): 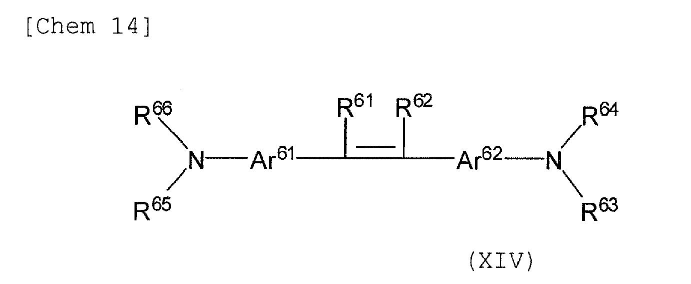 where:
R⁶¹ and R⁶² each independently have the same meaning as that of each of R¹ and R²;
R⁶³ to R⁶⁶ each independently have the same meaning as that of each of R³ to R⁶;
Ar⁶¹ represents a substituted or unsubstituted fused ring group having 10 to 50 carbon atoms;
Ar⁶² represents a substituted or unsubstituted phenylene group; and
R⁶¹ and R⁶² may be linked to each other to form a ring except an aromatic ring.

20. The aromatic amine derivative according to claim 1, wherein the aromatic amine derivative is represented by the following general formula (XV) : 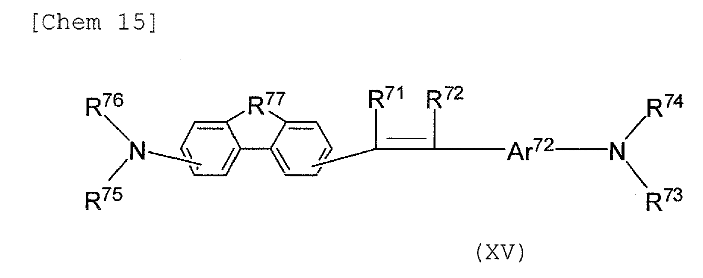 where:
R⁷¹ and R⁷² each independently have the same meaning as that of each of R¹ and R²;
R⁷³ to R⁷⁶ each independently have the same meaning as that of each of R³ to R⁶;
R⁷⁷ represents -O-, -S-, -SO₂-, -SiR¹⁷¹R¹⁷²-, or -NR¹⁷³- where R¹⁷¹, R¹⁷², and R¹⁷³ each independently represent a substituted or unsubstituted alkyl group having 1 to 50 carbon atoms, or a substituted or unsubstituted aryl group having 5 to 50 carbon atoms;
Ar⁷² represents a substituted or unsubstituted phenylene group; and
R⁷¹ and R⁷² may be linked to each other to form a ring except an aromatic ring.

21. The aromatic amine derivative according to claim 1, wherein the aromatic amine derivative is represented by the following general formula (XVI): 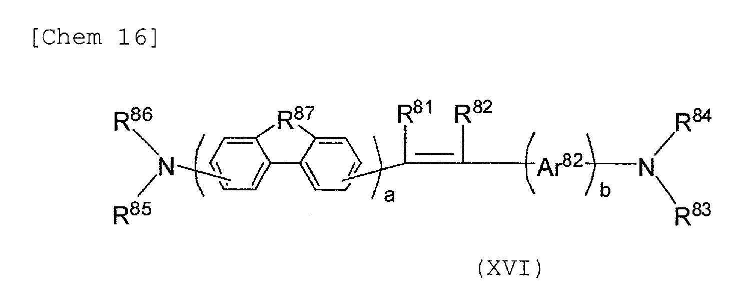 where:
R⁸¹ and R⁸² each independently have the same meaning as that of each of R¹ and R²;
R⁸³ to R⁸⁶ each independently have the same meaning as that of each of R³ to R⁶;
R⁸⁷ represents a substituted or unsubstituted alkylene group having 1 to 50 carbon atoms, -O-, -S-, -SO2-, -SiR¹⁸¹R¹⁸²-, or -NR¹⁸³- where R¹⁸¹, R¹⁸², and R¹⁸³ each independently represent a substituted or unsubstituted alkyl group having 1 to 50 carbon atoms, or a substituted or unsubstituted aryl group having 5 to 50 carbon atoms;
Ar⁸² represents a substituted or unsubstituted arylene group having 5 to 50 carbon atoms, or a substituted or unsubstituted, divalent heterocyclic group having 5 to 50 carbon atoms;
a represents an integer of 1 to 3, and b represents an integer of 2 or 3; and
R⁷¹ and R⁷² may be linked to each other to form a ring except an aromatic ring.

22. The aromatic amine derivative according to claim 1, wherein the aromatic amine derivative is represented by the following general formula (XVII) : 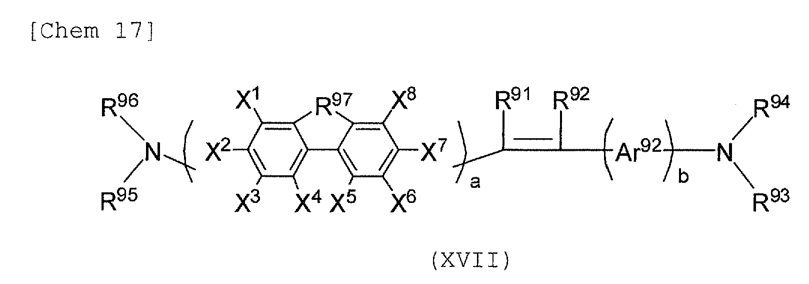 where:
R⁹¹ and R⁹² each independently have the same meaning as that of each of R¹ and R²;
R⁹³ to R⁹⁶ each independently have the same meaning as that of each of R³ to R⁶;
R⁹⁷ represents a substituted or unsubstituted alkylene group having 1 to 50 carbon atoms, -O-, -S-, -SO₂-_{.} -SiR¹⁹¹R¹⁹²-, or -NR¹⁹³- where R¹⁹¹, R¹⁹², and R¹⁹³ each independently represent a substituted or unsubstituted alkyl group having 1 to 50 carbon atoms, or a substituted or unsubstituted aryl group having 5 to 50 carbon atoms;
X¹ to X⁸ each independently represent a single bond, a hydrogen atom, an alkyl group having 1 to 4 carbon atoms, a cycloalkyl group having 5 or 6 carbon atoms, or an aryl group having 6 to 16 carbon atoms, provided that groups represented by one or more pairs selected from X¹ and X², X² and X³, X³ and X⁴, X⁵ and X⁶, X⁶ and X⁷, and X⁷ and X⁸ are linked to each other to form unsaturated rings, and the unsaturated rings may each independently have a single bond, a hydrogen atom, an alkyl group having 1 to 4 carbon atoms, a cycloalkyl group having 5 or 6 carbon atoms, or an aryl group having 6 to 16 carbon atoms;
Ar⁹² represents a substituted or unsubstituted arylene group having 5 to 50 carbon atoms, or a substituted or unsubstituted, divalent heterocyclic group having 5 to 50 carbon atoms;
a represents an integer of 1 to 3, and b represents an integer of 1 to 3; and
R⁹¹ and R⁹² may be linked to each other to form a ring except an aromatic ring.

23. The aromatic amine derivative according to claim 1, wherein the aromatic amine derivative is represented by the following general formula (XVIII): 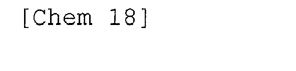 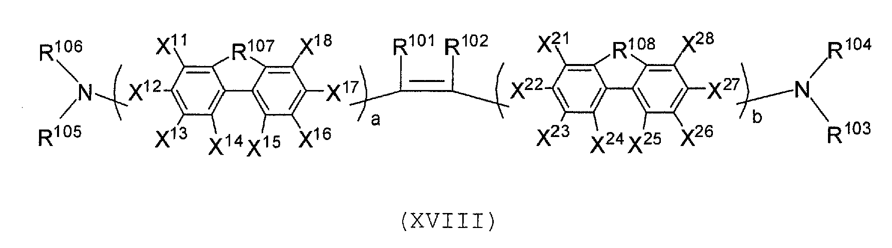 where:
R¹⁰¹ and R¹⁰² each independently have the same meaning as that of each of R¹ and R²;
R¹⁰³ to R¹⁰⁶ each independently have the same meaning as that of each of R³ to R⁶;
R¹⁰⁷ and R¹⁰⁸ each independently represent a substituted or unsubstituted alkylene group having 1 to 50 carbon atoms, -O-, -S-, -SO₂-, -SiR¹⁹¹R¹⁹²⁰-, or -NR¹⁹³- where R¹⁹¹, R¹⁹², and R¹⁹³ each independently represent a substituted or unsubstituted alkyl group having 1 to 50 carbon atoms, or a substituted or unsubstituted aryl group having 5 to 50 carbon atoms;
X¹¹ to X¹⁸ and X²¹ to X²⁸ each independently represent a single bond, a hydrogen atom, an alkyl group having 1 to 4 carbon atoms, a cycloalkyl group having 5 or 6 carbon atoms, or an aryl group having 6 to 16 carbon atoms, provided that groups represented by one or more pairs selected from X¹¹ and X¹², X¹² and X¹³, X¹³ and X¹⁴, X¹⁵ and X¹⁶, X¹⁶ and X¹⁷, X¹⁷ and X¹⁸, X²¹ and X²², X²² and X²³, X²³ and X²⁴, X²⁵ and X²⁶, X²⁶ and X²⁷, and X²⁷ and X²⁸ are linked to each other to form unsaturated rings, and the unsaturated rings may each independently have a single bond, a hydrogen atom, an alkyl group having 1 to 4 carbon atoms, a cycloalkyl group having 5 or 6 carbon atoms, or an aryl group having 6 to 16 carbon atoms;
a represents an integer of 1 to 3, and b represents an integer of 1 to 3; and
R¹⁰¹ and R¹⁰² may be linked to each other to form a ring except an aromatic ring.

24. The aromatic amine derivative according to any one of claims 7 to 23, wherein R¹³ to R¹⁶, R²³ to R²⁶, R³³ to R³⁶, R⁴³ to R⁴⁶, R⁵³ to R⁵⁶, R⁶³ to R⁶⁶, R⁷³ to R⁷⁶, R⁸³ to R⁸⁶, R⁹³ to R⁹⁶, and R¹⁰³ to R¹⁰⁶ in the general formulae (II) to (XVIII) each represent an unsubstituted phenyl group.

25. The aromatic amine derivative according to claim 1, wherein the aromatic amine derivative comprises a doping material for an organic electroluminescence device.

26. An organic electroluminescence device, comprising an organic thin film layer formed of one or more layers including at least a light emitting layer and interposed between a cathode and an anode,
wherein at least one layer of the organic thin film layer contains the aromatic amine derivative according to claim 1.

27. The organic electroluminescence device according to claim 26, wherein the light emitting layer contains the aromatic amine derivative.

28. The organic electroluminescence device according to claim 17, wherein the light emitting layer contains the aromatic amine derivative and a compound having a structure having an anthracene central skeleton and represented by the following general formula (2a) : 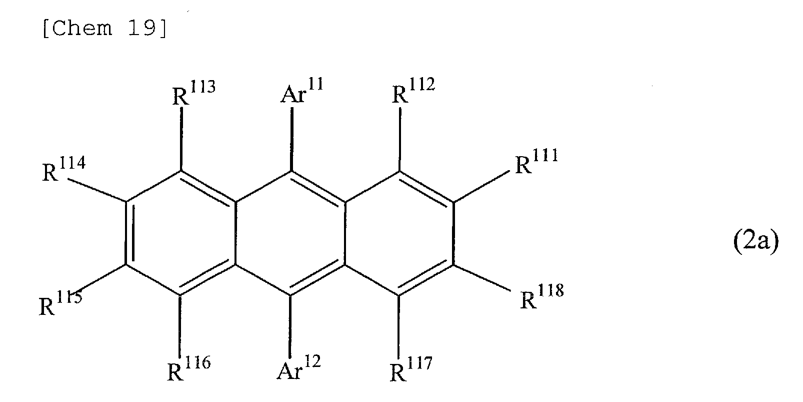 where:
Ar¹¹ and Ar¹² each independently represent a group derived from a substituted or unsubstituted aromatic ring having 6 to 20 carbon atoms; and
R¹¹¹ to R¹¹⁸ each independently represent a group selected from a hydrogen atom, a substituted or unsubstituted aryl group having 6 to 50 carbon atoms, a substituted or unsubstituted heteroaryl group having 4 to 50 carbon atoms, a substituted or unsubstituted alkyl group having 1 to 50 carbon atoms, a substitutedorunsubstituted cycloalkyl group having 3 to 50 carbon atoms, a substituted or unsubstituted alkoxy group having 1 to 50 carbon atoms, a substituted or unsubstituted aralkyl group whose aryl portion has 6 to 50 carbon atoms and whose alkyl portion has 1 to 50 carbon atoms, a substituted or unsubstituted aryloxy group having 5 to 50 carbon atoms, a substituted or unsubstituted arylthio group having 5 to 50 carbon atoms, a substituted or unsubstituted alkoxycarbonyl group whose alkyl portion has 1 to 50 carbon atoms, a substituted or unsubstituted silyl group, a carboxyl group, a halogen atom, a cyano group, a nitro group, and a hydroxyl group.

29. The organic electroluminescence device according to claim 28, wherein, in the general formula (2a), Ar¹¹ and Ar¹² represent different groups.

30. The organic electroluminescence device according to claim 26, wherein the light emitting layer contains the aromatic amine derivative and a compound having a structure having a pyrene central skeleton and represented by the following general formula (2b): 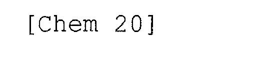 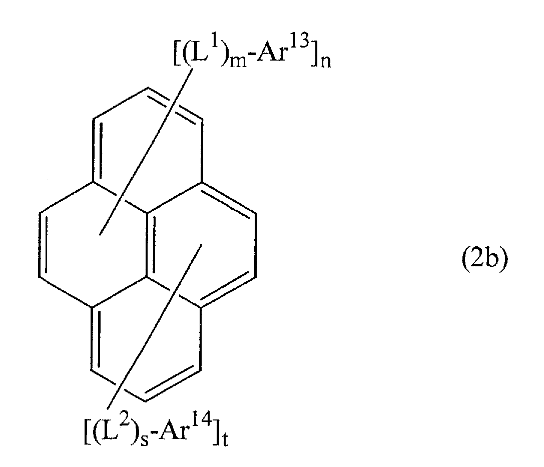 where:
Ar¹³ and Ar¹⁴ each independently represent a substituted or unsubstituted aryl group having 6 to 50 carbon atoms;
L¹ and L² each independently represent a group selected from a substituted or unsubstituted phenylene group, a substituted or unsubstituted naphthalenylene group, a substituted or unsubstituted fluorenylene group, and a substituted or unsubstituted dibenzosilolylene group;
m represents an integer of 0 to 2, n represents an integer of 1 to 4, s represents an integer of 0 to 2, and t represents an integer of 0 to 4; and
L¹ or Ar¹³ is bonded to any one of 1- to 5-positions of pyrene, and L² or Ar¹⁴ is bonded to any one of 6- to 10-positions of pyrene.

31. The organic electroluminescence device according to claim 26, wherein the light emitting layer contains the aromatic amine derivative and a compound having a structure having a triphenylamine skeleton and represented by the following general formula (2c): 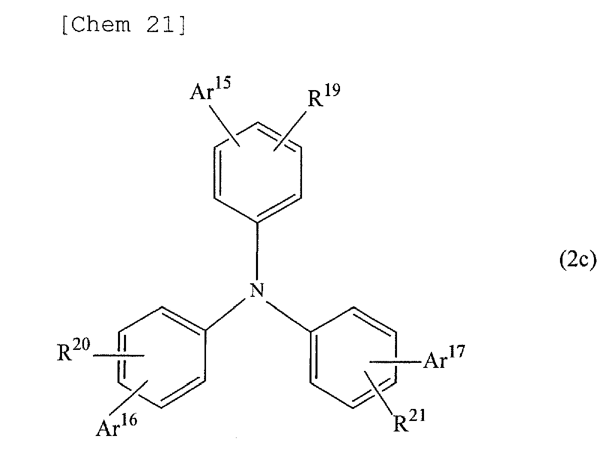 where:
Ar¹⁵ to Ar¹⁷ are each independently selected from a group having an anthracene structure, a group having a phenanthrene structure, and a group having a pyrene structure; and
R¹⁹ to R²¹ each independently represent a hydrogen atom or a substituent.

32. The organic electroluminescence device according to claim 26, wherein the light emitting layer contains the aromatic amine derivative and a compound having a structure represented by the following general formula (2d): 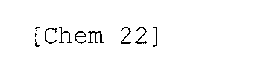 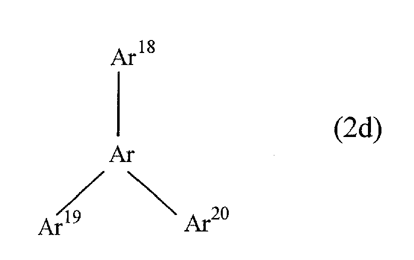 where:
Ar¹⁸ to Ar²⁰ each independently represent an aryl group having 6 to 50 ring carbon atoms; and
Ar represents a trivalent group derived from an aromatic ring or from a heterocyclic aromatic ring.

33. An organic electroluminescence material-containing solution, comprising:
the aromatic amine derivative according to claim 1 as one of organic electroluminescence materials; and
a solvent.

34. The organic electroluminescence material-containing solution according to claim 33, wherein:
the organic electroluminescence materials include a host material and a dopant material;
the dopant material comprises the aromatic amine derivative according to claim 1; and
the host material comprises at least one kind selected from the compound represented by the general formula (2a) according to claim 28, the compound represented by the general formula (2b) according to claim 21, the compound represented by the general formula (2c) according to claim 31, and the compound represented by the general formula (2d) according to claim 32.
